# EUROPEAN PATENT APPLICATION

(11) **EP 1 705 251 A1**
(43) Date of publication of application: **27.09.2006**
(21) Application number: 04773767.1
(22) Date of filing: 08.10.2004
(51) Int. Cl.: C12P 21/08, C12N 15/10, C12N 1/19, C12N 5/10

(54) **PROCESS FOR PRODUCING ANTIBODY COMPOSITION BY USING RNA INHIBITING THE FUNCTION OF a1,6-FUCOSYLTRANSFERASE**

(30) Priority: 09.10.2003 JP 2003350167
(71) Applicant: KYOWA HAKKO KOGYO CO., LTD., Chiyoda-ku, Tokyo 100-8185 (JP)
(72) Inventor: NISHIYA, Harue, c/o BioFrontier Laboratories, 3 chome Machida-shi, Tokyo 194-8533 (JP); SATOH, Mitsuo,c/o BioFrontier Laboratories, 3-chome Machida-shi, Tokyo 194-8533 (JP); MORI, Katsuhiro,c/o BioFrontier Laboratories, 3-chome Machida-shi, Tokyo 194-8533 (JP)
(74) Representative: Vossius & Partner
(86) International application number: PCT/JP2004/015316
(87) International publication number: WO 2005/035778

(57) **Abstract**

The present invention provides a process for producing an antibody composition using a cell, which comprises using a cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced; the RNA used in the production process; a DNA corresponding to the RNA; a cell in which the RNA or DNA is introduced or expressed; a process for producing the cell; and a method for suppressing the enzyme.

SEQUENCE LISTING

<110> KYOWA HAKKO KOGYO CO., LTD.

<120> Process for producing the antibody composition using RNA which inhibits
a function of α1,6-fucosyltransferase

<130> 11621WO1

<150> P2003-350167
<151> 2003-10-09

<160> 35

<170> PatentIn Ver. 2.1

<210> 1
<211> 2008
<212> DNA
<213> Cricetulus griseus

<400> 1
aacagaaact tattttcctg tgtggctaac tagaaccaga gtacaatgtt tccaattctt 60

tgagctccga gaagacagaa gggagttgaa actctgaaaa tgcgggcatg gactggttcc 120

tggcgttgga ttatgctcat tctttttgcc tgggggacct tattgtttta tataggtggt 180

catttggttc gagataatga ccaccctgac cattctagca gagaactctc caagattctt 240

gcaaagctgg agcgcttaaa acaacaaaat gaagacttga ggagaatggc tgagtctctc 300

cgaataccag aaggccctat tgatcagggg acagctacag gaagagtccg tgttttagaa 360

gaacagcttg ttaaggccaa agaacagatt gaaaattaca agaaacaagc taggaatgat 420

ctgggaaagg atcatgaaat cttaaggagg aggattgaaa atggagctaa agagctctgg 480

ttttttctac aaagtgaatt gaagaaatta aagaaattag aaggaaacga actccaaaga 540

catgcagatg aaattctttt ggatttagga catcatgaaa ggtctatcat gacagatcta 600

tactacctca gtcaaacaga tggagcaggt gagtggcggg aaaaagaagc caaagatctg 660

acagagctgg tccagcggag aataacatat ctgcagaatc ccaaggactg cagcaaagcc 720

agaaagctgg tatgtaatat caacaaaggc tgtggctatg gatgtcaact ccatcatgtg 780

gtttactgct tcatgattgc ttatggcacc cagcgaacac tcatcttgga atctcagaat 840

tggcgctatg ctactggagg atgggagact gtgtttagac ctgtaagtga gacatgcaca 900

gacaggtctg gcctctccac tggacactgg tcaggtgaag tgaaggacaa aaatgttcaa 960

gtggtcgagc tccccattgt agacagcctc catcctcgtc ctccttactt acccttggct 1020

gtaccagaag accttgcaga tcgactcctg agagtccatg gtgatcctgc agtgtggtgg 1080

gtatcccagt ttgtcaaata cttgatccgt ccacaacctt ggctggaaag ggaaatagaa 1140

gaaaccacca agaagcttgg cttcaaacat ccagttattg gagtccatgt cagacgcact 1200

gacaaagtgg gaacagaagc agccttccat cccattgagg aatacatggt acacgttgaa 1260

gaacattttc agcttctcga acgcagaatg aaagtggata aaaaaagagt gtatctggcc 1320

actgatgacc cttctttgtt aaaggaggca aagacaaagt actccaatta tgaatttatt 1380

agtgataact ctatttcttg gtcagctgga ctacacaacc gatacacaga aaattcactt 1440

cggggcgtga tcctggatat acactttctc tcccaggctg acttccttgt gtgtactttt 1500

tcatcccagg tctgtagggt tgcttatgaa atcatgcaaa cactgcatcc tgatgcctct 1560

gcaaacttcc attctttaga tgacatctac tattttggag gccaaaatgc ccacaaccag 1620

attgcagttt atcctcacca acctcgaact aaagaggaaa tccccatgga acctggagat 1680

atcattggtg tggctggaaa ccattggaat ggttactcta aaggtgtcaa cagaaaacta 1740

ggaaaaacag gcctgtaccc ttcctacaaa gtccgagaga agatagaaac agtcaaatac 1800

cctacatatc ctgaagctga aaaatagaga tggagtgtaa gagattaaca acagaattta 1860

gttcagacca tctcagccaa gcagaagacc cagactaaca tatggttcat tgacagacat 1920

gctccgcacc aagagcaagt gggaaccctc agatgctgca ctggtggaac gcctctttgt 1980

gaagggctgc tgtgccctca agcccatg 2008

<210> 2
<211> 1728
<212> DNA
<213> Mus musculus

<400> 2
atgcgggcat ggactggttc ctggcgttgg attatgctca ttctttttgc ctgggggacc 60

ttgttatttt atataggtgg tcatttggtt cgagataatg accaccctga tcactccagc 120

agagaactct ccaagattct tgcaaagctt gaacgcttaa aacagcaaaa tgaagacttg 180

aggcgaatgg ctgagtctct ccgaatacca gaaggcccca ttgaccaggg gacagctaca 240

ggaagagtcc gtgttttaga agaacagctt gttaaggcca aagaacagat tgaaaattac 300

aagaaacaag ctagaaatgg tctggggaag gatcatgaaa tcttaagaag gaggattgaa 360

aatggagcta aagagctctg gtttttctta caaagcgaac tgaagaaatt aaagcattta 420

gaaggaaatg aactccaaag acatgcagat gaaattcttt tggatttagg acaccatgaa 480

aggtctatca tgacagatct atactacctc agtcaaacag atggagcagg ggattggcgt 540

gaaaaagagg ccaaagatct gacagagctg gtccagcgga gaataacata tctccagaat 600

cctaaggact gcagcaaagc caggaagctg gtgtgtaaca tcaataaagg ctgtggctat 660

ggttgtcaac tccatcacgt ggtctactgt ttcatgattg cttatggcac ccagcgaaca 720

ctcatcttgg aatctcagaa ttggcgctat gctactggtg gatgggagac tgtgtttaga 780

cctgtaagtg agacatgtac agacagatct ggcctctcca ctggacactg gtcaggtgaa 840

gtaaatgaca aaaacattca agtggtcgag ctccccattg tagacagcct ccatcctcgg 900

cctccttact taccactggc tgttccagaa gaccttgcag accgactcct aagagtccat 960

ggtgaccctg cagtgtggtg ggtgtcccag tttgtcaaat acttgattcg tccacaacct 1020

tggctggaaa aggaaataga agaagccacc aagaagcttg gcttcaaaca tccagttatt 1080

ggagtccatg tcagacgcac agacaaagtg ggaacagaag cagccttcca ccccatcgag 1140

gagtacatgg tacacgttga agaacatttt cagcttctcg cacgcagaat gcaagtggat 1200

aaaaaaagag tatatctggc tactgatgat cctactttgt taaaggaggc aaagacaaag 1260

tactccaatt atgaatttat tagtgataac tctatttctt ggtcagctgg actacacaat 1320

cggtacacag aaaattcact tcggggtgtg atcctggata tacactttct ctcacaggct 1380

gactttctag tgtgtacttt ttcatcccag gtctgtcggg ttgcttatga aatcatgcaa 1440

accctgcatc ctgatgcctc tgcgaacttc cattctttgg atgacatcta ctattttgga 1500

ggccaaaatg cccacaatca gattgctgtt tatcctcaca aacctcgaac tgaagaggaa 1560

attccaatgg aacctggaga tatcattggt gtggctggaa accattggga tggttattct 1620

aaaggtatca acagaaaact tggaaaaaca ggcttatatc cctcctacaa agtccgagag 1680

aagatagaaa cagtcaagta tcccacatat cctgaagctg aaaaatag 1728

<210> 3
<211> 979
<212> DNA
<213> Rattus norvegicus

<400> 3
actcatcttg gaatctcaga attggcgcta tgctactggt ggatgggaga ctgtgtttag 60

acctgtaagt gagacatgca cagacagatc tggcctctcc actggacact ggtcaggtga 120

agtgaatgac aaaaatattc aagtggtgga gctccccatt gtagacagcc ttcatcctcg 180

gcctccttac ttaccactgg ctgttccaga agaccttgca gatcgactcg taagagtcca 240

tggtgatcct gcagtgtggt gggtgtccca gttcgtcaaa tatttgattc gtccacaacc 300

ttggctagaa aaggaaatag aagaagccac caagaagctt ggcttcaaac atccagtcat 360

tggagtccat gtcagacgca cagacaaagt gggaacagag gcagccttcc atcccatcga 420

agagtacatg gtacatgttg aagaacattt tcagcttctc gcacgcagaa tgcaagtgga 480

taaaaaaaga gtatatctgg ctaccgatga ccctgctttg ttaaaggagg caaagacaaa 540

gtactccaat tatgaattta ttagtgataa ctctatttct tggtcagctg gactacacaa 600

tcggtacaca gaaaattcac ttcggggcgt gatcctggat atacactttc tctctcaggc 660

tgacttccta gtgtgtactt tttcatccca ggtctgtcgg gttgcttatg aaatcatgca 720

aaccctgcat cctgatgcct ctgcaaactt ccactcttta gatgacatct actattttgg 780

aggccaaaat gcccacaacc agattgccgt ttatcctcac aaacctcgaa ctgatgagga 840

aattccaatg gaacctggag atatcattgg tgtggctgga aaccattggg atggttattc 900

taaaggtgtc aacagaaaac ttggaaaaac aggcttatat ccctcctaca aagtccgaga 960

gaagatagaa acggtcaag 979

<210> 4
<211> 1728
<212> DNA
<213> Homo Sapience

<400> 4
   atgcggccat ggactggttc ctggcgttgg attatgctca ttctttttgc ctgggggacc 60
   ttgctgtttt atataggtgg tcacttggta cgagataatg accatcctga tcactctagc 120
   cgagaactgt ccaagattct ggcaaagctt gaacgcttaa aacagcagaa tgaagacttg 180
   aggcgaatgg ccgaatctct ccggatacca gaaggcccta ttgatcaggg gccagctata 240
   ggaagagtac gcgttttaga agagcagctt gttaaggcca aagaacagat tgaaaattac 300
   aagaaacaga ccagaaatgg tctggggaag gatcatgaaa tcctgaggag gaggattgaa 360
   aatggagcta aagagctctg gtttttccta cagagtgaat tgaagaaatt aaagaactta 420
   gaaggaaatg aactccaaag acatgcagat gaatttcttt tggatttagg acatcatgaa 480
   aggtctataa tgacggatct atactacctc agtcagacag atggagcagg tgattggcgg 540
   gaaaaagagg ccaaagatct gacagaactg gttcagcgga gaataacata tcttcagaat 600
   cccaaggact gcagcaaagc caaaaagctg gtgtgtaata tcaacaaagg ctgtggctat 660
   ggctgtcagc tccatcatgt ggtctactgc ttcatgattg catatggcac ccagcgaaca 720
   ctcatcttgg aatctcagaa ttggcgctat gctactggtg gatgggagac tgtatttagg 780
   cctgtaagtg agacatgcac agacagatct ggcatctcca ctggacactg gtcaggtgaa 840
   gtgaaggaca aaaatgttca agtggtcgag cttcccattg tagacagtct tcatccccgt 900
   cctccatatt tacccttggc tgtaccagaa gacctcgcag atcgacttgt acgagtgcat 960
   ggtgaccctg cagtgtggtg ggtgtctcag tttgtcaaat acttgatccg cccacagcct 1020
   tggctagaaa aagaaataga agaagccacc aagaagcttg gcttcaaaca tccagttatt 1080
   ggagtccatg tcagacgcac agacaaagtg ggaacagaag ctgccttcca tcccattgaa 1140
   gagtacatgg tgcatgttga agaacatttt cagcttcttg cacgcagaat gcaagtggac 1200
   aaaaaaagag tgtatttggc cacagatgac ccttctttat taaaggaggc aaaaacaaag 1260
   taccccaatt atgaatttat tagtgataac tctatttcct ggtcagctgg actgcacaat 1320
   cgatacacag aaaattcact tcgtggagtg atcctggata tacattttct ctctcaggca 1380
   gacttcctag tgtgtacttt ttcatcccag gtctgtcgag ttgcttatga aattatgcaa 1440
   acactacatc ctgatgcctc tgcaaacttc cattctttag atgacatcta ctattttggg 1500
   ggccagaatg cccacaatca aattgccatt tatgctcacc aaccccgaac tgcagatgaa 1560
   attcccatgg aacctggaga tatcattggt gtggctggaa atcattggga tggctattct 1620
   aaaggtgtca acaggaaatt gggaaggacg ggcctatatc cctcctacaa agttcgagag 1680
   aagatagaaa cggtcaagta ccccacatat cctgaggctg agaaataa 1728

<210> 5
<211> 575
<212> PRT

<213> Cricetulus griseus

<400> 5
Met Arg Ala Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Thr Ala Thr
65                  70                  75                  80

Gly Arg Val Arg Val Leu Glu Glu Gln Leu Val Lys Ala Lys Glu Gln
                85                  90                  95

Ile Glu Asn Tyr Lys Lys Gln Ala Arg Asn Asp Leu Gly Lys Asp His
            100                 105                 110

Glu Ile Leu Arg Arg Arg Ile Glu Asn Gly Ala Lys Glu Leu Trp Phe
        115                 120                 125

Phe Leu Gln Ser Glu Leu Lys Lys Leu Lys Lys Leu Glu Gly Asn Glu
    130                 135                 140

Leu Gln Arg His Ala Asp Glu Ile Leu Leu Asp Leu Gly His His Glu
145                 150                 155                 160

Arg Ser Ile Met Thr Asp Leu Tyr Tyr Leu Ser Gln Thr Asp Gly Ala
                165                 170                 175

Gly Glu Trp Arg Glu Lys Glu Ala Lys Asp Leu Thr Glu Leu Val Gln
            180                 185                 190

Arg Arg Ile Thr Tyr Leu Gln Asn Pro Lys Asp Cys Ser Lys Ala Arg
        195                 200                 205

Lys Leu Val Cys Asn Ile Asn Lys Gly Cys Gly Tyr Gly Cys Gln Leu
    210                 215                 220

His His Val Val Tyr Cys Phe Met Ile Ala Tyr Gly Thr Gln Arg Thr
225                 230                 235                 240

Leu Ile Leu Glu Ser Gln Asn Trp Arg Tyr Ala Thr Gly Gly Trp Glu
                245                 250                 255

Thr Val Phe Arg Pro Val Ser Glu Thr Cys Thr Asp Arg Ser Gly Leu
            260                 265                 270

Ser Thr Gly His Trp Ser Gly Glu Val Lys Asp Lys Asn Val Gln Val
        275                 280                 285

Val Glu Leu Pro Ile Val Asp Ser Leu His Pro Arg Pro Pro Tyr Leu
    290                 295                 300

Pro Leu Ala Val Pro Glu Asp Leu Ala Asp Arg Leu Leu Arg Val His
305                 310                 315                 320

Gly Asp Pro Ala Val Trp Trp Val Ser Gln Phe Val Lys Tyr Leu Ile
                325                 330                 335

Arg Pro Gln Pro Trp Leu Glu Arg Glu Ile Glu Glu Thr Thr Lys Lys
            340                 345                 350

Leu Gly Phe Lys His Pro Val Ile Gly Val His Val Arg Arg Thr Asp
        355                 360                 365

Lys Val Gly Thr Glu Ala Ala Phe His Pro Ile Glu Glu Tyr Met Val
    370                 375                 380

His Val Glu Glu His Phe Gln Leu Leu Glu Arg Arg Met Lys Val Asp

385                 390                 395                 400

Lys Lys Arg Val Tyr Leu Ala Thr Asp Asp Pro Ser Leu Leu Lys Glu
                405                 410                 415

Ala Lys Thr Lys Tyr Ser Asn Tyr Glu Phe Ile Ser Asp Asn Ser Ile
            420                 425                 430

Ser Trp Ser Ala Gly Leu His Asn Arg Tyr Thr Glu Asn Ser Leu Arg
        435                 440                 445

Gly Val Ile Leu Asp Ile His Phe Leu Ser Gln Ala Asp Phe Leu Val
    450                 455                 460

Cys Thr Phe Ser Ser Gln Val Cys Arg Val Ala Tyr Glu Ile Met Gln
465                 470                 475                 480

Thr Leu His Pro Asp Ala Ser Ala Asn Phe His Ser Leu Asp Asp Ile
                485                 490                 495

Tyr Tyr Phe Gly Gly Gln Asn Ala His Asn Gln Ile Ala Val Tyr Pro
            500                 505                 510

His Gln Pro Arg Thr Lys Glu Glu Ile Pro Met Glu Pro Gly Asp Ile
        515                 520                 525

Ile Gly Val Ala Gly Asn His Trp Asn Gly Tyr Ser Lys Gly Val Asn
    530                 535                 540

Arg Lys Leu Gly Lys Thr Gly Leu Tyr Pro Ser Tyr Lys Val Arg Glu
545                 550                 555                 560

Lys Ile Glu Thr Val Lys Tyr Pro Thr Tyr Pro Glu Ala Glu Lys
                565                 570

<210> 6
<211> 575

<212> PRT
<213> Mus musculus

<400> 6
Met Arg Ala Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Thr Ala Thr
65                  70                  75                  80

Gly Arg Val Arg Val Leu Glu Glu Gln Leu Val Lys Ala Lys Glu Gln
                85                  90                  95

Ile Glu Asn Tyr Lys Lys Gln Ala Arg Asn Gly Leu Gly Lys Asp His
            100                 105                 110

Glu Ile Leu Arg Arg Arg Ile Glu Asn Gly Ala Lys Glu Leu Trp Phe
        115                 120                 125

Phe Leu Gln Ser Glu Leu Lys Lys Leu Lys His Leu Glu Gly Asn Glu
    130                 135                 140

Leu Gln Arg His Ala Asp Glu Ile Leu Leu Asp Leu Gly His His Glu
145                 150                 155                 160

Arg Ser Ile Met Thr Asp Leu Tyr Tyr Leu Ser Gln Thr Asp Gly Ala
                165                 170                 175

Gly Asp Trp Arg Glu Lys Glu Ala Lys Asp Leu Thr Glu Leu Val Gln

180                 185                 190

Arg Arg Ile Thr Tyr Leu Gln Asn Pro Lys Asp Cys Ser Lys Ala Arg
        195                 200                 205

Lys Leu Val Cys Asn Ile Asn Lys Gly Cys Gly Tyr Gly Cys Gln Leu
    210                 215                 220

His His Val Val Tyr Cys Phe Met Ile Ala Tyr Gly Thr Gln Arg Thr
225                 230                 235                 240

Leu Ile Leu Glu Ser Gln Asn Trp Arg Tyr Ala Thr Gly Gly Trp Glu
                245                 250                 255

Thr Val Phe Arg Pro Val Ser Glu Thr Cys Thr Asp Arg Ser Gly Leu
            260                 265                 270

Ser Thr Gly His Trp Ser Gly Glu Val Asn Asp Lys Asn Ile Gln Val
        275                 280                 285

Val Glu Leu Pro Ile Val Asp Ser Leu His Pro Arg Pro Pro Tyr Leu
    290                 295                 300

Pro Leu Ala Val Pro Glu Asp Leu Ala Asp Arg Leu Leu Arg Val His
305                 310                 315                 320

Gly Asp Pro Ala Val Trp Trp Val Ser Gln Phe Val Lys Tyr Leu Ile
                325                 330                 335

Arg Pro Gln Pro Trp Leu Glu Lys Glu Ile Glu Glu Ala Thr Lys Lys
            340                 345                 350

Leu Gly Phe Lys His Pro Val Ile Gly Val His Val Arg Arg Thr Asp
        355                 360                 365

Lys Val Gly Thr Glu Ala Ala Phe His Pro Ile Glu Glu Tyr Met Val
    370                 375                 380

His Val Glu Glu His Phe Gln Leu Leu Ala Arg Arg Met Gln Val Asp
385                 390                 395                 400

Lys Lys Arg Val Tyr Leu Ala Thr Asp Asp Pro Thr Leu Leu Lys Glu
                405                 410                 415

Ala Lys Thr Lys Tyr Ser Asn Tyr Glu Phe Ile Ser Asp Asn Ser Ile
            420                 425                 430

Ser Trp Ser Ala Gly Leu His Asn Arg Tyr Thr Glu Asn Ser Leu Arg
        435                 440                 445

Gly Val Ile Leu Asp Ile His Phe Leu Ser Gln Ala Asp Phe Leu Val
    450                 455                 460

Cys Thr Phe Ser Ser Gln Val Cys Arg Val Ala Tyr Glu Ile Met Gln
465                 470                 475                 480

Thr Leu His Pro Asp Ala Ser Ala Asn Phe His Ser Leu Asp Asp Ile
                485                 490                 495

Tyr Tyr Phe Gly Gly Gln Asn Ala His Asn Gln Ile Ala Val Tyr Pro
            500                 505                 510

His Lys Pro Arg Thr Glu Glu Glu Ile Pro Met Glu Pro Gly Asp Ile
        515                 520                 525

Ile Gly Val Ala Gly Asn His Trp Asp Gly Tyr Ser Lys Gly Ile Asn
    530                 535                 540

Arg Lys Leu Gly Lys Thr Gly Leu Tyr Pro Ser Tyr Lys Val Arg Glu
545                 550                 555                 560

Lys Ile Glu Thr Val Lys Tyr Pro Thr Tyr Pro Glu Ala Glu Lys
                565                 570

<210> 7

<211> 575
<212> PRT
<214> Rattus norvegicus

<400> 7
Met Arg Ala Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Thr Ala Thr
65                  70                  75                  80

Gly Arg Val Arg Val Leu Glu Glu Gln Leu Val Lys Ala Lys Glu Gln
                85                  90                  95

Ile Glu Asn Tyr Lys Lys Gln Ala Arg Asn Gly Leu Gly Lys Asp His
            100                 105                 110

Glu Ile Leu Arg Arg Arg Ile Glu Asn Gly Ala Lys Glu Leu Trp Phe
        115                 120                 125

Phe Leu Gln Ser Glu Leu Lys Lys Leu Lys His Leu Glu Gly Asn Glu
    130                 135                 140

Leu Gln Arg His Ala Asp Glu Ile Leu Leu Asp Leu Gly His His Glu
145                 150                 155                 160

Arg Ser Ile Met Thr Asp Leu Tyr Tyr Leu Ser Gln Thr Asp Gly Ala
                165                 170                 175

Gly Asp Trp Arg Glu Lys Glu Ala Lys Asp Leu Thr Glu Leu Val Gln
            180                 185                 190

Arg Arg Ile Thr Tyr Leu Gln Asn Pro Lys Asp Cys Ser Lys Ala Arg
        195                 200                 205

Lys Leu Val Cys Asn Ile Asn Lys Gly Cys Gly Tyr Gly Cys Gln Leu
    210                 215                 220

His His Val Val Tyr Cys Phe Met Ile Ala Tyr Gly Thr Gln Arg Thr
225                 230                 235                 240

Leu Ile Leu Glu Ser Gln Asn Trp Arg Tyr Ala Thr Gly Gly Trp Glu
                245                 250                 255

Thr Val Phe Arg Pro Val Ser Glu Thr Cys Thr Asp Arg Ser Gly Leu
            260                 265                 270

Ser Thr Gly His Trp Ser Gly Glu Val Asn Asp Lys Asn Ile Gln Val
        275                 280                 285

Val Glu Leu Pro Ile Val Asp Ser Leu His Pro Arg Pro Pro Tyr Leu
    290                 295                 300

Pro Leu Ala Val Pro Glu Asp Leu Ala Asp Arg Leu Val Arg Val His
305                 310                 315                 320

Gly Asp Pro Ala Val Trp Trp Val Ser Gln Phe Val Lys Tyr Leu Ile
                325                 330                 335

Arg Pro Gln Pro Trp Leu Glu Lys Glu Ile Glu Glu Ala Thr Lys Lys
            340                 345                 350

Leu Gly Phe Lys His Pro Val Ile Gly Val His Val Arg Arg Thr Asp
        355                 360                 365

Lys Val Gly Thr Glu Ala Ala Phe His Pro Ile Glu Glu Tyr Met Val
    370                 375                 380

His Val Glu Glu His Phe Gln Leu Leu Ala Arg Arg Met Gln Val Asp
385                 390                 395                 400

Lys Lys Arg Val Tyr Leu Ala Thr Asp Asp Pro Ala Leu Leu Lys Glu
                405                 410                 415

Ala Lys Thr Lys Tyr Ser Asn Tyr Glu Phe Ile Ser Asp Asn Ser Ile
            420                 425                 430

Ser Trp Ser Ala Gly Leu His Asn Arg Tyr Thr Glu Asn Ser Leu Arg
        435                 440                 445

Gly Val Ile Leu Asp Ile His Phe Leu Ser Gln Ala Asp Phe Leu Val
    450                 455                 460

Cys Thr Phe Ser Ser Gln Val Cys Arg Val Ala Tyr Glu Ile Met Gln
465                 470                 475                 480

Thr Leu His Pro Asp Ala Ser Ala Asn Phe His Ser Leu Asp Asp Ile
                485                 490                 495

Tyr Tyr Phe Gly Gly Gln Asn Ala His Asn Gln Ile Ala Val Tyr Pro
            500                 505                 510

His Lys Pro Arg Thr Asp Glu Glu Ile Pro Met Glu Pro Gly Asp Ile
        515                 520                 525

Ile Gly Val Ala Gly Asn His Trp Asp Gly Tyr Ser Lys Gly Val Asn
    530                 535                 540

Arg Lys Leu Gly Lys Thr Gly Leu Tyr Pro Ser Tyr Lys Val Arg Glu
545                 550                 555                 560

Lys Ile Glu Thr Val Lys Tyr Pro Thr Tyr Pro Glu Ala Glu Lys
                565                 570

<210> 8
<211> 575
<212> PRT
<215> Homo Sapience

<400> 8
Met Arg Pro Trp Thr Gly Ser Trp Arg Trp Ile Met Leu Ile Leu Phe
1               5                   10                  15

Ala Trp Gly Thr Leu Leu Phe Tyr Ile Gly Gly His Leu Val Arg Asp
            20                  25                  30

Asn Asp His Pro Asp His Ser Ser Arg Glu Leu Ser Lys Ile Leu Ala
        35                  40                  45

Lys Leu Glu Arg Leu Lys Gln Gln Asn Glu Asp Leu Arg Arg Met Ala
    50                  55                  60

Glu Ser Leu Arg Ile Pro Glu Gly Pro Ile Asp Gln Gly Pro Ala Ile
65                  70                  75                  80

Gly Arg Val Arg Val Leu Glu Glu Gln Leu Val Lys Ala Lys Glu Gln
                85                  90                  95

Ile Glu Asn Tyr Lys Lys Gln Thr Arg Asn Gly Leu Gly Lys Asp His
            100                 105                 110

Glu Ile Leu Arg Arg Arg Ile Glu Asn Gly Ala Lys Glu Leu Trp Phe
        115                 120                 125

Phe Leu Gln Ser Glu Leu Lys Lys Leu Lys Asn Leu Glu Gly Asn Glu
    130                 135                 140

Leu Gln Arg His Ala Asp Glu Phe Leu Leu Asp Leu Gly His His Glu
145                 150                 155                 160

Arg Ser Ile Met Thr Asp Leu Tyr Tyr Leu Ser Gln Thr Asp Gly Ala
                165                 170                 175

Gly Asp Trp Arg Glu Lys Glu Ala Lys Asp Leu Thr Glu Leu Val Gln
            180                 185                 190

Arg Arg Ile Thr Tyr Leu Gln Asn Pro Lys Asp Cys Ser Lys Ala Lys
        195                 200                 205

Lys Leu Val Cys Asn Ile Asn Lys Gly Cys Gly Tyr Gly Cys Gln Leu
    210                 215                 220

His His Val Val Tyr Cys Phe Met Ile Ala Tyr Gly Thr Gln Arg Thr
225                 230                 235                 240

Leu Ile Leu Glu Ser Gln Asn Trp Arg Tyr Ala Thr Gly Gly Trp Glu
                245                 250                 255

Thr Val Phe Arg Pro Val Ser Glu Thr Cys Thr Asp Arg Ser Gly Ile
            260                 265                 270

Ser Thr Gly His Trp Ser Gly Glu Val Lys Asp Lys Asn Val Gln Val
        275                 280                 285

Val Glu Leu Pro Ile Val Asp Ser Leu His Pro Arg Pro Pro Tyr Leu
    290                 295                 300

Pro Leu Ala Val Pro Glu Asp Leu Ala Asp Arg Leu Val Arg Val His
305                 310                 315                 320

Gly Asp Pro Ala Val Trp Trp Val Ser Gln Phe Val Lys Tyr Leu Ile
                325                 330                 335

Arg Pro Gln Pro Trp Leu Glu Lys Glu Ile Glu Glu Ala Thr Lys Lys
            340                 345                 350

Leu Gly Phe Lys His Pro Val Ile Gly Val His Val Arg Arg Thr Asp
        355                 360                 365

Lys Val Gly Thr Glu Ala Ala Phe His Pro Ile Glu Glu Tyr Met Val

370                 375                 380

His Val Glu Glu His Phe Gln Leu Leu Ala Arg Arg Met Gln Val Asp
385                 390                 395                 400

Lys Lys Arg Val Tyr Leu Ala Thr Asp Asp Pro Ser Leu Leu Lys Glu
                405                 410                 415

Ala Lys Thr Lys Tyr Pro Asn Tyr Glu Phe Ile Ser Asp Asn Ser Ile
            420                 425                 430

Ser Trp Ser Ala Gly Leu His Asn Arg Tyr Thr Glu Asn Ser Leu Arg
        435                 440                 445

Gly Val Ile Leu Asp Ile His Phe Leu Ser Gln Ala Asp Phe Leu Val
    450                 455                 460

Cys Thr Phe Ser Ser Gln Val Cys Arg Val Ala Tyr Glu Ile Met Gln
465                 470                 475                 480

Thr Leu His Pro Asp Ala Ser Ala Asn Phe His Ser Leu Asp Asp Ile
                485                 490                 495

Tyr Tyr Phe Gly Gly Gln Asn Ala His Asn Gln Ile Ala Ile Tyr Ala
            500                 505                 510

His Gln Pro Arg Thr Ala Asp Glu Ile Pro Met Glu Pro Gly Asp Ile
        515                 520                 525

Ile Gly Val Ala Gly Asn His Trp Asp Gly Tyr Ser Lys Gly Val Asn
    530                 535                 540

Arg Lys Leu Gly Arg Thr Gly Leu Tyr Pro Ser Tyr Lys Val Arg Glu
545                 550                 555                 560

Lys Ile Glu Thr Val Lys Tyr Pro Thr Tyr Pro Glu Ala Glu Lys
                565                 570

<210> 9
<211> 40
<212> RNA
<213> Artificial Sequence

<220>
<223> Description of Artificial Sequence: Synthetic RNA

<400> 9
gaagggaguu gaaacucuga aaaugcgggc auggacuggu                                40


<210> 10
<211> 31
<212> RNA
<213> Artificial Sequence

<220>
<223> Description of Artificial Sequence: Synthetic RNA

<400> 10
gaggagaaug gcugagucuc uccgaauacc a                                         31


<210> 11
<211> 33
<212> RNA
<213> Artificial Sequence

<220>
<223> Description of Artificial Sequence: Synthetic RNA

<400> 11
ccaaagacau gcagaugaaa uucuuuugga uuu                                       33


<210> 12

<211> 35
<212> RNA
<213> Artificial Sequence

<220>
<223> Description of Artificial Sequence: Synthetic RNA

<400> 12
ucuuggaauc ucagaauugg cgcuaugcua cugga                                35


<210> 13
<211> 32
<212> RNA
<213> Artificial Sequence

<220>
<223> Description of Artificial Sequence: Synthetic RNA

<400> 13
auacacagaa aauucacuuc ggggcgugau cc                                   32


<210> 14
<211> 34
<212> RNA
<213> Artificial Sequence

<220>
<223> Description of Artificial Sequence: Synthetic RNA

<400> 14
ucaucccagg ucuguagggu ugcuuaugaa auca                                 34


<210> 15
<211> 36
<212> RNA

<213> Artificial Sequence

<220>
<223> Description of Artificial Sequence: Synthetic RNA

<400> 15
caucuacuau uuuggaggcc aaaaugccca caacca                                36


<210> 16
<211> 31
<212> RNA
<213> Artificial Sequence

<220>
<223> Description of Artificial Sequence: Synthetic RNA

<400> 16
ugcacuggug gaacgccucu uugugaaggg c                                     31


<210> 17
<211> 34
<212> RNA
<213> Artificial Sequence

<220>
<223> Description of Artificial Sequence: Synthetic RNA

<400> 17
caagaagcuu ggcuucaaac auccaguuau ugga                                  34


<210> 18
<211> 35
<212> RNA
<213> Artificial Sequence

<220>
<223> Description of Artificial Sequence: Synthetic RNA

<400> 18
uauggcaccc agcgaacacu caucuuggaa ucuca                                35

<210> 19
<211> 31
<212> RNA
<213> Artificial Sequence

<220>
<223> Description of Artificial Sequence: Synthetic RNA

<400> 19
gaggcgaaug gcugagucuc uccgaauacc a                                    31

<210> 20
<211> 31
<212> RNA
<213> Artificial Sequence

<220>
<223> Description of Artificial Sequence: Synthetic RNA

<400> 20
gaggcgaaug gccgaaucuc uccggauacc a                                    31

<210> 21
<211> 33
<212> RNA
<213> Artificial Sequence

<220>
<223> Description of Artificial Sequence: Synthetic RNA

<400> 21
ccaaagacau gcagaugaau uucuuuugga uuu                                33

<210> 22
<211> 35
<212> RNA
<213> Artificial Sequence

<220>
<223> Description of Artificial Sequence: Synthetic RNA

<400> 22
ucuuggaauc ucagaauugg cgcuaugcua cuggu                              35

<210> 23
<211> 32
<212> RNA
<213> Artificial Sequence

<220>
<223> Description of Artificial Sequence: Synthetic RNA

<400> 23
guacacagaa aauucacuuc gggugugau cc                                  32

<210> 24
<211> 32
<212> RNA
<213> Artificial Sequence

<220>
<223> Description of Artificial Sequence: Synthetic RNA

<400> 24

auacacagaa aauucacuuc guggagugau cc 32

<210> 25
<211> 32
<212> RNA
<213> Artificial Sequence

<220>
<223> Description of Artificial Sequence: Synthetic RNA

<400> 25
guacacagaa aauucacuuc ggggcgugau cc 32

<210> 26
<211> 34
<212> RNA
<213> Artificial Sequence

<220>
<223> Description of Artificial Sequence: Synthetic RNA

<400> 26
ucaucccagg ucugucgggu ugcuuaugaa auca 34

<210> 27
<211> 34
<212> RNA
<213> Artificial Sequence

<220>
<223> Description of Artificial Sequence: Synthetic RNA

<400> 27
ucaucccagg ucugucgagu ugcuuaugaa auua 34

<210> 28
<211> 36
<212> RNA
<213> Artificial Sequence

<220>
<223> Description of Artificial Sequence: Synthetic RNA

<400> 28
caucuacuau uuuggaggcc aaaaugccca caauca                                36


<210> 29
<211> 36
<212> RNA
<213> Artificial Sequence

<220>
<223> Description of Artificial Sequence: Synthetic RNA

<400> 29
caucuacuau uuugggggcc agaaugccca caauca                               36


<210> 30
<211> 34
<212> RNA
<213> Artificial Sequence

<220>
<223> Description of Artificial Sequence: Synthetic RNA

<400> 30
caagaagcuu ggcuucaaac auccagucau ugga                                  34


<210> 31

<211> 24
<212> DNA
<213> Artificial Sequence

<220>
<223> Description of Artificial Sequence: Synthetic DNA

<400> 31
gtctgaagca ttatgtgttg aagc                                              24


<210> 32
<211> 23
<212> DNA
<213> Artificial Sequence

<220>
<223> Description of Artificial Sequence: Synthetic DNA

<400> 32
gtgagtacat tcattgtact gtg                                               23


<210> 33
<211> 17
<212> DNA
<213> Artificial Sequence

<220>
<223> Description of Artificial Sequence: Synthetic DNA

<400> 33
ttcccagtca cgacgtt                                                      17


<210> 34
<211> 17
<212> DNA

<213> Artificial Sequence

<220>
<223> Description of Artificial Sequence: Synthetic DNA

<400> 34
caggaaacag ctatgac                                                    17

<210> 35
<211> 18
<212> PRT
<213> Homo sapiens
<220>
<400> 35
Asp Glu Ser Ile Tyr Ser Asn Tyr Tyr Leu Tyr Glu Ser Ile Pro Lys
 1               5                   10                  15
Pro Cys

<210> 36
<211> 22
<212> DNA
<213> Artificial Sequence

<220>
<223> Description of Artificial Sequence: Synthetic DNA

<400> 36
atcctcgtcc tccttactta cc                                              22

<210> 37
<211> 22
<212> DNA
<213> Artificial Sequence

<220>
<223> Description of Artificial Sequence: Synthetic DNA

<400> 37
tccagctgac caagaaatag ag                                                22

<210> 38
<211> 24
<212> DNA
<213> Artificial Sequence

<220>
<223> Description of Artificial Sequence: Synthetic DNA

<400> 38
gatatcgctg cgctcgtcgt cgac                                              24

<210> 39
<211> 24
<212> DNA
<213> Artificial Sequence

<220>
<223> Description of Artificial Sequence: Synthetic DNA

<400> 39
caggaaggaa ggctggaaga gagc                                              24

<210> 40
<211> 40
<212> DNA
<213> Artificial Sequence

<220>
<223> Description of Artificial Sequence: Synthetic DNA

<400> 40

cccaagcttg atatcaaggt cgggcaggaa gagggcctat 40

<210> 41
<211> 52
<212> DNA
<213> Artificial Sequence
<220>

<223> Description of Artificial Sequence: Synthetic DNA

<400> 41
gctctagaga tatcaaaaaa ggtaccgagc tcggtgtttc gtcctttcca ca 52

<210> 42
<211> 74
<212> DNA
<213> Artificial Sequence

<220>
<223> Description of Artificial Sequence: Synthetic DNA

<400> 42
cgaatggctg agtctctccg aataccagaa cttcctgtca ttctggtatt cggagagact 60

cagccattcg gtac 74

<210> 43
<211> 74
<212> DNA
<213> Artificial Sequence

<220>
<223> Description of Artificial Sequence: Synthetic DNA

<400> 43

cgaatggctg agtctctccg aataccagaa tgacaggaag ttctggtatt cggagagact 60

cagccattcg agct 74

<210> 44
<211> 74
<212> DNA
<213> Artificial Sequence

<220>
<223> Description of Artificial Sequence: Synthetic DNA

<400> 44
cccagcgaac actcatcttg gaatctcaga cttcctgtca tctgagattc caagatgagt 60

gttcgctggg gtac 74

<210> 45
<211> 74
<212> DNA
<213> Artificial Sequence

<220>
<223> Description of Artificial Sequence: Synthetic DNA

<400> 45
cccagcgaac actcatcttg gaatctcaga tgacaggaag tctgagattc caagatgagt 60

gttcgctggg agct 74

<210> 46
<211> 32
<212> DNA
<213> Artificial Sequence

<220>
<223> Description of Artificial Sequence: Synthetic DNA

<400> 46
ggcagctgcg ccagggtttt cccagtcacg ac                                32

<210> 47
<211> 44
<212> DNA
<213> Artificial Sequence

<220>
<223> Description of Artificial Sequence: Synthetic DNA

<400> 47
cccagctgaa aaaaggtacc ctatgagctc ggggttggtt tttg                   44

<210> 48
<211> 32
<212> DNA
<213> Artificial Sequence

<220>
<223> Description of Artificial Sequence: Synthetic DNA

<400> 48
taaatagaat tcggcatcat gtggcagctg ct                                32

<210> 49
<211> 34
<212> DNA
<213> Artificial Sequence

<220>
<223> Description of Artificial Sequence: Synthetic DNA

<400> 49
aataaaggat cctggggtca tttgtcttga gggt                                34

<210> 50
<211> 788
<212> DNA
<213> Homo sapiens

<220>
<221> CDS
<222> (13)..(774)

<400> 50
gaa ttc ggc atc atg tgg cag ctg ctc ctc cca act gct ctg cta ctt  48
                Met Trp Gln Leu Leu Leu Pro Thr Ala Leu Leu Leu
                1               5                   10
cta gtt tca gct ggc atg cgg act gaa gat ctc cca aag gct gtg gtg  96
Leu Val Ser Ala Gly Met Arg Thr Glu Asp Leu Pro Lys Ala Val Val
        15                  20                  25
ttc ctg gag cct caa tgg tac agg gtg ctc gag aag gac agt gtg act 144
Phe Leu Glu Pro Gln Trp Tyr Arg Val Leu Glu Lys Asp Ser Val Thr
    30                  35                  40
ctg aag tgc cag gga gcc tac tcc cct gag gac aat tcc aca cag tgg 192
Leu Lys Cys Gln Gly Ala Tyr Ser Pro Glu Asp Asn Ser Thr Gln Trp
45                  50                  55                  60
ttt cac aat gag agc ctc atc tca agc cag gcc tcg agc tac ttc att 240
Phe His Asn Glu Ser Leu Ile Ser Ser Gln Ala Ser Ser Tyr Phe Ile
                65                  70                  75
gac gct gcc aca gtc gac gac agt gga gag tac agg tgc cag aca aac 288
Asp Ala Ala Thr Val Asp Asp Ser Gly Glu Tyr Arg Cys Gln Thr Asn
            80                  85                  90
ctc tcc acc ctc agt gac ccg gtg cag cta gaa gtc cat atc ggc tgg 336
Leu Ser Thr Leu Ser Asp Pro Val Gln Leu Glu Val His Ile Gly Trp
        95                  100                 105
ctg ttg ctc cag gcc cct cgg tgg gtg ttc aag gag gaa gac cct att 384
Leu Leu Leu Gln Ala Pro Arg Trp Val Phe Lys Glu Glu Asp Pro Ile

110                 115                 120
cac ctg agg tgt cac agc tgg aag aac act gct ctg cat aag gtc aca 432
His Leu Arg Cys His Ser Trp Lys Asn Thr Ala Leu His Lys Val Thr
125                 130                 135                 140
tat tta cag aat ggc aaa ggc agg aag tat ttt cat cat aat tct gac 480
Tyr Leu Gln Asn Gly Lys Gly Arg Lys Tyr Phe His His Asn Ser Asp
                145                 150                 155
ttc tac att cca aaa gcc aca ctc aaa gac agc ggc tcc tac ttc tgc 528
Phe Tyr Ile Pro Lys Ala Thr Leu Lys Asp Ser Gly Ser Tyr Phe Cys
            160                 165                 170
agg ggg ctt ttt ggg agt aaa aat gtg tct tca gag act gtg aac atc 576
Arg Gly Leu Phe Gly Ser Lys Asn Val Ser Ser Glu Thr Val Asn Ile
        175                 180                 185
acc atc act caa ggt ttg gca gtg tca acc atc tca tca ttc ttt cca 624
Thr Ile Thr Gln Gly Leu Ala Val Ser Thr Ile Ser Ser Phe Phe Pro
    190                 195                 200
cct ggg tac caa gtc tct ttc tgc ttg gtg atg gta ctc ctt ttt gca 672
Pro Gly Tyr Gln Val Ser Phe Cys Leu Val Met Val Leu Leu Phe Ala
205                 210                 215                 220
gtg gac aca gga cta tat ttc tct gtg aag aca aac att cga agc tca 720
Val Asp Thr Gly Leu Tyr Phe Ser Val Lys Thr Asn Ile Arg Ser Ser
                225                 230                 235
aca aga gac tgg aag gac cat aaa ttt aaa tgg aga aag gac cct caa 768
Thr Arg Asp Trp Lys Asp His Lys Phe Lys Trp Arg Lys Asp Pro Gln
            240                 245                 250
gac aaa tga ccc cag gat cc                                      788
Asp Lys

<210> 51
<211> 254
<212> PRT
<213> Homo sapiens

<400> 51
Met Trp Gln Leu Leu Leu Pro Thr Ala Leu Leu Leu Leu Val Ser Ala
  1               5                  10                  15
Gly Met Arg Thr Glu Asp Leu Pro Lys Ala Val Val Phe Leu Glu Pro

            20                  25                  30
Gln Trp Tyr Arg Val Leu Glu Lys Asp Ser Val Thr Leu Lys Cys Gln
        35                  40                  45
Gly Ala Tyr Ser Pro Glu Asp Asn Ser Thr Gln Trp Phe His Asn Glu
    50                  55                  60
Ser Leu Ile Ser Ser Gln Ala Ser Ser Tyr Phe Ile Asp Ala Ala Thr
65                  70                  75                  80
Val Asp Asp Ser Gly Glu Tyr Arg Cys Gln Thr Asn Leu Ser Thr Leu
                85                  90                  95
Ser Asp Pro Val Gln Leu Glu Val His Ile Gly Trp Leu Leu Leu Gln
            100                 105                 110
Ala Pro Arg Trp Val Phe Lys Glu Glu Asp Pro Ile His Leu Arg Cys
        115                 120                 125
His Ser Trp Lys Asn Thr Ala Leu His Lys Val Thr Tyr Leu Gln Asn
    130                 135                 140
Gly Lys Gly Arg Lys Tyr Phe His His Asn Ser Asp Phe Tyr Ile Pro
145                 150                 155                 160
Lys Ala Thr Leu Lys Asp Ser Gly Ser Tyr Phe Cys Arg Gly Leu Phe
                165                 170                 175
Gly Ser Lys Asn Val Ser Ser Glu Thr Val Asn Ile Thr Ile Thr Gln
            180                 185                 190
Gly Leu Ala Val Ser Thr Ile Ser Ser Phe Phe Pro Pro Gly Tyr Gln
        195                 200                 205
Val Ser Phe Cys Leu Val Met Val Leu Leu Phe Ala Val Asp Thr Gly
    210                 215                 220
Leu Tyr Phe Ser Val Lys Thr Asn Ile Arg Ser Ser Thr Arg Asp Trp
225                 230                 235                 240
Lys Asp His Lys Phe Lys Trp Arg Lys Asp Pro Gln Asp Lys
                245                 250

<210> 52
<211> 51
<212> DNA
<213> Artificial Sequence

<220>
<223> Description of Artificial Sequence: Synthetic DNA

<400> 52
tgttggatcc tgtcaatgat gatgatgatg atgaccttga gtgatggtga t          51

<210> 53
<211> 620
<212> DNA
<213> Homo sapiens

<220>
<221> CDS
<222> (13)..(609)

<400> 53
gaa ttc ggc atc atg tgg cag ctg ctc ctc cca act gct ctg cta ctt  48
                Met Trp Gln Leu Leu Leu Pro Thr Ala Leu Leu Leu
                1               5                   10
cta gtt tca gct ggc atg cgg act gaa gat ctc cca aag gct gtg gtg  96
Leu Val Ser Ala Gly Met Arg Thr Glu Asp Leu Pro Lys Ala Val Val
        15                  20                  25
ttc ctg gag cct caa tgg tac agg gtg ctc gag aag gac agt gtg act 144
Phe Leu Glu Pro Gln Trp Tyr Arg Val Leu Glu Lys Asp Ser Val Thr
    30                  35                  40
ctg aag tgc cag gga gcc tac tcc cct gag gac aat tcc aca cag tgg 192
Leu Lys Cys Gln Gly Ala Tyr Ser Pro Glu Asp Asn Ser Thr Gln Trp
45                  50                  55                  60
ttt cac aat gag agc ctc atc tca agc cag gcc tcg agc tac ttc att 240
Phe His Asn Glu Ser Leu Ile Ser Ser Gln Ala Ser Ser Tyr Phe Ile
                65                  70                  75
gac gct gcc aca gtc gac gac agt gga gag tac agg tgc cag aca aac 288
Asp Ala Ala Thr Val Asp Asp Ser Gly Glu Tyr Arg Cys Gln Thr Asn
            80                  85                  90
ctc tcc acc ctc agt gac ccg gtg cag cta gaa gtc cat atc ggc tgg 336
Leu Ser Thr Leu Ser Asp Pro Val Gln Leu Glu Val His Ile Gly Trp
        95                  100                 105
ctg ttg ctc cag gcc cct cgg tgg gtg ttc aag gag gaa gac cct att 384
Leu Leu Leu Gln Ala Pro Arg Trp Val Phe Lys Glu Glu Asp Pro Ile

    110                 115                 120
cac ctg agg tgt cac agc tgg aag aac act gct ctg cat aag gtc aca 432
His Leu Arg Cys His Ser Trp Lys Asn Thr Ala Leu His Lys Val Thr
125                 130                 135                 140
tat tta cag aat ggc aaa ggc agg aag tat ttt cat cat aat tct gac 480
Tyr Leu Gln Asn Gly Lys Gly Arg Lys Tyr Phe His His Asn Ser Asp
                145                 150                 155
ttc tac att cca aaa gcc aca ctc aaa gac agc ggc tcc tac ttc tgc 528
Phe Tyr Ile Pro Lys Ala Thr Leu Lys Asp Ser Gly Ser Tyr Phe Cys
            160                 165                 170
agg ggg ctt ttt ggg agt aaa aat gtg tct tca gag act gtg aac atc 576
Arg Gly Leu Phe Gly Ser Lys Asn Val Ser Ser Glu Thr Val Asn Ile
        175                 180                 185
acc atc act caa ggt cat cat cat cat cat cat tga cag gat cc 620
Thr Ile Thr Gln Gly His His His His His His
    190                 195

<210> 54
<211> 199
<212> PRT
<213> Homo sapiens

<400> 54
Met Trp Gln Leu Leu Leu Pro Thr Ala Leu Leu Leu Leu Val Ser Ala
1               5                   10                  15
Gly Met Arg Thr Glu Asp Leu Pro Lys Ala Val Val Phe Leu Glu Pro
            20                  25                  30
Gln Trp Tyr Arg Val Leu Glu Lys Asp Ser Val Thr Leu Lys Cys Gln
        35                  40                  45
Gly Ala Tyr Ser Pro Glu Asp Asn Ser Thr Gln Trp Phe His Asn Glu
    50                  55                  60
Ser Leu Ile Ser Ser Gln Ala Ser Ser Tyr Phe Ile Asp Ala Ala Thr
65                  70                  75                  80
Val Asp Asp Ser Gly Glu Tyr Arg Cys Gln Thr Asn Leu Ser Thr Leu
                85                  90                  95
Ser Asp Pro Val Gln Leu Glu Val His Ile Gly Trp Leu Leu Leu Gln
            100                 105                 110

Ala Pro Arg Trp Val Phe Lys Glu Glu Asp Pro Ile His Leu Arg Cys
        115                 120                 125
His Ser Trp Lys Asn Thr Ala Leu His Lys Val Thr Tyr Leu Gln Asn
    130                 135                 140
Gly Lys Gly Arg Lys Tyr Phe His His Asn Ser Asp Phe Tyr Ile Pro
145                 150                 155                 160
Lys Ala Thr Leu Lys Asp Ser Gly Ser Tyr Phe Cys Arg Gly Leu Phe
                165                 170                 175
Gly Ser Lys Asn Val Ser Ser Glu Thr Val Asn Ile Thr Ile Thr Gln
            180                 185                 190
Gly His His His His His His
        195

## Description

### Technical Field

The present invention relates to a process for producing an antibody composition using a cell, which comprises using a cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced; the RNA used in the process; a DNA corresponding to the RNA; a cell into which the RNA or the DNA is introduced or expressed; a method for constructing the cell; and a method for suppressing the enzyme.

### Background Art

In general, most of the humanized antibodies considered to be applicable to medicaments are prepared by using genetic recombination techniques and produced using an animal cell such as Chinese hamster ovary tissue-derived CHO cell as the host cell. Since a sugar chain structure, particularly addition of fucose to N-acetylglucosamine in the reducing end in the N-glycoside-linked sugar chain of an antibody, plays a remarkably important role in the effector function of the antibody which causes cytotoxic activities such as antibody-dependent cellular cytotoxicity (hereinafter referred to as "ADCC activity") and complement-dependent cytotoxicity (hereinafter referred to as "CDC activity") in the effector cell (WO 02/3-1140), and a difference is observed in the sugar chain structure of a glycoprotein expressed by a host cell [J. Biol. Chem., 278, 3466 (2003)], development of a host cell which can be used for the production of an antibody having higher effector function is desired.

In recent years, in the treatment of non Hodgkin's lymphoma patients by Rituxan and the treatment of breast cancer patients by Herceptin, when a therapeutic antibody induces high ADCC activity in effector cells of the patients, higher therapeutic effects can be obtained [Blood, 99, 754 (2002); J. Clin. Oncol., 21, 3940 (2003); Clin. Cancer Res., 10, 5650 (2004)].

Application of inhibitors of an enzyme relating to the modification of a sugar chain has been attempted as a method for controlling the activity of an enzyme relating to the modification of a sugar chain in a cell and modifying the sugar chain structure of the produced glycoprotein. However, since the inhibitors have low specificity and it is difficult to sufficiently inhibit the target enzyme, it is difficult to surely control the sugar chain structure of the produced antibody.

Furthermore, the modification of a sugar chain structure of a produced glycoprotein has been attempted by introducing a gene encoding an enzyme relating to the modification of a sugar chain [J. Biol. Chem., 261, 13848 (1989), Science, 252, 1668 (1991)]. When an antibody is expressed by using a CHO cell into which β1,4-N-acetylglucosamine transferase III (GnTIII) is introduced, the antibody had ADCC activity 16 times higher than the antibody expressed by using the parent cell [Glycobiology, 5, 813 (1995), WO99/54342]. However, since it has been reported that excess expression of GnTIII or β-1,4-N-acetylglucosamine transferase V (GnTV) shows toxicity for CHO cells, it is not suitable for the production of therapeutic antibodies.

It has been reported that a glycoprotein having changed sugar chain structure can be produced when a mutant in which the activity of a gene encoding an enzyme relating to the modification of a sugar chain is used as a host cell [J. Immunol., 160, 3393 (1998)]. It has been recently reported that an antibody having high ADCC activity can be produced using a cell line having reduced expression of GDP-mannose 4,6-dehydratase (hereinafter referred to as "GMD") which is an enzyme relating to biosynthesis of an intracellular sugar nucleotide, GDP-fucose, and such cell line includes, for example, a CHO cell line Lecl3 [J. Biol. Chem., 277, 26733 (2002)].

Since a mutation is introduced at random by a mutagen treatment in these cell lines, they are not appropriate as cell lines used in the production of pharmaceutical preparations.

As is described above, attempts have been made for controlling the activity of an enzyme or protein relating to the modification of a sugar chain in a host cell in order to modify the sugar chain structure of a produced glycoprotein. However, since the modification mechanism of the sugar chain is various and complicated and the physiological functions of the sugar chain have not been sufficiently solved, trial and error are repeated at present.

As the enzyme relating to the modification of a sugar chain in which 1-position of fucose is bound to 6-position of N-acetylglucosamine in the reducing end through α-bond in a complex type N-glycoside-linked sugar chain, in the case of a mammal, the presence of α1,6-fucosyltransferase (FUT8) is known [Biochem. Biophys. Res. Commun., 72, 909 (1976)]. The gene structure of FUT8 (EC 2.4.1,68) was revealed in 1996 [WO 92/27303, J. Biol. Chem., 271, 27817 (1996), J. Biochem., 121, 626 (1997)].

Under such a situation, it has been reported that ADCC activity of the antibody itself is changed by the binding of fucose to N-acetylglucosamine in the reducing end of a complex type N-glycoside-linked sugar chain of immunoglobulin IgG, and relationship between the activity of α1,6-fucosyltransferase and the ADCC activity has been drawing attention [WO 02/31140, WO 00/61739, J. Biol. Chem., 278, 3466 (2003), J. Biol. Chem., 277, 26733 (2002)]. Specifically, it has been shown that 1) the ADCC activity of an antibody produced by a clone in which α1,6-fucosyltransferase is overexpressed is decreased, and 2) the antibody-dependent cellular cytotoxicity of an antibody produced by a clone in which one of the allele of α1,6-fucosyltransferase is disrupted is increased (WO 02/31140).

However, other than the above-mentioned gene disruption method by homologous recombination, no methods for artificially suppressing the function of an enzyme relating to the modification of a sugar chain in which 1-position of fucose is bound to 6-position of the N-acetylglucosamine in the reducing end through α-bond in a complex type N-glycoside-linked sugar chain have been known.

### Disclosure of the Invention

An object of the present invention is to provide a process for producing an antibody composition using a cell, which comprises using a cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced; the RNA used in the process; a DNA corresponding to the RNA; a cell into which the RNA or the DNA is introduced or expressed; a method for constructing the cell; and a method for suppressing the enzyme. The antibody composition produced by the process of the present invention has high effector functions and is useful as medicaments.

The present invention relates to the following (1) to (29):
(1) A process for producing an antibody composition using a cell, which comprises using a cell into which a double-stranded RNA comprising an RNA selected from the following (a) or (b) and its complementary RNA is introduced:
   (a) an RNA comprising the nucleotide sequence represented by any one of SEQ ID NOs:9 to 30;
   (b) an RNA consisting of a nucleotide sequence in which one or several nucleotide(s) is/are deleted, substituted, inserted and/or added in the nucleotide sequence represented by any one of SEQ ID NOs:9 to 30 and having activity of suppressing the function of an enzyme relating to the modification of a sugar chain in which 1-position of fucose is bound to 6-position of N-acetylglucosamine in the reducing end through α-bond in a complex type N-glycoside-linked sugar chain.
(2) The process according to (1), wherein the enzyme relating to the modification of a sugar chain in which 1-position of fucose is bound to 6-position of N-acetylglucosamine in the reducing end through α-bond in the N-glycoside-linked sugar chain is α1,6-fucosyltransferase.
(3) The process according to (2), wherein the α1,6-fucosyltransferase is a protein encoded by a DNA selected from the group consisting of the following (a) to (h):
   (a) a DNA comprising the nucleotide sequence represented by SEQ ID NO:1;
   (b) a DNA comprising the nucleotide sequence represented by SEQ ID NO:2;
   (c) a DNA comprising the nucleotide sequence represented by SEQ ID NO:3;
   (d) a DNA comprising the nucleotide sequence represented by SEQ ID NO:4;
   (e) a DNA which hybridizes with a DNA consisting of the nucleotide sequence represented by SEQ ID NO:1 under stringent conditions and encodes a protein having α1,6-fucosyltransferase activity;
   (f) a DNA which hybridizes with a DNA consisting of the nucleotide sequence represented by SEQ ID NO:2 under stringent conditions and encodes a protein having α1,6-fucosyltransferase activity;
   (g) a DNA which hybridizes with a DNA consisting of the nucleotide sequence represented by SEQ ID NO:3 under stringent conditions and encodes a protein having α1,6-fucosyltransferase activity;
   (h) a DNA which hybridizes with a DNA consisting of the nucleotide sequence represented by SEQ ID NO:4 under stringent conditions and encodes a protein having α1,6-fucosyltransferase activity.
(4) The process according to (2), wherein the α1,6-fucosyltransferase is a protein selected from the group consisting of the following (a) to (I):
   (a) a protein comprising the amino acid sequence represented by SEQ ID NO:5;
   (b) a protein comprising the amino acid sequence represented by SEQ ID NO:6;
   (c) a protein comprising the amino acid sequence represented by SEQ ID NO:7;
   (d) a protein comprising the amino acid sequence represented by SEQ ID NO:8;
   (e) a protein consisting of an amino acid sequence in which one or more amino acid(s) is/are deleted, substituted, inserted and/or added in the amino acid sequence represented by SEQ ID NO:5 and having α1,6-fucosyltransferase activity;
   (f) a protein consisting of an amino acid sequence in which one or more amino acid(s) is/are deleted, substituted, inserted and/or added in the amino acid sequence represented by SEQ ID NO:6 and having α1,6-fucosyltransferase activity;
   (g) a protein consisting of an amino acid sequence in which one or more amino acid(s) is/are deleted, substituted, inserted and/or added in the amino acid sequence represented by SEQ ID NO:7 and having α1,6-fucosyltransferase activity;
   (h) a protein consisting of an amino acid sequence in which one or more amino acid(s) is/are deleted, substituted, inserted and/or added in the amino acid sequence represented by SEQ ID NO:8 and having α1,6-fucosyltransferase activity;
   (i) a protein consisting of an amino acid sequence which has 80% or more homology to the amino acid sequence represented by SEQ ID NO:5 and having α1,6-fucosyltransferase activity;
   (j) a protein consisting of an amino acid sequence which has 80% or more homology to the amino acid sequence represented by SEQ ID NO:6 and having α1,6-fucosyltransferase activity;
   (k) a protein consisting of an amino acid sequence which has 80% or more homology to the amino acid sequence represented by SEQ ID NO:7 and having α1,6-fucosyltransferase activity;
   (l) a protein consisting of an amino acid sequence which has 80% or more homology to the amino acid sequence represented by SEQ ID NO:8 and having α1,6-fucosyltransferase activity.
(5) The process according to any one of (1) to (4), wherein the cell into which the RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced is a cell which is resistant to a lectin which recognizes a sugar chain structure in which 1-position of fucose is bound to 6-position of N-acetylglucosamine in the reducing end through α-bond in an N-glycoside-linked sugar chain.
(6) The process according to (5), wherein the cell is resistant to at least one lectin selected from the group consisting of the following (a) to (d):
   (a) a *Lens culinaris* lectin;
   (b) a *Pisum sativum* lectin;
   (c) a *Vicia faba* lectin;
   (d) an *Aleuria aurantia* lectin.
(7) The process according to any one of (1) to (6), wherein the cell is selected from the group consisting of a yeast cell, an animal cell, an insect cell and a plant cell.
(8) The process according to any one of (1) to (7), wherein the cell is a cell selected from the group consisting of the following (a) to (i):
   (a) a CHO cell derived from Chinese hamster ovary tissue;
   (b) a rat myeloma cell line YB2/3HL.P2.G11.16Ag.20 cell;
   (c) a mouse myeloma cell line NS0 cell;
   (d) a mouse myeloma cell line SP2/0-Ag14 cell;
   (e) a BHK cell derived from Syrian hamster kidney tissue;
   (f) an antibody-producing hybridoma cell;
   (g) a human leukemia cell line Namalwa cell;
   (h) an embryonic stem cell;
   (i) a fertilized egg cell.
(9) The process according to any one of (1) to (8), wherein the cell is a transformant into which a gene encoding an antibody molecule is introduced.
(10) The process according to (9), wherein the antibody molecule is selected from the group consisting of the following (a) to (d):
   (a) a human antibody;
   (b) a humanized antibody;
   (c) an antibody fragment comprising the Fc region of (a) or (b);
   (d) a fusion protein comprising the Fc region of (a) or (b).
(11) The process according to (9) or (10), wherein the antibody molecule belongs to an IgG class.
(12) The process according to any one of (1) to (11), wherein the antibody composition is an antibody composition having higher antibody-dependent cell-mediated cytotoxic activity than an antibody composition produced by a parent cell into which a double-stranded RNA comprising an RNA selected from the following (a) or (b) and its complementary RNA is not introduced:
   (a) an RNA comprising the nucleotide sequence represented by any one of SEQ ID NOs:9 to 30;
   (b) an RNA consisting of a nucleotide sequence in which one or several nucleotide(s) is/are deleted, substituted, inserted and/or added in the nucleotide sequence represented by any one of SEQ ID NOs:9 to 30 and having activity of suppressing the function of an enzyme relating to the modification of a sugar chain in which 1-position of fucose is bound to 6-position of N-acetylglucosamine in the reducing end through α-bond in a complex type N-glycoside-linked sugar chain.
(13) The process according to (12), wherein the antibody composition having higher antibody-dependent cell-mediated cytotoxic activity is an antibody composition which comprises antibody molecules having complex type N-glycoside-linked sugar chains in the Fc region, and in which a ratio of sugar chains in which fucose is not bound to N-acetylglucosamine in the reducing end in the sugar chains among the complex type N-glycoside-linked sugar chains is higher than that of an antibody composition produced by the parent cell.
(14) The process according to (13), wherein the complex type N-glycoside-linked sugar chains are sugar chains in which 1 position of fucose is not bound to 6 position of N-acetylglucosamine in the reducing end through α-bond in the sugar chains.
(15) The process according to any one of (12) to (14), wherein the antibody composition having higher antibody-dependent cell-mediated cytotoxic activity is an antibody composition which comprises antibody molecules having complex type N-glycoside-linked sugar chains in the Fc region, and in which the ratio of sugar chains in which fucose is not bound to N-acetylglucosamine in the reducing end in the sugar chains among the complex type N-glycoside-linked sugar chains is 20% or more.
(16) The process according to any one of (12) to (15), wherein the antibody composition having higher antibody-dependent cell-mediated cytotoxic activity is an antibody composition which comprises antibody molecules having complex type N-glycoside-linked sugar chains in the Fc region, and in which the complex type N-glycoside-linked sugar chains are sugar chains in which fucose is not bound to N-acetylglucosamine in the reducing end.
(17) A cell into which an RNA capable of suppressing the function of an enzyme relating to the modification of a sugar chain in which 1-position of fucose is bound to 6-position of N-acetylglucosamine in the reducing end through α-bond in a complex type N-glycoside-linked sugar chain is introduced, and which is used in the process according to any one of (1) to (16).
(18) The cell according to (17), wherein the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.
(19) A cell in which an RNA selected from RNAs of the group consisting of the nucleotide sequences represented by any one of SEQ ID NOs:9 to 30 is introduced or expressed.
(20) A double-stranded RNA consisting of an RNA selected from the following (a) or (b) and its complementary RNA:
   (a) an RNA comprising the nucleotide sequence represented by any one of SEQ ID NOs:9 to 30;
   (b) an RNA consisting of a nucleotide sequence in which one or several nucleotide(s) is/are deleted, substituted, inserted and/or added in the nucleotide sequence represented by any one of SEQ ID NOs:9 to 30 and having activity of suppressing the function of an enzyme relating to the modification of a sugar chain in which 1-position of fucose is bound to 6-position of N-acetylglucosamine in the reducing end through α-bond in a complex type N-glycoside-linked sugar chain.
(21) A DNA corresponding to the RNA described in (20) and a complementary DNA to the DNA.
(22) A recombinant DNA which is obtainable by introducing a DNA corresponding to the RNA described in the above (20) and a complementary DNA to the DNA into a vector.
(23) The recombinant DNA according to (22), which expresses the double-stranded RNA according to (20).
(24) A transformant which is obtainable by introducing the recombinant DNA according to (22) or (23) into a cell.
(25) A method for constructing a cell which is resistant to a lectin which recognizes a sugar chain structure in which 1-position of fucose is bound to 6-position of N-acetylglucosamine in the reducing end through α-bond in a complex type N-glycoside-linked sugar chain, which comprises introducing or expressing the double-stranded RNA described in (20) in a cell.
(26) The method according to the above (25), wherein the cell which is resistant to a lectin which recognizes a sugar chain structure in which 1-position of fucose is bound to 6-position of N-acetylglucosamine in the reducing end through α-bond in a complex type N-glycoside-linked sugar chain is resistant to at least one lectin selected from the group consisting of the following (a) to (d):
   (a) a *Lens culinaris* lectin;
   (b) a *Pisum sativum* lectin;
   (c) a *Vicia faba* lectin;
   (d) an *Aleuria aurantia* lectin.
(27) A method for suppressing the function of an enzyme relating to the modification of a sugar chain in which 1-position of fucose is bound to 6-position of N-acetylglucosamine in the reducing end through α-bond in a complex type N-glycoside-linked sugar chain, which comprises using an RNA selected from RNAs of the group consisting of the nucleotide sequences of any one of SEQ ID NOs:9 to 30.
(28) The method according to the above (27), wherein the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.

The present invention is described below in detail. This application is based on the priority of Japanese patent application No. 2003-350167 filed on October 9, 2003, and the entire contents of the specification and the drawings in the patent application are incorporated hereinto by reference.

The present invention relates to a process for producing an antibody composition using a cell, which comprises using a cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced; the RNA used in the process; a DNA corresponding to the RNA; a cell in which the RNA or the DNA is introduced or expressed; a method for constructing the cell; and a method for suppressing the enzyme.

The process for producing an antibody composition using a cell includes a process for producing a monoclonal antibody using a hybridoma cell, a process for producing a human antibody and a humanized antibody using a host cell into which a gene encoding an antibody is introduced, a process for producing a human antibody using a transgenic non-human animal which is developed after transplanting a non-human embryonic stem cell or fertilized egg cell into which a gene encoding an antibody is introduced into a non-human animal early stage embryo; a process for producing à human antibody or a humanized antibody by using a transgenic plant obtained from a plant callus cell into which a gene encoding an antibody is introduced; and the like.

The cell used in the present invention may be any cell, so long as it can express an antibody molecule. Examples include an yeast, an animal cell, an insect cell, a plant cell and the like, and an animal cell is preferred. Examples of the animal cell include a CHO cell derived from a Chinese hamster ovary tissue, a rat myeloma cell line YB2/3HL.P2.G11.16Ag.20 cell, a mouse myeloma cell line NS0 cell, a mouse myeloma SP2/0-Ag14 cell, a BHK cell derived from a syrian hamster kidney tissue, an antibody-producing-hybridoma cell, a human leukemia cell line Namalwa cell, an embryonic stem cell, a fertilized egg cell, and the like.

The cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain in the present invention is resistant to a lectin which recognizes a sugar chain structure in which 1-position of fucose is bound to 6-position of N-acetylglucosamine in the reducing end through α-bond in an N-glycoside-linked sugar chain.

Accordingly, in the present invention, as the cell resistant to a lectin which recognizes a sugar chain in which 1-position of fucose is bound to 6-position of N-acetylglucosamine in the reducing end through α-bond in a complex N-glycoside-linked sugar chain, any cell can be used, so long as it is a cell such as an yeast, an animal cell, an insect cell or a plant cell which can be used for producing an antibody composition and is a cell resistant to a lectin which recognizes a sugar chain in which 1-position of fucose is bound to 6-position of N-acetylglucosamine in the reducing end through α-bond in a complex N-glycoside-linked sugar chain. Examples include a hybridoma cell, a host cell for producing a human antibody and humanized antibody, an embryonic stem cell and fertilized egg cell for producing a transgenic non-human animal which produces a human antibody, a plant callus cell for producing a transgenic plant which produces a human antibody, a myeloma cell, a cell derived from a transgenic non-human animal and the like which are resistant to lectin which recognizes a sugar chain in which 1-position of fucose is bound to 6-position of N-acetylglucosamine in the reducing end through α-bond in a complex N-glycoside-linked sugar chain. The myeloma cell which is derived from the transgenic non-human animal of the present invention can be used as a fusion cell for producing a hybridoma cell. Also, a hybridoma cell can be produced by immunizing a transgenic non-human animal with an antigen and using spleen cells of the animal.

The lectin-resistant cell is a cell of which growth is not inhibited when a lectin is applied at an effective concentration.

In the present invention, the effective concentration of lectin that does not inhibit growth may be appropriately determined according to each cell line used as the parent cell. It is usually 10 µg/mL to 10 mg/mL, preferably 0.5 mg/mL to 2.0 mg/mL. When an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced into a parent cell, the effective concentration is a concentration higher than the lowest concentration that does not allow the normal growth of a parent cell line, preferably equal to the lowest concentration that does not allow the normal growth of the parent cell, more preferably 2 to 5 times, further preferably 10 times, most preferably 20 or more times the lowest concentration that does not allow the normal growth of the parent cell.

The parent cell means a cell prior to the introduction of an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain.

Although the parent cell is not particularly limited, the following cells are exemplified.

The parent cell of NS0 cell includes NS0 cells described in literatures such as BIO/TECHNOLOGY, 10, 169 (1992) and Biotechnol. Bioeng., 73, 261 (2001), NS0 cell line (RCB 0213) registered at RIKEN Cell Bank, The Institute of Physical and Chemical Research, sub-cell lines obtained by acclimating these cell lines to media in which they can grow, and the like.

The parent cell of SP2/0-Ag14 cell includes SP2/0-Ag14 cells described in literatures such as J. Immunol., 126, 317 (1981), Nature, 276, 269 (1978) and *Human* Antibodies and Hybridomas, 3, 129 (1992), SP2/0-Ag14 cell (ATCC CRL-1581) registered at ATCC, sub-cell lines obtained by acclimating these cell lines to media in which they can grow (ATCC CRL-1581.1), and the like.

The parent cell of CHO cell derived from Chinese hamster ovary tissue includes CHO cells described in literatures such as Journal of Experimental Medicine (Jikken Igaku), 108, 945 (1958), Proc. Natl. Acad. Sci. USA, 60, 1275 (1968), Genetics, 55, 513 (1968), Chromosoma, 41, 129 (1973), Methods in Cell Science, 18, 115 (1996), Radiation Research, 148, 260 (1997), Proc. Natl. Acad. Sci. USA, 77, 4216 (1980), Proc. Natl. Acad. Sci. USA, 60, 1275 (1968), Cell, 6, 121 (1975) *and* Molecular Cell Genetics, Appendix I, II (p. 883-900), cell line CHO-K1 (ATCC CCL-61), cell line DUXB11 (ATCC CRL-9096) and cell line Pro-5 (ATCC CRL-1781) registered at ATCC, commercially available cell line CHO-S (Cat # 11619 of Life Technologies), sub-cell lines obtained by acclimating these cell lines to media in which they can grow, and the like.

The parent cell of a rat myeloma cell line YB2/3HL.P2.G11.16Ag.20 cell includes cell lines established from Y3/Ag1.2.3 cell (ATCC CRL-1631) such as YB2/3HL.P2.G11.16Ag.20 cell described in literatures such as J. Cell. Biol., 93, 576 (1982) and Methods Enzymol., 73B, 1 (1981), YB2/3HL.P2.G11.16Ag.20 cell (ATCC CRL-1662) registered at ATCC, sub-lines obtained by acclimating these cell lines to media in which they can grow, and the like.

As the lectin which recognizes a sugar chain structure in which 1-position of fucose is bound to 6-position of *N*-acetylglucosamine in the reducing end through α-bond in the *N*-glycoside-linked sugar chain, any lectin can be used, so long as it can recognize the sugar chain structure. Examples include a *Lens culinaris* lectin LCA (lentil agglutinin derived from *Lens culinaris*), a pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.

The enzyme relating to the modification of a sugar chain in which 1-position of fucose is bound to 6-position of N-acetylglucosamine in the reducing end through α-bond in a complex type N-glycoside-linked sugar chain includes an enzyme relating to the modification of a sugar chain in which 1-position of fucose is bound to 6-position of N-acetylglucosamine in the reducing end through α-bond in a complex type N-glycoside-linked sugar chain.

Specific examples of the enzyme relating to the modification of a sugar chain in which 1-position of fucose is bound to 6-position of N-acetylglucosamine in the reducing end through α-bond in a complex type N-glycoside-linked sugar chain include α1,6-fucosyltransferase and the like.

In the present invention, the α1,6-fucosyltransferase includes a protein encoded by a DNA of the following (a) to (h), a protein of the following (i) to (t), and the like:
(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:3;
(d) a DNA comprising the nucleotide sequence represented by SEQ ID NO:4;
(e) a DNA which hybridizes with DNA consisting of the nucleotide sequence represented by SEQ ID NO:1 under stringent conditions and which encodes a protein having α1,6-fucosyltransferase activity;
(f) a DNA which hybridizes with a DNA consisting of the nucleotide sequence represented by SEQ ID NO:2 under stringent conditions and which encodes a protein having α1,6-fucosyltransferase activity;
(g) a DNA which hybridizes with a DNA consisting of the nucleotide sequence represented by SEQ ID NO:3 under stringent conditions and which encodes a protein having α1,6-fucosyltransferase activity;
(h) a DNA which hybridizes with a DNA consisting of the nucleotide sequence represented by SEQ ID NO:4 under stringent conditions and which encodes a protein having α1,6-fucosyltransferase activity; or
(i) a protein comprising the amino acid sequence represented by SEQ ID NO:5;
(j) a protein comprising the amino acid sequence represented by SEQ ID NO:6;
(k) a protein comprising the amino acid sequence represented by SEQ ID NO:7;
(l) a protein comprising the amino acid sequence represented by SEQ ID NO:8;
(m) a protein consisting of an amino acid sequence in which one or more amino acid(s) is/are deleted, substituted, inserted and/or added in the amino acid sequence represented by SEQ ID NO:5 and having α1,6-fucosyltransferase activity;
(n) a protein consisting of an amino acid sequence in which one or more amino acid(s) is/are deleted, substituted, inserted and/or added in the amino acid sequence represented by SEQ ID NO:6 and having α1,6-fucosyltransferase activity;
(o) a protein consisting of an amino acid sequence in which one or more amino acid(s) is/are deleted, substituted, inserted and/or added in the amino acid sequence represented by SEQ ID NO:7 and having α1,6-fucosyltransferase activity;
(p) a protein consisting of an amino acid sequence in which one or more amino acid(s) is/are deleted, substituted, inserted and/or added in the amino acid sequence represented by SEQ ID NO:8 and having α1,6-fucosyltransferase activity;
(q) a protein consisting of an amino acid sequence which has 80% or more homology to the amino acid sequence represented by SEQ ID NO:5 and having α1,6-fucosyltransferase activity;
(r) a protein consisting of an amino acid sequence which has 80% or more homology to the amino acid sequence represented by SEQ ID NO:6 and having α1,6-fucosyltransferase activity;
(s) a protein consisting of an amino acid sequence which has 80% or more homology to the amino acid sequence represented by SEQ ID NO:7 and having α1,6-fucosyltransferase activity;
(t) a protein consisting of an amino acid sequence which has 80% or more homology to the amino acid sequence represented by SEQ ID NO:8 and having α1,6-fucosyltransferase activity.

In the present invention, the DNA which hybridizes under stringent conditions refers to a DNA which is obtained by colony hybridization, plaque hybridization, Southern hybridization or the like using, for example, a DNA consisting of the nucleotide sequence represented by SEQ ID NO:1, 2, 3 or 4 or a fragment thereof as a probe. A specific example of such DNA is a DNA which can be identified by performing hybridization at 65°C in the presence of 0.7 to 1.0 M sodium chloride using a filter with colony- or plaque-derived DNA immobilized thereon, and then washing the filter at 65°C with a 0.1 to 2-fold concentration SSC solution (1-fold concentration SSC solution: 150 mM sodium chloride and 15 mM sodium citrate). Hybridization can be carried out according to the methods described in Molecular Cloning, A Laboratory Manual, Second Edition, Cold Spring Harbor Lab. Press (1989), Current Protocols in Molecular Biology, John Wiley & Sons (1987-1997); DNA Cloning 1: Core Techniques, A Practical Approach, Second Edition, Oxford University (1995); and the like. Specifically, the DNA capable of hybridization under stringent conditions includes DNA having at least 60% or more homology, preferably 70% or more homology, more preferably 80% or more homology, further preferably 90% or more homology, particularly preferably 95% or more homology, most preferably 98% or more homology to the nucleotide sequence represented by SEQ ID NO:1, 2, 3 or 4.

In the present invention, the protein consisting of an amino acid sequence in which one or more amino acid(s) is/are deleted, substituted, inserted and/or added in the amino acid sequence represented by SEQ ID NO:5, 6, 7 or 8 and having α1,6-fucosyltransferase activity can be obtained, e.g., by introducing a site-directed mutation into a DNA encoding a protein consisting of the amino acid sequence represented by SEQ ID NO:5, 6, 7 or 8, respectively, by the site-directed mutagenesis described, e.g., in Molecular Cloning: A Laboratory Manual, Cold Spring Harbor Lab. Press New York (1989); Current Protocols in Molecular Biology, John Wiley & Sons (1987-1997); Nucleic Acids Research, 10, 6487 (1982); Proc. Natl. Acad Sci. USA, 79, 6409 (1982); Gene, 34, 315 (1985); Nucleic Acids Research, 13, 4431 (1985); Proc. Natl. Acad. Sci. USA, 82, 488 (1985); and the like.

The number of amino acid residues which are deleted, substituted, inserted and/or added is one or more, and is not specifically limited, but it is within the range where deletion, substitution or addition is possible by known methods such as the above site-directed mutagenesis. The suitable number is 1 to dozens, preferably 1 to 20, more preferably 1 to 10, further preferably 1 to 5.

Also, in the present invention, the protein consisting of an amino acid sequence which has 80% or more homology to the amino acid sequence represented by SEQ ID NO:5, 6, 7 or 8 and having α1,6-fucosyltransferase activity includes a protein having at least 80% or more homology, preferably 85% or more homology, more preferably 90% or more homology, further preferably 95% or more homology, particularly preferably 97% or more homology, most preferably 99% or more homology to the amino acid sequence represented by SEQ ID NO:5, 6, 7 or 8, respectively, as calculated by use of analysis software such as BLAST [J. Mol. Biol., 215, 403 (1990)] or FASTA *[*Methods in Enzymology, 183, 63 (1990)].

In the present invention, regarding the length of the RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain, a continuous RNA of 10 to 40, preferably 10 to 35, and more preferably 15 to 29, as exemplified below, are mentioned.

### Examples include:

(a) an RNA corresponding to a DNA consisting of a nucleotide sequence represented by a sequence of continued 10 to 40 bases in a region which does not contain continued 5 or more adenine or thymidine bases in the nucleotide sequence represented by SEQ ID NO:1;
(b) an RNA corresponding to a DNA consisting of a nucleotide sequence represented by a sequence of continued 10 to 40 bases in a region which does not contain continued 5 or more adenine or thymidine bases in the nucleotide sequence represented by SEQ ID NO:2;
(c) an RNA corresponding to a DNA consisting of a nucleotide sequence represented by a sequence of continued 10 to 40 bases in a region which does not contain continued 5 or more adenine or thymidine bases in the nucleotide sequence represented by SEQ ID NO:3; and
(d) an RNA corresponding to a DNA consisting of a nucleotide sequence represented by a sequence of continued 10 to 40 bases in a region which does not contain continued 5 or more adenine or thymidine bases in the nucleotide sequence represented by SEQ ID NO:4.

### Specific examples include:

(e) an RNA comprising the nucleotide sequence represented by SEQ ID NO:9;
(f) an RNA consisting of a nucleotide sequence in which one or several nucleotide(s) is/are deleted, substituted, inserted and/or added in the nucleotide sequence represented by SEQ ID NO:9 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(g) an RNA comprising the nucleotide sequence represented by SEQ ID NO:10;
(h) an RNA consisting of a nucleotide sequence in which one or several nucleotide(s) is/are deleted, substituted, inserted and/or added in the nucleotide sequence represented by SEQ ID NO:10 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(i) an RNA comprising the nucleotide sequence represented by SEQ ID NO:11;
(j) an RNA consisting of a nucleotide sequence in which one or several nucleotide(s) is/are deleted, substituted, inserted and/or added in the nucleotide sequence represented by SEQ ID NO:11 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(k) an RNA comprising the nucleotide sequence represented by SEQ ID NO:12;
(l) an RNA consisting of a nucleotide sequence in which one or several nucleotide(s) is/are deleted, substituted, inserted and/or added in the nucleotide sequence represented by SEQ ID NO:12 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(m) an RNA comprising the nucleotide sequence represented by SEQ ID NO:13;
(n) an RNA consisting of a nucleotide sequence in which one or several nucleotide(s) is/are deleted, substituted, inserted and/or added in the nucleotide sequence represented by SEQ ID NO:13 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(o) an RNA comprising the nucleotide sequence represented by SEQ ID NO:14;
(p) an RNA consisting of a nucleotide sequence in which one or several nucleotide(s) is/are deleted, substituted, inserted and/or added in the nucleotide sequence represented by SEQ ID NO:14 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(q) an RNA comprising the nucleotide sequence represented by SEQ ID NO:15;
(r) an RNA consisting of a nucleotide sequence in which one or several nucleotide(s) is/are deleted, substituted, inserted and/or added in the nucleotide sequence represented by SEQ ID NO:15 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(s) an RNA comprising the nucleotide sequence represented by SEQ ID NO:16;
(t) an RNA consisting of a nucleotide sequence in which one or several nucleotide(s) is/are deleted, substituted, inserted and/or added in the nucleotide sequence represented by SEQ ID NO:16 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(u) an RNA comprising the nucleotide sequence represented by SEQ ID NO:17;
(v) an RNA consisting of a nucleotide sequence in which one or several nucleotide(s) is/are deleted, substituted, inserted and/or added in the nucleotide sequence represented by SEQ ID NO:17 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(w) an RNA comprising the nucleotide sequence represented by SEQ ID NO:18;
(x) an RNA consisting of a nucleotide sequence in which one or several nucleotide(s) is/are deleted, substituted, inserted and/or added in the nucleotide sequence represented by SEQ ID NO:18 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
(y) an RNA comprising the nucleotide sequence represented by SEQ ID NO:19;
(z) an RNA consisting of a nucleotide sequence in which one or several nucleotide(s) is/are deleted, substituted, inserted and/or added in the nucleotide sequence represented by SEQ ID NO:19 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (A) an RNA comprising the nucleotide sequence represented by SEQ ID NO:20;
   (B) an RNA consisting of a nucleotide sequence in which one or several nucleotide(s) is/are deleted, substituted, inserted and/or added in the nucleotide sequence represented by SEQ ID NO:20 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (C) an RNA comprising the nucleotide sequence represented by SEQ ID NO:21;
   (D) an RNA consisting of a nucleotide sequence in which one or several nucleotide(s) is/are deleted, substituted, inserted and/or added in the nucleotide sequence represented by SEQ ID NO:21 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (E) an RNA comprising the nucleotide sequence represented by SEQ ID NO:22;
   (F) an RNA consisting of a nucleotide sequence in which one or several nucleotide(s) is/are deleted, substituted, inserted and/or added in the nucleotide sequence represented by SEQ ID NO:22 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (G) an RNA comprising the nucleotide sequence represented by SEQ ID NO:23;
   (H) an RNA consisting of a nucleotide sequence in which one or several nucleotide(s) is/are deleted, substituted, inserted and/or added in the nucleotide sequence represented by SEQ ID NO:23 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (I) an RNA comprising the nucleotide sequence represented by SEQ ID NO:24;
   (J) an RNA consisting of a nucleotide sequence in which one or several nucleotide(s) is/are deleted, substituted, inserted and/or added in the nucleotide sequence represented by SEQ ID NO:24 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (K) an RNA comprising the nucleotide sequence represented by SEQ ID NO:25;
   (L) an RNA consisting of a nucleotide sequence in which one or several nucleotide(s) is/are deleted, substituted, inserted and/or added in the nucleotide sequence represented by SEQ ID NO:25 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (M) an RNA comprising the nucleotide sequence represented by SEQ ID NO:26;
   (N) an RNA consisting of a nucleotide sequence in which one or several nucleotide(s) is/are deleted, substituted, inserted and/or added in the nucleotide sequence represented by SEQ ID NO:26 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (O) an RNA comprising the nucleotide sequence represented by SEQ ID NO:27;
   (P) an RNA consisting of a nucleotide sequence in which one or several nucleotide(s) is/are deleted, substituted, inserted and/or added in the nucleotide sequence represented by SEQ ID NO:27 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (Q) an RNA comprising the nucleotide sequence represented by SEQ ID NO:28;
   (R) an RNA consisting of a nucleotide sequence in which one or several nucleotide(s) is/are deleted, substituted, inserted and/or added in the nucleotide sequence represented by SEQ ID NO:28 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (S) an RNA comprising the nucleotide sequence represented by SEQ ID NO:29;
   (T) an RNA consisting of a nucleotide sequence in which one or several nucleotide(s) is/are deleted, substituted, inserted and/or added in the nucleotide sequence represented by SEQ ID NO:29 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain;
   (U) an RNA comprising the nucleotide sequence represented by SEQ ID NO:30; and
   (V) an RNA consisting of a nucleotide sequence in which one or several nucleotide(s) is/are deleted, substituted, inserted and/or added in the nucleotide sequence represented by SEQ ID NO:30 and having activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain.

Regarding the nucleotide sequence in which one or several nucleotide(s) is/are deleted, substituted, inserted and/or added in the nucleotide sequence represented by any one of SEQ ID NOs:9 to 30, a double-stranded RNA caused by the deletion, substitution, insertion and/or addition of the nucleotide may be an RNA in which the nucleotide is deleted, substituted, inserted and/or added in only one of the strands, that is, the double-stranded RNA may be an incomplete complementary strand, so long as it has activity of suppressing the function of an enzyme relating to an enzyme relating to the modification of a sugar chain in which 1-position of fucose is bound to 6-position of N-acetylglucosamine in the reducing end through α-bond in a complex type N-glycoside-linked sugar chain.

In the present invention, the antibody composition is a composition which comprises an antibody molecule having a complex type N-glycoside-linked sugar chain in the Fc region.

The antibody is a tetramer in which two molecules of each of two polypeptide chains, a heavy chain and a light chain (hereinafter referred to as "H chain" and "L chain", respectively), are respectively associated. Each of about a quarter of the N-terminal side of the H chain and about a half of the N-terminal side of the L chain (more than 100 amino acids for each) is called V region which is rich in diversity and directly relates to the binding with an antigen. The greater part of the moiety other than the V region is called a constant region (hereinafter referred to as "C region"). Based on homology with the C region, antibody molecules are classified into classes IgG, IgM, IgA, IgD and IgE.

Also, the IgG class is further classified, for example about a human, into subclasses IgG1 to IgG4 based on homology with the C region.

The H chain is divided into four immunoglobulin domains, an antibody H chain V region (hereinafter referred to as "VH"), an antibody H chain C region 1 (hereinafter referred to as "CH1"), an antibody H chain C region 2 (hereinafter referred to as "CH2") and an antibody H chain C region (hereinafter referred to as "CH3"), from its N-terminal side, and a highly flexible peptide region called hinge region is present between CH1 and CH2 to divide CH1 and CH2. A structural unit comprising CH2 and CH3 under the downstream of the hinge region is called Fc region to which a complex *N*-glycoside-linked sugar chain is bound. Fc region is a region to which an Fc receptor, a complement and the like are bound (Immunology Illustrated, the Original, 5th edition, published on February 10, 2000, by Nankodo ; Handbook of Antibody Technology (Kotai Kogaku Nyumon), 1st edition on January 25, 1994, by Chijin Shokan).

Sugar chains of glycoproteins such as an antibody are roughly classified into two types, namely a sugar chain which binds to asparagine (N-glycoside-linked sugar chain) and a sugar chain which binds to other amino acid such as serine, threonine (O-glycoside-linked sugar chain), based on the binding form to the protein moiety.

In the present invention, the N-glycoside-linked sugar chains are shown by the following chemical formula 1.

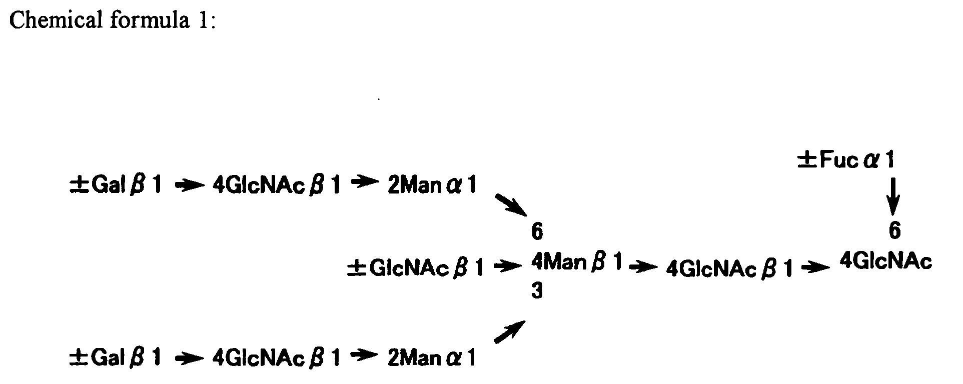

In chemical formula 1, the sugar chain terminus which binds to asparagine is called a reducing end, and the opposite side is called a non-reducing end.

The N-glycoside-linked sugar chain may be any N-glycoside-linked sugar chain, so long as it comprises the core structure of chemical formula 1. Examples include a high mannose type in which mannose alone binds to the non-reducing end of the core structure; a complex type in which the non-reducing end side of the core structure comprises at least one parallel branches of galactose-N-acetylglucosamine (hereinafter referred to as "Gal-GlcNAc") and the non-reducing end side of Gal-GlcNAc comprises a structure of sialic acid, bisecting N-acetylglucosamine or the like; a hybrid type in which the non-reducing end side of the core structure comprises branches of both of the high mannose type and complex type; and the like.

Since the Fc region in the antibody molecule comprises positions to which N-glycoside-linked sugar chains are separately bound, two sugar chains are bound per one antibody molecule. Since the N-glycoside-linked sugar chain which binds to an antibody molecule includes any sugar chain having the core structure represented by chemical formula 1, there are a number of combinations of sugar chains for the two N-glycoside-linked sugar chains which bind to the antibody.

Accordingly, in the present invention, an antibody composition produced by using a cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced may comprise an antibody having the same sugar chain structure or an antibody having different sugar chain structures, so long as the effect of the present invention is obtained from the composition.

The ratio of a sugar chain in which fucose is not bound to N-acetylglucosamine in the reducing end in the sugar chain among the total complex N-glycoside-linked sugar chains bound to the Fc region contained in the antibody composition (hereinafter referred to the "ratio of sugar chains of the present invention") is a ratio of the number of a sugar chain in which fucose is not bound to N-acetylglucosamine in the reducing end in the sugar chain to the total number of the complex N-glycoside-linked sugar chains bound to the Fc region contained in the composition.

The sugar chain in which fucose is not bound to N-acetylglucosamine in the reducing end in the complex N-glycoside-linked sugar chain is a sugar chain in which fucose is not bound to N-acetylglucosamine in the reducing end through α-bond in the complex N-glycoside-linked sugar chain. Specifically, it is a complex N-glycoside-linked sugar chain in which 1-position of fucose is not bound to 6-position of N-acetylglucosamine through α-bond. The higher the ratio of sugar chains of the present invention is, the higher the ADCC activity of the antibody composition is.

The antibody composition having higher ADCC activity includes an antibody composition in which the ratio of sugar chains of the present invention is preferably 20% or more, more preferably 30% or more, still more preferably 40% or more, particularly preferably 50% or more, and most preferably 100%.

Furthermore, the present invention relates to a process for producing an antibody composition having higher ADCC activity than an antibody composition produced by its parent cell.

The ADCC activity is a cytotoxic activity in which an antibody bound to a cell surface antigen on a tumor cell *in vivo* activate an effector cell through an Fc receptor existing on the antibody Fc region and effector cell surface and thereby obstruct the tumor cell and the like [Monoclonal Antibodies: Principles and Applications, Wiley-Liss, Inc., Chapter 2.1 (1995)]. The effector cell includes a killer cell, a natural killer cell, an activated macrophage and the like.

The ratio of sugar chains in which fucose is not bound to N-acetylglucosamine in the reducing end in the sugar chains contained in the composition which comprises an antibody molecule having complex type N-glycoside-linked sugar chains in the Fc region can be determined by releasing the sugar chain from the antibody molecule, carrying out fluorescence labeling or radioisotope labeling of the released sugar chain and then separating the labeled sugar chain by chromatography using a known method such as hydrazinolysis or enzyme digestion *[Biochemical Experimentation Methods 23 Method for Studying Glycoprotein Sugar Chain* (Japan Scientific Societies Press), edited by Reiko Takahashi (1989)]. Also, the released sugar chain can also be determined by analyzing it with the HPAED-PAD method [*J. Liq. Chromatogr.,* 6*,* 1577 (1983)].

The antibody molecule may be any antibody molecule, so long as it comprises the Fc region of an antibody. Examples include an antibody, an antibody fragment, a fusion protein comprising an Fc region, and the like.

Examples of the antibody include an antibody secreted by a hybridoma cell prepared from a spleen cell of an animal immunized with an antigen; an antibody prepared by a genetic recombination technique, namely an antibody obtained by introducing an antibody gene-inserted antibody expression vector into a host cell; and the like. Specific examples include an antibody produced by a hybridoma, a humanized antibody, a human antibody and the like.

A hybridoma is a cell which is obtained by cell fusion between a B cell obtained by immunizing a non-human mammal with an antigen and a myeloma cell derived from a mouse, a rat or the like and which can produce a monoclonal antibody having the antigen specificity of interest.

The humanized antibody includes a human chimeric antibody, a human CDR-grafted antibody and the like.

A human chimeric antibody is an antibody which comprises H chain V region (hereinafter referred to as "HV" or "VH") and L chain V region (hereinafter referred to as "LV" or "VL"), both of a non-human animal antibody, a human antibody H chain C region (hereinafter also referred to as "CH") and a human antibody L chain C region (hereinafter also referred to as "CL"). The non-human animal may be any animal such as mouse, rat, hamster or rabbit, so long as a hybridoma can be prepared therefrom.

The human chimeric antibody can be produced by obtaining cDNAs encoding VH and VL from a monoclonal antibody-producing hybridoma, inserting them into an expression vector for host cell having genes encoding human antibody CH and human antibody CL to thereby construct a human chimeric antibody expression vector, and then introducing the vector into a host cell to express the antibody.

As the CH of human chimeric antibody, any CH can be used, so long as it belongs to human immunoglobulin (hereinafter referred to as "hIg") can be used, and those belonging to the hIgG class are preferred, and any one of the subclasses belonging to the hIgG class, such as hIgG1, hIgG2, hIgG3 and hIgG4, can be used. As the CL of human chimeric antibody, any CL can be used, so long as it belongs to the hIg class, and those belonging to the κ class or λ class can be used.

A human CDR-grafted antibody is an antibody in which amino acid sequences of CDRs of VH and VL of a non-human animal antibody are grafted into appropriate positions of VH and VL of a human antibody.

The human CDR-grafted antibody can be produced by constructing cDNAs encoding V regions in which CDRs of VH and VL of a non-human animal antibody are grafted into CDRs of VH and VL of a human antibody, inserting them into an expression vector for host cell having genes encoding human antibody CH and human antibody CL to thereby construct a human CDR-grafted antibody expression vector, and then introducing the expression vector into a host cell to express the human CDR-grafted antibody.

As the CH of human CDR-grafted antibody, any CH can be used, so long as it belongs to the hIg, and those of the hIgG class are preferred and any one of the subclasses belonging to the hIgG class, such as hIgG1, hIgG2, hIgG3 and hJgG4, can be used. As the CL of human CDR-grafted antibody, any CL can be used, so long as it belongs to the hIg class, and those belonging to the κ class or λ class can be used.

A human antibody is originally an antibody naturally existing in the human body, but it also includes antibodies obtained from a human antibody phage library, a human antibody-producing transgenic non-transgenic animal and a human antibody-producing transgenic plant, which are prepared based on the recent advance in genetic engineering, cell engineering and embryological 1 engineering techniques.

The antibody existing in the human body can be prepared, for example by isolating a human peripheral blood lymphocyte, immortalizing it by its infection with EB virus or the like and then cloning it to thereby obtain lymphocytes capable of producing the antibody, culturing the lymphocytes thus obtained, and purifying the antibody from the culture.

The human antibody phage library is a library in which antibody fragments such as Fab and single chain antibody are expressed on the phage surface by inserting a gene encoding an antibody prepared from a human B cell into a phage gene. A phage expressing an antibody fragment having the desired antigen binding activity can be recovered from the library, using its activity to bind to an antigen-immobilized substrate as the marker. The antibody fragment can be converted further into a human antibody molecule comprising two full H chains and two full L chains by genetic engineering techniques.

A human antibody-producing transgenic non-human animal is an animal in which a human antibody gene is introduced into cells. Specifically, a human antibody-producing transgenic non-human animal can be prepared by introducing a human antibody gene into ES cell of a mouse, transplanting the ES cell into an early stage embryo of other mouse and then developing it. By introducing a human chimeric antibody gene into a fertilized egg and developing it, the transgenic non-human animal can be also prepared. A human antibody is prepared from the human antibody-producing transgenic non-human animal by obtaining a human antibody-producing hybridoma by a hybridoma preparation method usually carried out in non-human mammals, culturing the obtained hybridoma and accumulating the human antibody in the culture.

The transgenic non-human animal includes a cattle, a sheep, a goat, a pig, a horse, a mouse, a rat, a fowl, a monkey, a rabbit and the like.

In the present invention, as the antibody, preferred are an antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen, and a human antibody which belongs to the IgG class is preferred.

An antibody fragment is a fragment which comprises at least a part of the Fc region of an antibody.

The antibody fragment compositions of the present invention include compositions of antibody fragments, e.g., Fab, Fab', F(ab')₂, scFv, diabody, dsFv and a peptide comprising CDR, containing a part or the whole of the antibody Fc region in which fucose is not bound to the N-acetylglucosamine in the reducing end in complex type N-glycoside-linked sugar chains. When the antibody fragment composition does not contain a part or the whole of the antibody Fc region, the antibody fragment may be fused with a part or the whole of the Fc region of the antibody having sugar chains in which fucose is not bound to N-acetylglucosamine in the reducing end in the complex type N-glycoside-linked sugar chains as a fusion protein, or the antibody fragment may be used as a fusion protein composition with a protein comprising a part or the whole of the Fc region.

An Fab fragment is one of the fragments obtained by treatment of IgG with the proteolytic enzyme, papain (cleavage at amino acid residue 224 of H chain). It is an antibody fragment with a molecular weight of approximately 50,000 having antigen-binding activity and composed of the N-terminal half of H chain and the entire L chain linked by a disulfide bond.

The Fab fragment of the present invention can be obtained by treating the above antibody with the protease, papain. Alternatively, the Fab fragment may be produced by inserting DNA encoding the Fab fragment of the antibody into an expression vector for prokaryote or eukaryote, and introducing the vector into a prokaryote or eukaryote to induce expression.

An F(ab')₂ fragment is one of the fragments obtained by treatment of IgG with the proteolytic enzyme, pepsin (cleavage at amino acid residue 234 of H chain). It is an antibody fragment with a molecular weight of approximately 100,000 having antigen-binding activity, which is slightly larger than the Fab fragments linked together by a disulfide bond at the hinge region.

The F(ab')₂ fragment of the present invention can be obtained by treating the above antibody with the protease, pepsin. Alternatively, the F(ab')₂ fragment may be prepared by binding Fab' fragments described below by a thioether bond or a disulfide bond.

An Fab' fragment is an antibody fragment with a molecular weight of approximately 50,000 having antigen-binding activity, which is obtained by cleaving the disulfide bond at the hinge region of the above F(ab')₂ fragment.

The Fab' fragment of the present invention can be obtained by treating the above F(ab')₂ fragment with a reducing agent, dithiothreitol. Alternatively, the Fab' fragment may be produced by inserting DNA encoding the Fab' fragment of the antibody into an expression vector for prokaryote or eukaryote, and introducing the vector into a prokaryote or eukaryote to induce expression.

An scFv fragment is a VH-P-VL or VL-P-VH polypeptide in which one VH and one VL are linked via an appropriate peptide linker (hereinafter referred to as P) and which has antigen-binding activity.

The scFv fragment of the present invention can be produced by obtaining cDNAs encoding the VH and VL of the above antibody, constructing DNA encoding the scFv fragment, inserting the DNA into an expression vector for prokaryote or eukaryote, and introducing the expression vector into a prokaryote or eukaryote to induce expression.

A diabody is an antibody fragment which is an scFv dimer showing bivalent antigen binding activity, which may be either monospecific or bispecific.

The diabody of the present invention can be produced by obtaining cDNAs encoding the VH and VL of the above antibody, constructing DNA encoding scFv fragments with P having an amino acid sequence of 8 or less amino acid residues, inserting the DNA into an expression vector for prokaryote or eukaryote, and introducing the expression vector into a prokaryote or eukaryote to induce expression.

A dsFv fragment is an antibody fragment wherein polypeptides in which one amino acid residue of each of VH and VL is substituted with a cysteine residue are linked by a disulfide bond between the cysteine residues. The amino acid residue to be substituted with a cysteine residue can be selected based on antibody tertiary structure prediction according to the method proposed by Reiter, et al. (Protein Engineering, 7, 697-704, 1994).

The dsFv fragment of the present invention can be produced by obtaining cDNAs encoding the VH and VL of the above antibody, constructing DNA encoding the dsFv fragment, inserting the DNA into an expression vector for prokaryote or eukaryote, and introducing the vector into a prokaryote or eukaryote to induce expression.

A peptide comprising CDR comprises one or more region CDR of VH or VL. A peptide comprising plural CDRs can be prepared by binding CDRs directly or via an appropriate peptide linker.

The peptide comprising CDR of the present invention can be produced by constructing DNA encoding CDR of VH and VL of the above antibody, inserting the DNA into an expression vector for prokaryote or eukaryote, and introducing the expression vector into a prokaryote or eukaryote to induce expression.

The peptide comprising CDR can also be produced by chemical synthesis methods such as the Fmoc method (fluorenylmethyloxycarbonyl method) and the tBoc method (t-butyloxycarbonyl method).

The production process of the present invention is explained below in detail.

### 1. Construction of cell used in the production of the present invention

The cell into which an RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain in the present invention can be prepared, for example, as follows.

A cDNA or a genomic DNA encoding an enzyme relating to the modification of a sugar chain in which 1-position of fucose is bound to 6-position of N-acetylglucosamine in the reducing end through α-bond in a complex type N-glycoside-linked sugar chain is prepared.

The nucleotide sequence of the prepared cDNA or genomic DNA is determined.

Based on the determined DNA sequence, a construct of an RNAi gene comprising a coding region encoding the enzyme relating to the modification of a sugar chain in which 1-position of fucose is bound to 6-position of N-acetylglucosamine in the reducing end through α-bond in a complex type N-glycoside-linked sugar chain or a non-coding region at an appropriate length is designed.

In order to express the RNAi gene in a cell, a recombinant vector is obtained by inserting a fragment or full length of the prepared DNA into downstream of the promoter of an appropriate expression vector.

A transformant is obtained by introducing the recombinant vector into a host cell suitable for the expression vector.

The cell of the present invention can be obtained by selecting a transformant based on the activity of the enzyme relating to the modification of a sugar chain in which 1-position of fucose is bound to 6-position of N-acetylglucosamine in the reducing end through α-bond in a complex type N-glycoside-linked sugar chain or the sugar chain structure of the produced antibody molecule or the glycoprotein on the cell surface.

As the host cell, any cell such as an yeast, an animal cell, an insect cell or a plant cell can be used, so long as it has a gene encoding the target enzyme relating to the modification of a sugar chain in which 1-position of fucose is bound to 6-position of N-acetylglucosamine in the reducing end through α-bond in a complex type N-glycoside-linked sugar chain. Examples include host cells described in the following 2.

As the expression vector, a vector which can be autonomously replicable in the host cell or can be integrated into the chromosome and comprises a promoter at such a position that the designed RNAi gene can be transcribed is used. Examples include expression vectors where transcription is carried out by polymerase III or expression vectors described in the following 2.

As the method for introducing a gene into various host cells, the methods for introducing recombinant vectors suitable for various host cells described in the following 2 can be used.

Preparation of a cDNA or genomic DNA encoding the enzyme relating to the modification of a sugar chain in which 1-position of fucose is bound to 6-position of N-acetylglucosamine in the reducing end through α-bond in a complex type N-glycoside-linked sugar chain can be carried out, for example, by the following method.

### Preparation method of cDNA:

Total RNA or mRNA is prepared from a various host cell tissue or cell.

A cDNA library is prepared from the total RNA or mRNA.

Degenerative primers are prepared based on the known amino acid sequence of the enzyme relating to modification of a sugar chain in which 1-position of fucose is bound to 6-position of N-acetylglucosamine in the reducing end through α-bond in a complex type N-glycoside-linked sugar chain, such as an amino acid sequence in human, and a gene fragment encoding the enzyme relating to modification of a sugar chain in which 1-position of fucose is bound to 6-position of N-acetylglucosamine in the reducing end through α-bond in a complex type N-glycoside-linked sugar chain is obtained by PCR using the prepared cDNA library as the template.

A cDNA encoding the enzyme relating to the modification of a sugar chain in which 1-position of fucose is bound to 6-position of N-acetylglucosamine in the reducing end through α-bond in a complex type N-glycoside-linked sugar chain can be obtained by screening the cDNA library using the obtained gene fragment as a probe.

The mRNA of various host cells, commercially available one (for example, manufactured by Clontech) may be used, or it may be prepared from various host cells in the following manner. The method for preparing a total mRNA from various host cells include the guanidine thiocyanate-cesium trifluoroacetate method [Methods in Enzymology, 154, 3 (1987)], the acidic guanidine thiocyanate-phenol-chloroform (AGPC) method [Analytical Biochemistry, 162, 156 (1987); Experimental Medicine (Jikken Igaku), 9, 193 7 (1991)] and the like.

The methods for preparing mRNA as poly(A)+RNA from the total RNA include the oligo (dT) immobilized cellulose column method [Molecular Cloning: A Laboratory Manual, Cold Spring Harbor Lab. Press New York (1989)].

It is also possible to prepare mRNA by using a commercially available kit such as Fast Track mRNA Isolation Kit (manufactured by Invitrogen) or Quick Prep mRNA Purification Kit (manufactured by Pharmacia).

A cDNA library is prepared from the obtained mRNA of various host cells. The methods for preparing the cDNA library include the methods described in Molecular Cloning: A Laboratory Manual, Cold Spring Harbor Lab. Press New York (1989), Current Protocols in Molecular Biology, John Wiley & Sons (1987-1997); *etc.,* and methods using commercially available kits such as SuperScript Plasmid System for cDNA Synthesis and Plasmid Cloning (manufactured by Life Technologies) and ZAP-cDNA Synthesis Kit (manufactured by STRATAGENE).

As the cloning vector for preparing the cDNA library, any vectors, e.g. phage vectors and plasmid vectors, can be used so long as they are autonomously replicable in *Escherichia coli* K12. Examples of suitable vectors include ZAP Express [manufactured by STRATAGENE, Strategies, 5, 58 (1992)], pBluescript II SK(+) *[*Nucleic Acids Research, 17, 9494 (1989)], Lambda ZAP II (manufactured by STRATAGENE), λgt10 and λgt1 [DNA Cloning, A Practical Approach, 1, 49 (1985)], λTriplEx (manufactured by Clontech), λExCell (manufactured by Pharmacia), pT7T318U (manufactured by Pharmacia), pcD2 [Mol. Cell. Biol., 3, 280 (1983)], pUC18 *[*Gene, 33, 103 (1985)] and the like.

Any microorganism can be used as the host microorganism for preparing the cDNA library, but *Escherichia coli* is preferably used. Examples of suitable host microorganisms are *Escherichia coli* XL1-Blue MRF' [manufactured by STRATAGENE, Strategies, 5, 81 (1992)], *Escherichia coli* C600 [Genetics, 39, 440 (1954)], *Escherichia coli* Y1088 [Science, 222, 778 (1983)], *Escherichia coli* Y1090 [Science, 222, 778 (1983)], *Escherichia coli* NM522 [J. Mol. Biol., 166, 1 (1983)], *Escherichia coli* K802 [J. Mol. Biol., 16, 118 (1966)], *Escherichia coli* JM105 [Gene, 38, 275 (1985)] and the like.

The cDNA library may be used as such in the following analysis. Alternatively, in order to efficiently obtain full-length cDNAs by decreasing the ratio of partial cDNAs, a cDNA library prepared using the oligo-cap method developed by Sugano, et al. [Gene, 138, 171 (1994); Gene, 200, 149 (1997); Protein, Nucleic Acid and Enzyme, 41, 603 (1996); Experimental Medicine, 11, 2491 (1993); cDNA Cloning (Yodosha) (1996); Methods for Preparing Gene Libraries (Yodosha) (1994)] may be used in the following analysis.

Degenerative primers specific for the 5'-terminal and 3'-terminal nucleotide sequences of a nucleotide sequence presumed to encode the amino acid sequence of an enzyme relating to the modification of a sugar chain in which 1-position of fucose is bound to 6-position of N-acetylglucosamine in the reducing end through α-bond in a complex type N-glycoside-linked sugar chain are prepared based on the amino acid sequence of the enzyme. A gene fragment encoding the enzyme relating to the modification of a sugar chain in which 1-position of fucose is bound to 6-position of N-acetylglucosamine in the reducing end through α-bond in a complex type N-glycoside-linked sugar chain can be obtained by DNA amplification by PCR [PCR Protocols, Academic Press (1990)] using the prepared cDNA library as a template.

It can be confirmed that the obtained gene fragment is a cDNA encoding the enzyme relating to the modification of a sugar chain in which 1-position of fucose is bound to 6-position of N-acetylglucosamine in the reducing end through α-bond in a complex type N-glycoside-linked sugar chain by analyzing the nucleotide sequence by generally employed methods such as the dideoxy method of Sanger, et al. [Proc. Natl. Acad. Sci. U.S.A., 74, 5463 (1977)] or by use of nucleotide sequencers such as ABI PRISM 377 DNA Sequencer (manufactured by Applied Biosystems).

A DNA encoding the enzyme relating to the modification of a sugar chain in which 1-position of fucose is bound to 6-position of N-acetylglucosamine in the reducing end through α-bond in a complex type N-glycoside-linked sugar chain can be obtained from the cDNA or cDNA library synthesized from the mRNA contained in various host cells by colony hybridization or plaque hybridization (*Molecular Cloning,* Second Edition) using the above gene fragment as a probe.

A DNA encoding the enzyme relating to the modification of a sugar chain in which 1-position of fucose is bound to 6-position of N-acetylglucosamine in the reducing end through α-bond in a complex type N-glycoside-linked sugar chain can also be obtained by amplification by PCR using the cDNA or cDNA library synthesized from the mRNA contained in various host cells as a template and using the primers used for obtaining the gene fragment encoding the enzyme relating to the modification of a sugar chain in which 1-position of fucose is bound to 6-position of N-acetylglucosamine in the reducing end through α-bond in a complex type N-glycoside-linked sugar chain.

The nucleotide sequence of the obtained DNA encoding the enzyme relating to the modification of a sugar chain in which 1-position of fucose is bound to 6-position of N-acetylglucosamine in the reducing end through α-bond in a complex type N-glycoside-linked sugar chain can be determined by generally employed sequencing methods such as the dideoxy method of Sanger, et al. [Proc. Natl. Acad. Sci. U.S.A., 74, 5463 (1977)] or by use of nucleotide sequencers such as ABI PRISM 377 DNA Sequencer (manufactured by Applied Biosystems).

By carrying out a search of nucleotide sequence databases such as GenBank, EMBL or DDBJ using a homology search program such as BLAST based on the determined nucleotide sequence of the cDNA, it can be determined that the obtained DNA is a gene encoding the enzyme relating to the modification of a sugar chain in which 1-position of fucose is bound to 6-position of N-acetylglucosamine in the reducing end through α-bond in a complex type N-glycoside-linked sugar chain among the genes in the nucleotide sequence database.

Examples of the nucleotide sequences of the genes encoding the enzyme relating to the modification of a sugar chain in which 1-position of fucose is bound to 6-position of N-acetylglucosamine in the reducing end through α-bond in a complex type N-glycoside-linked sugar chain obtained by the above methods include the nucleotide sequences represented by SEQ ID NO:1, 2, 3 or 4.

The cDNA of the enzyme relating to the modification of a sugar chain in which 1-position of fucose is bound to 6-position of N-acetylglucosamine in the reducing end through α-bond in a complex type N-glycoside-linked sugar chain can also be obtained by chemical synthesis with a DNA synthesizer such as DNA Synthesizer Model 392 (manufactured by Perkin Elmer) utilizing the phosphoamidite method based on the determined nucleotide sequence of the DNA.

Preparation of a genomic DNA encoding the enzyme relating to the modification of a sugar chain in which 1-position of fucose is bound to 6-position of N-acetylglucosamine in the reducing end through α-bond in a complex type N-glycoside-linked sugar chain can be carried out, for example, by the following method.

### Preparation method of genomic DNA:

The genomic DNA can be prepared by known methods described in Molecular Cloning: A Laboratory Manual, Cold Spring Harbor Lab. Press New York (1989), Current Protocols in Molecular Biology, John Wiley & Sons (1987-1997) and the like. In addition, the genomic DNA encoding the enzyme relating to the modification of a sugar chain in which 1-position of fucose is bound to 6-position of N-acetylglucosamine in the reducing end through α-bond in a complex type N-glycoside-linked sugar chain can be obtained by using a kit such as Genomic DNA Library Screening System (manufactured by Genome Systems) or Universal Genome Walker™ Kits (manufactured by CLONTECH).

Selection of a transformant using, as a marker, the activity of an enzyme relating to the modification of a sugar chain in which 1-position of fucose is bound to 6-position of N-acetylglucosamine in the reducing end through α-bond in a complex type N-glycoside-linked sugar chain can be carried out, for example, by the following methods.

### Method for selecting transformant:

The method for selecting a cell in which the activity of the enzyme relating to the modification of a sugar chain in which 1-position of fucose is bound to 6-position of N-acetylglucosamine in the reducing end through α-bond in a complex type N-glycoside-linked sugar chain is decreased includes biochemical methods or genetic engineering techniques described in New Biochemical Experimentation Series 3-Saccharides I, Glycoprotein (Tokyo Kagaku Dojin), edited by Japanese Biochemical society (1988); Cell Engineering, Supplement, Experimental Protocol Series, Glycobiology Experimental Protocol, Glycoprotein, Glycolipid and Proteoglycan (Shujun-sha), edited by Naoyuki Taniguchi, Akemi Suzuki, Kiyoshi Furukawa and Kazuyuki Sugawara (1996); Molecular Cloning: A Laboratory Manual, Cold Spring Harbor Lab. Press New York (1989); Current Protocols in Molecular Biology, John Wiley & Sons (1987-1997); and the like. An example of the biochemical methods includes a method in which the enzyme activity is evaluated using an enzyme-specific substrate. Examples of the genetic engineering techniques include Northern analysis and RT-PCR in which the amount of mRNA for a gene encoding the enzyme relating to the modification of a sugar chain in which 1-position of fucose is bound to 6-position of N-acetylglucosamine in the reducing end through α-bond in a complex type N-glycoside-linked sugar chain is measured.

Furthermore, the method for selecting a cell based on morphological change caused by decrease of the activity of the enzyme relating to a sugar chain modification in which 1-position of fucose is bound to 6-position of N-acetylglucosamine in the reducing end through α-bond in a complex type N-glycoside-linked sugar chain includes a method for selecting a transformant based on the sugar structure of a produced antibody molecule, a method for selecting a transformant based on the sugar structure of a glycoprotein on a cell membrane, and the like. The method for selecting a transformant using the sugar structure of a produced antibody molecule includes method described in the item 4 below. The method for selecting a transformant using the sugar structure of a glycoprotein on a cell membrane a clone resistant to a lectin which recognizes a sugar chain structure wherein 1-position of fucose is bound to 6-position of N-acetylglucosamine in the reducing end through α-bond in a complex type N-glycoside-linked sugar chain. Examples include a method using a lectin described in Somatic Cell Mol. Genet., 12, 51 (1986).

As the lectin, any lectin can be used, so long as it is a lectin which recognizes a sugar chain structure in which 1-position of fucose is bound to 6-position of N-acetylglucosamine in the reducing end through α-bond in the N-glycoside-linked sugar chain. Examples include lentil lectin LCA (lentil agglutinin derived from *Lens culinaris*), pea lectin PSA (pea lectin derived from *Pisum sativum),* broad bean lectin VFA (agglutinin derived from *Vicia faba*) and *Aleuria aurantia* lectin AAL (lectin derived from *Aleuria aurantia*) and the like.

Specifically, the cell line of the present invention resistant to a lectin which recognizes a sugar chain structure in which 1-position of fucose is bound to 6-position of N-acetylglucosamine in the reducing end through α-bond in a complex type N-glycoside-linked sugar chain can be selected by culturing cells in a medium containing the above lectin at a concentration of 1 µg/mL to 1 mg/mL for one day to 2 weeks, preferably one day to one week, subculturing surviving cells or picking up a colony and transferring it into a culture vessel, and subsequently continuing the culturing using the medium containing the lectin.

The RNAi gene for suppressing the mRNA amount of a gene encoding the enzyme relating to the modification of a sugar chain in which 1-position of fucose is bound to 6-position of N-acetylglucosamine in the reducing end through α-bond in a complex type N-glycoside-linked sugar chain can be prepared by known methods or by using a DNA synthesizer.

The RNAi gene construct can be designed according to the descriptions in Nature, 391, 806 (1998); Proc. Natl. Acad Sci. USA, 95, 15502 (1998); Nature, 395, 854 (1998); Proc. Natl. Acad Sci. USA, 96, 5049 (1999); Cell, 95, 1017 (1998); Proc. Natl. Acad. Sci. USA, 96, 1451 (1999); Proc. Natl. Acad Sci. USA, 95, 13959 (1998); Nature Cell Biol., 2, 70 (2000) ; Proc. Natl. Acad. Sci. USA, 98, 9742, (2001); *etc.*

Further, the cell of the present invention can also be obtained without using an expression vector by directly introducing into a host cell the RNAi gene designed based on the nucleotide sequence encoding the enzyme relating to the modification of a sugar chain in which 1-position of fucose is bound to 6-position of N-acetylglucosamine in the reducing end through α-bond in a complex type N-glycoside-linked sugar chain.

The double-stranded RNA can be prepared by known methods or by using a DNA synthesizer. Specifically, it can be prepared based on the sequence information of an oligonucleotide having a corresponding sequence of 1 to 40 bases, preferably 5 to 40 bases, more preferably 10 to 35 bases, and most preferably 15 to 29 bases, among complementary RNA nucleotide sequences of a cDNA and a genomic DNA of the enzyme relating to the modification of a sugar chain in which 1-position of fucose is bound to 6-position of N-acetylglucosamine in the reducing end through α-bond in a complex type N-glycoside-linked sugar chain by synthesizing an oligonucleotide which corresponds to a sequence, and an oligonucleotide (antisense oligonucleotide) which corresponds to a sequence complementary to the oligonucleotide. The oligonucleotide and the antisense oligonucleotide may be independently synthesized or may be linked via a spacer nucleotide which does not obstruct the formation of the double-stranded RNA.

The oligonucleotide includes an oligo RNA and derivatives of the oligonucleotide (hereinafter referred to as "oligonucleotide derivatives"), and the like.

The oligonucleotide derivatives include an oligonucleotide derivative wherein the phosphodiester bond in the oligonucleotide is converted to a phosophorothioate bond, an oligonucleotide derivative wherein the phosphodiester bond in the oligonucleotide is converted to an N3'-P5' phosphoamidate bond, an oligonucleotide derivative wherein the ribose-phosphodiester bond in the oligonucleotide is converted to a peptide-nucleic acid bond, an oligonucleotide derivative wherein the uracil in the oligonucleotide is substituted by C-5 propynyluracil, an oligonucleotide derivative wherein the uracil in the oligonucleotide is substituted by C-5 thiazolyluracil, an oligonucleotide derivative wherein the cytosine in the oligonucleotide is substituted by C-5 propynylcytosine, an oligonucleotide derivative wherein the cytosine in the oligonucleotide is substituted by phenoxazine-modified cytosine, an oligonucleotide derivative wherein the ribose in the oligonucleotide is substituted by 2'-O-propylribose, and an oligonucleotide derivative wherein the ribose in the oligonucleotide is substituted by 2'-methoxyethoxyribose [Cell Technology, 16, 1463 (1997)].

### 2. Method for producing antibody composition

An antibody composition can be expressed and obtained in a host cell by using the method described in Molecular Cloning: A Laboratory Manual, Cold Spring Harbor Lab. Press New York (1989), Current Protocols in Molecular Biology, John Wiley & Sons (1987-1997), Antibodies, A Laboratory manual, Cold Spring Harbor Laboratory (1988), Monoclonal Antibodies: Principles and Practice, Third Edition, Acad. Press (1993), Antibody Engineering, A Practical Approach, IRL Press at Oxford University Press (1996) and the like, for example, as follows.

A cDNA encoding an antibody molecule is prepared.

Based on the full length cDNA encoding the prepared antibody molecule, a DNA fragment of an appropriate length comprising a region encoding the protein is prepared, if necessary.

A recombinant vector is prepared by inserting the DNA fragment or full-length DNA into a site downstream of a promoter in an appropriate expression vector.

The recombinant vector is introduced into a host cell suited for the expression vector to obtain a transformant producing the antibody molecule.

As the host cell, any cell such as an yeast, an animal cell, an insect cell or a plant cell, *etc.* that are capable of expressing the desired gene can be used. An animal cell is preferred.

A cell such as an yeast, an animal cell, an insect cell or a plant cell into which an enzyme relating to the modification of an N-glycoside-linked sugar chain which binds to the Fc region of the antibody molecule is introduced by a genetic engineering technique can also be used as the host cell.

The host cell used in the method for producing the antibody composition of the present invention includes a cell into which an RNA suppressing the function of an enzyme relating to the modification of a sugar chain in which 1-position of fucose is bound to 6-position of N-acetylglucosamine in the reducing end through α-bond in a complex type N-glycoside-linked sugar chain is introduced, prepared in the above 1.

The expression vectors that can be employed are those capable of autonomous replication or integration into the chromosome in the above various host cells and comprising a promoter at a position appropriate for the transcription of the DNA encoding the desired antibody molecule.

The cDNA can be prepared from a human or non-human animal tissue or cell according to "the methods for preparing a cDNA" described in the above 1 using, e.g., a probe or primers specific for the desired antibody molecule.

When an yeast is used as the host cell, YEP13 (ATCC 37115), YEp24 (ATCC 37051), YCp50 (ATCC 37419), *etc.* can be used as the expression vector.

As the promoter, any promoters capable of expressing in yeast strains can be used. Suitable promoters include promoters of genes of the glycolytic pathway such as hexokinase, PHO5 promoter, PGK promoter, GAP promoter, ADH promoter, gal 1 promoter, gal 10 promoter, heat shock protein promoter, MFα1 promoter and CUP 1 promoter.

Examples of suitable host cells are microorganisms belonging to the genera *Saccharomyces, Schizosaccharomyces, Kluyveromyces, Trichosporon* and *Schwanniomyces,* and specifically, *Saccharomyces cerevisiae, Schizosaccharomyces pombe, Kluyveromyces lactis, Trichosporon pullulans* and *Schwanniomyces alluvius.*

Introduction of the recombinant vector can be carried out by any of the methods for introducing DNA into an yeast, for example, electroporation [Methods Enzymol., 194, 182 (1990)], the spheroplast method *[*Proc. Natl. Acad Sci. USA, 84, 1929 (1978)], the lithium acetate method [J. Bacteriology, 153, 163 (1983)] and the method described *in* Proc. Natl. Acad. Sci. USA, 75, 1929 (1978).

When an animal cell is used as the host cell, pcDNAI, pcDM8 (commercially available from Funakoshi Co., Ltd.), pAGE107 [Japanese Published Unexamined Patent Application No. 22979/91; Cytotechnology, 3, 133 (1990)], pAS3-3 (Japanese Published Unexamined Patent Application No. 227075/90), pCDM8 [Nature, 329, 840 (1987)], pcDNAI/Amp (manufactured by Invitrogen Corp.), pREP4 (manufactured by Invitrogen Corp.), pAGE103 *[*J. Biochemistry, 101, 1307 (1987)], pAGE210, *etc.* can be used as the expression vector.

As the promoter, any promoters capable of expressing in animal cells can be used. Suitable promoters include the promoter of IE (immediate early) gene of cytomegalovirus (CMV), SV40 early promoter, the promoter of a retrovirus, metallothionein promoter, heat shock promoter, SRα promoter, *etc.* The enhancer of IE gene of human CMV may be used in combination with the promoter.

Examples of suitable host cells are human-derived Namalwa cells, monkey-derived COS cells, Chinese hamster-derived CHO cells, HBT5637 (Japanese Published Unexamined Patent Application No. 299/88), rat myeloma cells, mouse myeloma cells, cells derived from Syrian hamster kidney, embryonic stem cells and fertilized egg cells.

Introduction of the recombinant vector can be carried out by any of the methods for introducing DNA into animal cells, for example, electroporation *[*Cytotechnology, 3, 133 (1990)], the calcium phosphate method (Japanese Published Unexamined Patent Application No. 227075/90), lipofection [Proc. Natl. Acad. Sci. USA, 84, 7413 (1987)], the injection method (Manipulating the Mouse Embryo, A Laboratory Manual, Second Edition, Cold Spring Harbor Laboratory Press (1994)), the method using particle gun (gene gun) (Japanese Patent Nos. 2606856 and 2517813), the DEAE-dextran method [Biomanual Series 4 - Methods of Gene Transfer, Expression and Analysis (Yodosha), edited by Takashi Yokota and Kenichi Arai (1994)] and the virus vector method [Manipulating the Mouse Embryo, A Laboratory Manual, Second Edition, Cold Spring Harbor Laboratory Press (1994)].

When an insect cell is used as the host cell, the protein can be expressed by the methods described in Current Protocols in Molecular Biology; Baculovirus Expression Vectors, A Laboratory Manual, W. H. Freeman and Company, New York (1992); Bio/Technology, 6, 47 (1988), *etc.*

That is, the recombinant vector and a baculovirus are cotransfected into insect cells to obtain a recombinant virus in the culture supernatant of the insect cells, and then insect cells are infected with the recombinant virus, whereby the protein can be expressed.

The gene transfer vectors useful in this method include pVL1392, pVL1393 and pBlueBacIII (products of Invitrogen Corp.).

An example of the baculovirus is Autographa californica nuclear polyhedrosis virus, which is a virus infecting insects belonging to the family *Barathra.*

Examples of the insect cells are *Spodoptera frugiperda* ovarian cells Sf9 and Sf21 [Current Protocols in Molecular Biology; Baculovirus Expression Vectors, A Laboratory Manual, W. H. Freeman and Company, New York (1992)] and *Trichoplusia* ni ovarian cell High 5 (manufactured by Invitrogen Corp.).

Cotransfection of the above recombinant vector and the above baculovirus into insect cells for the preparation of the recombinant virus can be carried out by the calcium phosphate method (Japanese Published Unexamined Patent Application No. 227075/90), lipofection [Proc. Natl. Acad. Sci. USA, 84, 7413 (1987)], *etc.*

When a plant cell is used as the host cell, Ti plasmid, tobacco mosaic virus vector, *etc.* can be used as the expression vector.

As the promoter, any promoters capable of expressing in plant cells can be used. Suitable promoters include 35S promoter of cauliflower mosaic virus (CaMV), rice actin 1 promoter, *etc.*

Examples of suitable host cells are cells of plants such as tobacco, potato, tomato, carrot, soybean, rape, alfalfa, rice, wheat and barley.

Introduction of the recombinant vector can be carried out by any of the methods for introducing DNA into plant cells, for example, the method using *Agrobacterium* (Japanese Published Unexamined Patent Application Nos. 140885/84 and 70080/85, WO94/00977), electroporation (Japanese Published Unexamined Patent Application No. 251887/85) and the method using particle gun (gene gun) (Japanese Patent Nos. 2606856 and 2517813).

Expression of the antibody gene can be carried out not only by direct expression but also by secretory production, expression of a fusion protein of the Fc region and another protein, *etc.* according to the methods described in Molecular Cloning, Second Edition, etc*.*

When the gene is expressed in yeast, an animal cell, an insect cell or a plant cell carrying an introduced gene relating to the synthesis of a sugar chain, an antibody molecule to which a sugar or a sugar chain is added by the introduced gene can be obtained.

The antibody composition can be produced by culturing the transformant obtained as above in a medium, allowing the antibody molecules to form and accumulate in the culture, and recovering them from the culture. Culturing of the transformant in a medium can be carried out by conventional methods for culturing the host cell.

For the culturing of the transformant obtained by using a eucaryote such as yeast as the host, any of natural media and synthetic media can be used insofar as it is a medium suitable for efficient culturing of the transformant which contains carbon sources, nitrogen sources, inorganic salts, *etc*. which can be assimilated by the host used.

As the carbon sources, any carbon sources that can be assimilated by the host can be used. Examples of suitable carbon sources include carbohydrates such as glucose, fructose, sucrose, molasses containing them, starch and starch hydrolyzate; organic acids such as acetic acid and propionic acid; and alcohols such as ethanol and propanol.

As the nitrogen sources, ammonia, ammonium salts of organic or inorganic acids such as ammonium chloride, ammonium sulfate, ammonium acetate and ammonium phosphate, and other nitrogen-containing compounds can be used as well as peptone, meat extract, yeast extract, corn steep liquor, casein hydrolyzate, soybean cake, soybean cake hydrolyzate, and various fermented microbial cells and digested products thereof

Examples of the inorganic salts include potassium dihydrogenphosphate, dipotassium hydrogenphosphate, magnesium phosphate, magnesium sulfate, sodium chloride, ferrous sulfate, manganese sulfate, copper sulfate and calcium carbonate.

Culturing is usually carried out under aerobic conditions, for example, by shaking culture or submerged spinner culture under aeration. The culturing temperature is preferably 15 to 40°C, and the culturing period is usually 16 hours to 7 days. The pH is maintained at 3 to 9 during the culturing. The pH adjustment is carried out by using an organic or inorganic acid, an alkali solution, urea, calcium carbonate, ammonia, *etc*.

If necessary, antibiotics such as ampicillin and tetracycline may be added to the medium during the culturing.

When an yeast transformed with a recombinant vector comprising an inducible promoter is cultured, an inducer may be added to the medium, if necessary. For example, in the case of an yeast transformed with a recombinant vector comprising *lac* promoter, isopropyl-β-D-thiogalactopyranoside or the like may be added to the medium; and in the case of an yeast transformed with a recombinant vector comprising *trp* promoter, indoleacrylic acid or the like may be added.

For the culturing of the transformant obtained by using an animal cell as the host cell, generally employed media such as RPMI1640 medium [The Journal of the American Medical Association, 199, 519 (1967)], Eagle's MEM *[*Science, 122, 501 (1952)], Dulbecco's modified MEM [Virology, 8, 396 (1959)], 199 medium [Proceeding of the Society for the Biological Medicine, 73, 1 (1950)] and Whitten's medium *[*Developmental Engineering Experimentation Manual - Preparation of Transgenic Mice (Kodansha), edited by Motoya Katsuki (1987)], media prepared by adding fetal calf serum or the like to these media, *etc.* can be used as the medium.

Culturing is usually carried out under conditions of pH 6 to 8 at 30 to 40°C for 1 to 7 days in the presence of 5% CO₂.

If necessary, antibiotics such as kanamycin and penicillin may be added to the medium during the culturing.

For the culturing of the transformant obtained by using an insect cell as the host cell, generally employed media such as TNM-FH medium (manufactured by Pharmingen, Inc.), Sf-900 II SFM medium (manufactured by Life Technologies, Inc.), ExCell 400 and ExCell 405 (manufactured by JRH Biosciences, Inc.) and Grace's Insect Medium *[*Nature, 195, 788 (1962)] can be used as the medium.

Culturing is usually carried out under conditions of pH 6 to 7 at 25 to 30°C for 1 to 5 days.

If necessary, antibiotics such as gentamicin may be added to the medium during the culturing.

The transformant obtained by using a plant cell as the host cell may be cultured in the form of cells as such or after differentiation into plant cells or plant organs. For the culturing of such transformant, generally employed media such as Murashige-Skoog (MS) medium and White medium, media prepared by adding phytohormones such as auxin and cytokinin to these media, *etc.* can be used as the medium.

Culturing is usually carried out under conditions of pH 5 to 9 at 20 to 40°C for 3 to 60 days.

If necessary, antibiotics such as kanamycin and hygromycin may be added to the medium during the culturing.

As described above, the antibody composition can be produced by culturing, according to a conventional culturing method, the transformant derived from an animal cell or a plant cell and carrying an expression vector into which DNA encoding the antibody molecule has been inserted, allowing the antibody composition to form and accumulate, and recovering the antibody composition from the culture.

The antibody composition may be produced by intracellular production by host cells, extracellular secretion by host cells or production on outer membranes by host cells. A desirable production method can be adopted by changing the kind of the host cells used or the structure of the antibody molecule to be produced.

When the antibody composition is produced in host cells or on outer membranes of host cells, it is possible to force the antibody composition to be secreted outside the host cells by applying the method of Paulson, et al. [J. Biol. Chem., 264, 17619 (1989)], the method of Lowe, et al. [Proc. Natl. Acad. Sci. USA, 86, 8227 (1989); Genes Develop., 4, 1288 (1990)], or the methods described in Japanese Published Unexamined Patent Application No. 336963/93, WO94/23021, *etc.*

That is, it is possible to force the desired antibody molecule to be secreted outside the host cells by inserting DNA encoding the antibody molecule and DNA encoding a signal peptide suitable for the expression of the antibody molecule into an expression vector, introducing the expression vector into the host cells, and then expressing the antibody molecule by use of recombinant DNA techniques.

It is also possible to increase the production of the antibody composition by utilizing a gene amplification system using a dihydrofolate reductase gene or the like according to the method described in Japanese Published Unexamined Patent Application No. 227075/90.

Further, the antibody composition can be produced using an animal having an introduced gene (non-human transgenic animal) or a plant having an introduced gene (transgenic plant) constructed by redifferentiation of animal_or plant cells carrying the introduced gene.

When the transformant is an animal or plant, the antibody composition can be produced by raising or culturing the animal or plant in a usual manner, allowing the antibody composition to form and accumulate therein, and recovering the antibody composition from the animal or plant.

Production of the antibody composition using an animal can be carried out, for example, by producing the desired antibody composition in an animal constructed by introducing the gene according to known methods [American Journal of Clinical Nutrition, 63, 639S (1996); American Journal of Clinical Nutrition, 63, 627S (1996); Bio/Technology, 9, 830 (1991)].

In the case of an animal, the antibody composition can be produced, for example, by raising a non-human transgenic animal carrying the introduced DNA encoding the antibody molecule, allowing the antibody composition to form and accumulate in the animal, and recovering the antibody composition from the animal. The places where the antibody composition is formed and accumulated include milk (Japanese Published Unexamined Patent Application No. 309192/88), egg, *etc.* of the animal. As the promoter in this process, any promoters capable of expressing in an animal can be used. Preferred promoters include mammary gland cell-specific promoters such as α casein promoter, β casein promoter, β lactoglobulin promoter and whey acidic protein promoter.

Production of the antibody composition using a plant can be carried out, for example, by culturing a transgenic plant carrying the introduced DNA encoding the antibody molecule according to known methods *[*Soshiki Baiyo (Tissue Culture), 20 (1994); Soshiki Baiyo (Tissue Culture), 21 (1995); Trends in Biotechnology, 15, 45 (1997)], allowing the antibody composition to form and accumulate in the plant, and recovering the antibody composition from the plant.

When the antibody composition produced by the transformant carrying the introduced gene encoding the antibody molecule is expressed in a soluble form in cells, the cells are recovered by centrifugation after the completion of culturing and suspended in an aqueous buffer, followed by disruption using a sonicator, French press, Manton Gaulin homogenizer, Dynomill or the like to obtain a cell-free extract. A purified preparation of the antibody composition can be obtained by centrifuging the cell-free extract to obtain the supernatant and then subjecting the supernatant to ordinary means for isolating and purifying enzymes, e.g., extraction with a solvent, salting-out with ammonium sulfate, *etc.,* desalting, precipitation with an organic solvent, anion exchange chromatography using resins such as diethylaminoethyl (DEAE)-Sepharose and DIAION HPA-75 (manufactured by Mitsubishi Chemical Corporation), cation exchange chromatography using resins such as S-Sepharose FF (manufactured by Pharmacia), hydrophobic chromatography using resins such as butyl Sepharose and phenyl Sepharose, gel filtration using a molecular sieve, affinity chromatography, chromatofocusing, and electrophoresis such as isoelectric focusing, alone or in combination.

When the antibody composition is expressed as an inclusion body in cells, the cells are similarly recovered and disrupted, followed by centrifugation to recover the inclusion body of the antibody composition as a precipitate fraction. The recovered inclusion body of the antibody composition is solubilized with a protein-denaturing agent. The solubilized antibody solution is diluted or dialyzed, whereby the antibody composition is renatured to have normal conformation. Then, a purified preparation of the antibody composition can be obtained by the same isolation and purification steps as described above.

When the antibody composition is extracellularly secreted, the antibody composition or its derivative can be recovered in the culture supernatant. That is, the culture is treated by the same means as above, e.g., centrifugation, to obtain the culture supernatant. A purified preparation of the antibody composition can be obtained from the culture supernatant by using the same isolation and purification methods as described above.

The antibody composition thus obtained includes an antibody, an antibody fragment, a fusion protein comprising the Fc region of the antibody, and the like.

As examples for obtaining the antibody composition, processes for producing a humanized antibody composition and an Fc fusion protein are described below in detail, but other antibody compositions can also be obtained in the same manner similar to the above methods.

### A. Preparation of humanized antibody composition

### (1) Construction of vector for expression of humanized antibody

A vector for expression of humanized antibody is an expression vector for animal cells carrying inserted genes encoding CH and CL of a human antibody, which can be constructed by cloning each of the genes encoding CH and CL of a human antibody into an expression vector for animal cells.

The C regions of a human antibody may be CH and CL of any human antibody. Examples of the C regions include the C region of IgGl subclass human antibody H chain (hereinafter referred to as hCγl) and the C region of κ class human antibody L chain (hereinafter referred to as hCκ).

As the genes encoding CH and CL of a human antibody, a genomic DNA comprising exons and introns can be used. Also useful is a cDNA.

As the expression vector for animal cells, any vector for animal cells can be used so long as it is capable of inserting and expressing the gene encoding the C region of a human antibody. Suitable vectors include pAGE107 [Cytotechnology, 3, 133 (1990)], pAGE103 [J. Biochem., 101, 1307 (1987)], pHSG274 *[*Gene, 27, 223 (1984)], pKCR [Proc. Natl. Acad. Sci. USA, 78, 1527 (1981)] and pSG1βd2-4 [Cytotechnology, 4, 173 (1990)]. Examples of the promoter and enhancer for use in the expression vector for animal cells include SV40 early promoter and enhancer [J. Biochem., 101, 1307 (1987)], LTR of Moloney mouse leukemia virus [Biochem. Biophys. Res.Commun., 149, 960 (1987)] and immunoglobulin H chain promoter *[*Cell, 41, 479 (1985)] and enhancer *[*Cell, 33, 717 (1983)].

The vector for expression of humanized antibody may be either of the type in which the genes encoding antibody H chain and L chain exist on separate vectors or of the type in which both genes exist on the same vector (hereinafter referred to as tandem-type). The tandem-type ones are preferred in view of the easiness of construction of the vector for expression of humanized antibody, the easiness of introduction into animal cells, the balance between the expression of antibody H chain and that of antibody L chain in animal cells, *etc.* [J. Immunol. Methods, 167, 271 (1994)]. Examples of the tandem-type humanized antibody expression vectors include pKANTEX93 [Mol. Immunol., 37, 1035 (2000)] and pEE18 [Hybridoma, 17, 559 (1998)].

The constructed vector for expression of humanized antibody can be used for the expression of a human chimeric antibody and a human CDR-grafted antibody in animal cells.

### (2) Obtaining of cDNA encoding V region of an antibody derived from a non-human animal

cDNAs encoding VH and VL of an antibody derived from a non-human animal, e.g., a mouse antibody can be obtained in the following manner.

A cDNA is synthesized using, as a template, an mRNA extracted from a hybridoma cell producing a desired mouse antibody. The synthesized cDNA is cloned into a vector such as a phage or a plasmid to prepare a cDNA library. A recombinant phage or recombinant plasmid carrying a cDNA encoding VH and a recombinant phage or recombinant plasmid carrying a cDNA encoding VL are isolated from the cDNA library using DNA encoding the C region or V region of a known mouse antibody as a probe. The full length nucleotide sequences of VH and VL of the desired mouse antibody on the recombinant phages or recombinant plasmids are determined, and the full length amino acid sequences of VH and VL are deduced from the nucleotide sequences.

As the non-human animal, any animal can be used so long as hybridoma cells can be prepared from the animal. Suitable animals include mouse, rat, hamster and rabbit.

The methods for preparing total RNA from a hybridoma cell include the guanidine thiocyanate-cesium trifluoroacetate method [Methods in Enzymol., 154, 3 (1987)], and the methods for preparing mRNA from the total RNA include the oligo (dT) immobilized cellulose column method *(*Molecular Cloning: A Laboratory Manual, Cold Spring Harbor Lab. Press New York, 1989). Examples of the kits for preparing mRNA from a hybridoma cell include Fast Track mRNA Isolation Kit (Invitrogen) and Quick Prep mRNA Purification Kit (manufactured by Pharmacia).

The methods for synthesizing the cDNA and preparing the cDNA library include conventional methods (Molecular Cloning: A Laboratory Manual, Cold Spring Harbor Lab. Press New York, 1989; Current Protocols in Molecular Biology, Supplement 1-34), or methods using commercially available kits such as SuperScript™ Plasmid System for cDNA Synthesis and Plasmid Cloning (manufactured by GIBCO BRL) and ZAP-cDNA Synthesis Kit (manufactured by STRATAGENE).

In preparing the cDNA library, the vector for inserting the cDNA synthesized using the mRNA extracted from a hybridoma cell as a template may be any vector so long as the cDNA can be inserted. Examples of suitable vectors include ZAP Express [Strategies, 5, 58 (1992)], pBluescript II SK(+) [Nucleic Acids Research, 17, 9494 (1989)], λZAP II (manufactured by STRATAGENE), λgt10, λgt11 [DNA Cloning: A Practical Approach, I, 49 (1985)], Lambda BlueMid (manufactured by Clontech), λExCell, pT7T3 18U (manufactured by Pharmacia), pcD2 *[*Mol. Cell. Biol., 3, 280 (1983)] and pUC18 [Gene, 33, 103 (1985)].

As *Escherichia coli* for introducing the cDNA library constructed with a phage or plasmid vector, any *Escherichia coli* can be used so long as the cDNA library can be introduced, expressed and maintained. Examples of suitable *Escherichia coli* include XL1-Blue MRF' [Strategies, 5, 81 (1992)], C600 [Genetics, 39, 440 (1954)], Y1088, Y1090 [Science, 222, 778 (1983)], NM522 [J. Mol. Biol., 166, 1 (1983)], K802 [J.Mol. Biol., 16, 118 (1966)] and JM105 [Gene, 38, 275 (1985)].

The methods for selecting the cDNA clones encoding VH and VL of a non-human animal-derived antibody from the cDNA library include colony hybridization or plaque hybridization *(*Molecular Cloning: A Laboratory Manual, Cold Spring Harbor Lab. Press New York, 1989) using an isotope- or fluorescence-labeled probe. It is also possible to prepare the cDNAs encoding VH and VL by preparing primers and performing PCR *(*Molecular Cloning: A Laboratory Manual, Cold Spring Harbor Lab. Press New York, 1989; Current Protocols in Molecular Biology, Supplement 1-34) using the cDNA or cDNA library as a template.

The nucleotide sequences of the cDNAs selected by the above methods can be determined by cleaving the cDNAs with appropriate restriction enzymes, cloning the fragments into a plasmid such as pBluescript SK(-) (manufactured by STRATAGENE), and then analyzing the sequences by generally employed sequencing methods such as the dideoxy method of Sanger, et al. [Proc. Natl. Acad. Sci. USA, 74, 5463 (1977)] or by use of nucleotide sequencers such as ABI PRISM 377 DNA Sequencer (manufactured by Applied Biosystems).

The full length amino acid sequences of VH and VL are deduced from the determined nucleotide sequences and compared with the full length amino acid sequences of VH and VL of a known antibody (Sequences of Proteins of Immunological Interest, US Dept. Health and Human Services, 1991), whereby it can be confirmed that the obtained cDNAs encode full length amino acid sequences which comprise VH and VL of the antibody including secretory signal sequences.

### (3) Analysis of the amino acid sequence of the V region of an antibody derived from a non-human animal

By comparing the full length amino acid sequences of VH and VL of the antibody including secretory signal sequences with the full length amino acid sequences of VH and VL of a known antibody (Sequences of Proteins of Immunological Interest, US Dept. Health and Human Services, 1991), it is possible to deduce the length of the secretory signal sequences and the N-terminal amino acid sequences and further to know the subgroup to which the antibody belongs. In addition, the amino acid sequences of CDRs of VH and VL can be deduced by comparing them with the amino acid sequences of VH and VL of known antibodies [Sequences of Proteins of Immunological Interest, US Dep. Health and Human Services (1991)].

### (4) Construction of human chimeric antibody expression vector

A human chimeric antibody expression vector can be constructed by cloning the cDNAs encoding VH and VL of an antibody derived from a non-human animal into sites upstream of the genes encoding CH and CL of a human antibody in the vector for expression of humanized antibody described in the above 2 (1). For example, a human chimeric antibody expression vector can be constructed by ligating the cDNAs encoding VH and VL of an antibody derived from a non-human animal respectively to synthetic DNAs comprising the 3'-terminal nucleotide sequences of VH and VL of an antibody derived from a non-human animal and the 5'-terminal nucleotide sequences of CH and CL of a human antibody and also having recognition sequences for appropriate restriction enzymes at both ends, and cloning them into sites upstream of the genes encoding CH and CL of a human antibody in the vector for humanized antibody expression described in the above 2 (1) so as to express them in an appropriate form.

### (5) Construction of cDNA encoding V region of human CDR-grafted antibody

cDNAs encoding VH and VL of a human CDR-grafted antibody can be constructed in the following manner. First, amino acid sequences of FRs of VH and VL of a human antibody for grafting CDRs of VH and VL of a non-human animal-derived antibody are selected. The amino acid sequences of FRs of VH and VL of a human antibody may be any of those derived from human antibodies. Suitable sequences include the amino acid sequences of FRs of VHs and VLs of human antibodies registered at databases such as Protein Data Bank, and the amino acid sequences common to subgroups of FRs of VHs and VLs of human antibodies (Sequences of Proteins of Immunological Interest, US Dept. Health and Human Services, 1991). In order to prepare a human CDR-grafted antibody having a sufficient activity, it is preferred to select amino acid sequences having as high a homology as possible (at least 60% or more) with the amino acid sequences of FRs of VH and VL of the non-human animal-derived antibody of interest.

Next, the amino acid sequences of CDRs of VH and VL of the non-human animal-derived antibody of interest are grafted to the selected amino acid sequences of FRs of VH and VL of a human antibody to design amino acid sequences of VH and VL of a human CDR-grafted antibody. The designed amino acid sequences are converted into DNA sequences taking into consideration the frequency of occurrence of codons in the nucleotide sequences of antibody genes *(*Sequences of Proteins of Immunological Interest, US Dept. Health and Human Services, 1991), and DNA sequences encoding the amino acid sequences of VH and VL of the human CDR-grafted antibody are designed. Several synthetic DNAs constituting approximately 100-nucleotides are synthesized based on the designed DNA sequences, and PCR is carried out using the synthetic DNAs. It is preferred to design 6 synthetic DNAs for each of the H chain and the L chain in view of the reaction efficiency of PCR and the lengths of DNAs that can be synthesized.

Cloning into the vector for humanized antibody expression constructed in the above 2 A (1) can be easily carried out by introducing recognition sequences for appropriate restriction enzymes to the 5' ends of synthetic DNAs present on both ends. After the PCR, the amplification products are cloned into a plasmid such as pBluescript SK(-) (manufactured by STRATAGENE) and the nucleotide sequences are determined by the method described in the above 2 A (2) to obtain a plasmid carrying DNA sequences encoding the amino acid sequences of VH and VL of the desired human CDR-grafted antibody.

### (6) Modification of the amino acid sequence of V region of a human CDR-grafted antibody

It is known that a human CDR-grafted antibody prepared merely by grafting CDRs of VH and VL of the desired non-human animal antibody to FRs of VH and VL of a human antibody has a lower antigen-binding activity compared with the original non-human animal-derived antibody [BIO/TECHNOLOGY, 9, 266 (1991)]. This is probably because in VH and VL of the original non-human animal-derived antibody, not only CDRs but also some of the amino acid residues in FRs are involved directly or indirectly in the antigen-binding activity, and such amino acid residues are replaced by amino acid residues derived from FRs of VH and VL of the human antibody by CDR grafting. In order to solve this problem, attempts have been made in the preparation of a human CDR-grafted antibody to raise the lowered antigen-binding activity by identifying the amino acid residues in the amino acid sequences of FRs of VH and VL of a human antibody which are directly relating to the binding to an antigen or which are indirectly relating to it through interaction with amino acid residues in CDRs or maintenance of the tertiary structure of antibody, and modifying such amino acid residues to those derived from the original non-human animal-derived antibody [BIO/TECHNOLOGY, 9, 266 (1991)].

In the preparation of a human CDR-grafted antibody, it is most important to efficiently identify the amino acid residues in FR which are relating to the antigen-binding activity. For the efficient identification, construction and analyses of the tertiary structures of antibodies have been carried out by X ray crystallography [J. Mol. Biol., 112, 535 (1977)], computer modeling [Protein Engineering, 7, 1501 (1994*)*]*, etc.* Although these studies on the tertiary structures of antibodies have provided much information useful for the preparation of human CDR-grafted antibodies, there is no established method for preparing a human CDR-grafted antibody that is adaptable to any type of antibody. That is, at present, it is still necessary to make trial-and-error approaches, e.g., preparation of several modifications for each antibody and examination of each modification for the relationship with the antigen-binding activity.

Modification of the amino acid residues in FRs of VH and VL of a human antibody can be achieved by PCR as described in the above 2 A (5) using synthetic DNAs for modification. The nucleotide sequence of the PCR amplification product is determined by the method described in the above 2 A (2) to confirm that the desired modification has been achieved.

### (7) Construction of human CDR-grafted antibody expression vector

A human CDR-grafted antibody expression vector can be constructed by inserting the cDNAs encoding VH and VL of the human CDR-grafted antibody constructed in the above 2 A (5) and (6) into sites upstream of the genes encoding CH and CL of a human antibody in the vector for humanized antibody expression described in the above 2 A (1). For example, a human CDR-grafted antibody expression vector can be constructed by introducing recognition sequences for appropriate restriction enzymes to the 5' ends of synthetic DNAs present on both ends among the synthetic DNAs used for constructing VH and VL of the human CDR-grafted antibody in the above 2 A (5) and (6), and inserting them into sites upstream of the genes encoding CH and CL of a human antibody in the vector for humanized antibody expression described in the above 2 A (1) so as to express them in an appropriate form.

### (8) Stable production of humanized antibody

Transformants capable of stably producing a human chimeric antibody and a human CDR-grafted antibody (hereinafter collectively referred to as humanized antibody) can be obtained by introducing the humanized antibody expression vectors described in the above 2 A (4) and (7) into appropriate animal cells.

Introduction of the humanized antibody expression vector into an animal cell can be carried out by electroporation [Japanese Published Unexamined Patent Application No. 257891/90; Cytotechnology, 3, 133 (1990)], *etc.*

As the animal cell for introducing the humanized antibody expression vector, any animal cell capable of producing a humanized antibody can be used.

Examples of the animal cells include mouse myeloma cell lines NS0 and SP2/0; Chinese hamster ovary cells CHO/dhfr- and CHO/DG44, rat myeloma cell lines YB2/0 and IR983F, Syrian hamster kidney-derived BHK cell, and human myeloma cell line Namalwa. Preferred are Chinese hamster ovary cell CHO/DG44 and rat myeloma cell line YB2/0, cell described in above 1 and the like.

After the introduction of the humanized antibody expression vector, the transformant capable of stably producing the humanized antibody can be selected using a medium for animal cell culture containing a compound such as G418 sulfate (hereinafter referred to as G418; manufactured by SIGMA) according to the method described in Japanese Published Unexamined Patent Application No. 257891/90. Examples of the media for animal cell culture include RPMI1640 medium (manufactured by Nissui Pharmaceutical Co., Ltd.), GIT medium (manufactured by Nihon Pharmaceutical Co., Ltd.), EX-CELL 302 medium (manufactured by JRH), IMDM medium (manufactured by GIBCO BRL), Hybridoma-SFM medium (manufactured by GIBCO BRL), and media prepared by adding various additives such as fetal calf serum (hereinafter referred to as FCS) to these media. By culturing the obtained transformant in the medium, the humanized antibody can be formed and accumulated in the culture supernatant. The amount and the antigen-binding activity of the humanized antibody produced in the culture supernatant can be measured by enzyme-linked immunosorbent assay (hereinafter referred to as ELISA; Antibodies: A Laboratory Manual, Cold Spring Harbor Laboratory, Chapter 14, 1998; Monoclonal Antibodies: Principles and Practice, Academic Press Limited, 1996) or the like. The production of the humanized antibody by the transformant can be increased by utilizing a DHFR gene amplification system or the like according to the method described in Japanese Published Unexamined Patent Application No. 257891/90.

The humanized antibody can be purified from the culture supernatant of the transformant using a protein A column (Antibodies: A Laboratory Manual, Cold Spring Harbor Laboratory, Chapter 8, 1988; Monoclonal Antibodies: Principles and Practice, Academic Press Limited, 1996). In addition, purification methods generally employed for the purification of proteins can also be used. For example, the purification can be carried out by combinations of gel filtration, ion exchange chromatography, ultrafiltration and the like. The molecular weight of the H chain, L chain or whole antibody molecule of the purified humanized antibody can be measured by SDS-denatured polyacrylamide gel electrophoresis [hereinafter referred to as SDS-PAGE; Nature, 227, 680 (1970)], Western blotting (Antibodies: A Laboratory Manual, Cold Spring Harbor Laboratory, Chapter 12, 1988; Monoclonal Antibodies: Principles and Practice, Academic Press Limited, 1996), *etc.*

### B. Preparation of Fc fusion protein

### (1) Construction of Fc fusion protein expression vector

An Fc fusion protein expression vector is an expression vector for animal cell into which genes encoding the Fc region of a human antibody and a protein to be fused are inserted, which can be constructed by cloning each of genes encoding the Fc region of a human antibody and the protein to be fused into an expression vector for animal cell.

The Fc region of a human antibody includes those containing a part of a hinge region and/or CH1 in addition to regions containing CH2 and CH3 regions. Also, it can be any Fc region so long as at least one amino acid of CH2 or CH3 may be deleted, substituted, added or inserted, and substantially has the binding activity to the Fcγ receptor.

As the genes encoding the Fc region of a human antibody and the protein to be fused, a genomic DNA comprising exons and introns can be used. Also useful is a cDNA. The method for linking the genes and the Fc region includes PCR using each of the gene sequences as the template [Molecular Cloning: A Laboratory Manual, Cold Spring Harbor Lab. Press New York (1989), Current Protocols in Molecular Biology, John Wiley & Sons (1987-1997)].

As the expression vector for animal cells, any vector for animal cells can be used so long as it is capable of inserting and expressing the gene encoding the C region of a human antibody. Suitable vectors include pAGE107 [Cytotechnology, 3, 133 (1990)], pAGE103 [J. Biochem., 101, 1307 (1987)], pHSG274 *[*Gene, 27, 223 (1984)], pKCR [Proc. Natl. Acad. Sci. USA, 78, 1527 (1981)] and pSG1βd2-4 [Cytotechnology, 4, 173 (1990)]. Examples of the promoter and enhancer for use in the expression vector for animal cells include SV40 early promoter and enhancer [J. Biochem., 101, 1307 (1987)], LTR of Moloney mouse leukemia virus [Biochem. Biophys. Res. Commun., 149, 960 (1987)] and immunoglobulin H chain promoter *[*Cell, 41, 479 (1985)] and enhancer *[*Cell, 33, 717 (1983)].

### (2) Obtaining of DNA encoding Fc region of human antibody and protein to be fused with Fc region of human antibody

A DNA encoding the Fc region of a human antibody and the protein to be fused with the Fc region of a human antibody can be obtained in the following manner.

An mRNA extracted from a cell or tissue which expresses the desired protein to be fused with Fc, and then a cDNA is synthesized. The synthesized cDNA is cloned into a vector such as a phage or a plasmid to obtain a cDNA library. A recombinant phage or recombinant plasmid carrying a cDNA encoding the desired protein is isolated from the library by using a partial sequence of the gene of the desired protein as the probe. The full length nucleotide sequences of the desired protein on the recombinant phages or recombinant plasmids are determined, and the full length amino acid sequences are deduced from the nucleotide sequences.

As the non-human animal, any animal can be used so long as hybridoma cells can be prepared from the animal. Suitable animals include mouse, rat, hamster and rabbit.

The methods for preparing total RNA from a hybridoma cell include the guanidine thiocyanate-cesium trifluoroacetate method [Methods in Enzymol., 154, 3 (1987)], and the methods for preparing mRNA from the total RNA include the oligo (dT) immobilized cellulose column method (Molecular Cloning: A Laboratory Manual, Cold Spring Harbor Lab. Press New York, 1989). Examples of the kits for preparing mRNA from a hybridoma cell include Fast Track mRNA Isolation Kit (Invitrogen) and Quick Prep mRNA Purification Kit (manufactured by Pharmacia).

The methods for synthesizing the cDNA and preparing the cDNA library include conventional methods *(*Molecular Cloning: A Laboratory Manual, Cold Spring Harbor Lab. Press New York, 1989; Current Protocols in Molecular Biology, Supplement 1-34), or methods using commercially available kits such as SuperScript™ Plasmid System for cDNA Synthesis and Plasmid Cloning (manufactured by GIBCO BRL) and ZAP-cDNA Synthesis Kit (manufactured by STRATAGENE).

In preparing the cDNA library, the vector for inserting the cDNA synthesized using the mRNA extracted from a hybridoma cell as a template may be any vector so long as the cDNA can be inserted. Examples of suitable vectors include ZAP Express [Strategies, 5, 58 (1992)], pBluescript II SK(+) [Nucleic Acids Research, 17, 9494 (1989)], λZAP II (manufactured by STRATAGENE), λgt10, λgt11 [DNA Cloning: A Practical Approach, I, 49 (1985)], Lambda BlueMid (manufactured by Clontech), λExCell, pT7T3 18U (manufactured by Pharmacia), pcD2 [Mol. Cell. Biol., 3, 280 (1983)] and pUC18 *[*Gene, 33, 103 (1985)].

As *Escherichia coli* for introducing the cDNA library constructed with a phage or plasmid vector, any *Escherichia coli* can be used so long as the cDNA library can be introduced, expressed and maintained. Examples of suitable *Escherichia coli* include XL1-Blue MRF' [Strategies, 5, 81 (1992)], C600 [Genetics, 39, 440 (1954)], Y1088, Y1090 [Science, 222, 778 (1983)], NM522 [J. Mol. Biol., 166, 1 (1983)], K802 [J. Mol. Biol., 16, 118 (1966)] and JM105 *[*Gene, 38, 275 (1985)].

The methods for selecting the cDNA clones encoding the desired protein from the cDNA library include colony hybridization or plaque hybridization (Molecular Cloning: A Laboratory Manual, Cold Spring Harbor Lab. Press New York, 1989) using an isotope- or fluorescence-labeled probe. It is also possible to prepare the cDNAs encoding the desired protein by preparing primers and performing PCR (Molecular Cloning: A Laboratory Manual, Cold Spring Harbor Lab. Press New York, 1989; Current Protocols in Molecular Biology, Supplement 1-34) using the cDNA or cDNA library as a template.

The method for fusing the desired protein with the Fc region of a human antibody includes PCR. For example, any synthesized oligo DNAs (primers) are designed at the 5'-terminal and 3'-terminal of the gene sequence encoding the desired protein, and PCR is carried out to obtain a PCR product. In the same manner, any primers are designed for the gene sequence encoding the Fc region of a human antibody to be fused to obtain a PCR product. At this time, the primers are designed in such a manner that the same restriction enzyme site or the same gene sequence is present between the 3'-terminal of the PCR product of the protein to be fused and the 5'-terminal of the PCR product of the Fc region. When it is necessary to modify the amino acids around the linked site, mutation is introduced by using the primer into which the mutation is introduced. PCR is further carried out by using the two kinds of the obtained PCR fragments to link the genes. Also, they can be linked by carrying out ligation after treatment with the same restriction enzyme.

The nucleotide sequences of the cDNAs selected by the above methods can be determined by cleaving the cDNAs with appropriate restriction enzymes, cloning the fragments into a plasmid such as pBluescript SK(-) (manufactured by STRATAGENE), and then analyzing the sequences by generally employed sequencing methods such as the dideoxy method of Sanger, et al. [Proc. Natl. Acad. Sci. USA, 74, 5463 (1977)] or by use of nucleotide sequencers such as ABI PRISM 377 DNA Sequencer (manufactured by Applied Biosystems).

The full length amino acid sequences of an Fc fusion protein are deduced from the determined nucleotide sequences and compared with the full length amino acid sequences of the desired protein (Sequences of Proteins of Immunological Interest, US Dept. Health and Human Services, 1991), whereby it can be confirmed that the obtained cDNAs encode full length amino acid sequences which comprise the Fc fusion protein of the antibody including secretory signal sequences.

### (3) Stable production of Fc fusion protein

A transformant capable of stably producing an Fc fusion protein can be obtained by introducing the Fc fusion protein expression vector described in the 2 B (1) into an appropriate animal cell.

Introduction of the Fc fusion protein expression vector into an animal cell can be carried out by electroporation [Japanese Published Unexamined Patent Application No. 257891/90; Cytotechnology, 3, 133 (1990)], *etc.*

As the animal cell for introducing the Fc fusion protein expression vector, any animal cell capable of producing an Fc fusion protein can be used.

Examples of the animal cells include mouse myeloma cell lines NS0 and SP2/0, Chinese hamster ovary cells CHO/dhfr- and CHO/DG44, rat myeloma cell lines YB2/0 and IR983F, Syrian hamster kidney-derived BHK cell, and human myeloma cell line Namalwa. Preferred are Chinese hamster ovary cell CHO/DG44 and rat myeloma cell line YB2/0 and the host cells used in the method of the present invention described in the 1 are preferred.

After the introduction of the Fc fusion protein expression vector, the transformant capable of stably producing the Fc fusion protein can be selected using a medium for animal cell culture containing a compound such as G418 sulfate (hereinafter referred to as G418; manufactured by SIGMA) according to the method described in Japanese Published Unexamined Patent Application No. 257891/90. Examples of the media for animal cell culture include RPMI1640 medium (manufactured by Nissui Pharmaceutical Co., Ltd.), GIT medium (manufactured by Nihon Pharmaceutical Co., Ltd.), EX-CELL 302 medium (manufactured by JRH), IMDM medium (manufactured by GIBCO BRL), Hybridoma-SFM medium (manufactured by GIBCO BRL), and media prepared by adding various additives such as fetal calf serum (hereinafter referred to as FCS) to these media. By culturing the obtained transformant in the medium, the Fc fusion protein can be formed and accumulated in the culture supernatant. The amount and the antigen-binding activity of the Fc fusion protein produced in the culture supernatant can be measured by enzyme-linked immunosorbent assay (hereinafter referred to as ELISA; Antibodies: A Laboratory Manual, Cold Spring Harbor Laboratory, Chapter 14, 1998; Monoclonal Antibodies: Principles and Practice, Academic Press Limited, 1996) or the like. The production of the Fc fusion protein by the transformant can be increased by utilizing a DHFR gene amplification system or the like according to the method described in Japanese Published Unexamined Patent Application No. 257891/90.

The Fc fusion protein can be purified from the culture supernatant of the transformant using a protein A column or a protein G column (Antibodies: A Laboratory Manual, Cold Spring Harbor Laboratory, Chapter 8, 1988; Monoclonal Antibodies: Principles and Practice, Academic Press Limited, 1996). In addition, purification methods generally employed for the purification of proteins can also be used. For example, the purification can be carried out by combinations of gel filtration, ion exchange chromatography, ultrafiltration and the like. The molecular weight of the whole of the purified Fc fusion protein can be measured by SDS-denatured polyacrylamide gel electrophoresis [hereinafter referred to as SDS-PAGE; Nature, 227, 680 (1970)], Western blotting (Antibodies: A Laboratory Manual, Cold Spring Harbor Laboratory, 1988), *etc.*

Shown above are the methods for producing the antibody composition and Fc fusion protein using an animal cell as the host. As described above, the antibody composition and Fc fusion protein can also be produced using an yeast, an insect cell, a plant cell, an animal or a plant by similar methods.

When a host cell inherently has the ability to express the antibody molecule, the antibody composition of the present invention can be produced by preparing a cell expressing the antibody molecule using the method described in the above 1, culturing the cell, and then purifying the desired antibody composition from the culture.

### 3. Evaluation of the activity of the antibody composition

The protein amount, antigen-binding activity and effector function of the purified antibody composition can be measured using the known methods described in Monoclonal Antibodies: Principles and Practice, Third Edition, Acad. Press (1993), Antibody Engineering, A Practical Approach, IRL Press at Oxford University Press (1996), *etc.*

Specifically, when the antibody composition is a humanized antibody, the activity to bind to an antigen or an antigenically positive cultured cell line can be measured by ELISA, the fluorescent antibody technique [Cancer Immunol. Immunother., 36, 373 (1993)], *etc.* The cytotoxic activity against an antigenically positive cultured cell line can be evaluated by measuring CDC activity, ADCC activity, *etc.* [Cancer Immunol. Immunother., 36, 373 (1993)].

The safety and therapeutic effect of the antibody composition in human can be evaluated using an appropriate animal model of a species relatively close to human, e.g., cynomolgus monkey.

### 4. Analysis of sugar chains in the antibody composition

The sugar chain structure of antibody molecules expressed in various cells can be analyzed according to general methods of analysis of the sugar chain structure of glycoproteins. For example, a sugar chain bound to an IgG molecule consists of neutral sugars such as galactose, mannose and fucose, amino sugars such as N-acetylglucosamine, and acidic sugars such as sialic acid, and can be analyzed by techniques such as sugar composition analysis and sugar chain structure analysis using two-dimensional sugar chain mapping.

### (1) Composition analysis of neutral sugar and amino sugar

Composition analysis of the sugar chain of an antibody molecule can be analyzed by carrying out acid hydrolysis of sugar chains with trifluoroacetic acid or the like to release neutral sugars or amino sugars and analyzing the composition ratio thereof.

Specifically, the analysis can be carried out by a method using a carbohydrate analysis system (BioLC; product of Dionex). BioLC is a system for analyzing the composition of sugar by HPAEC-PAD (high performance anion-exchange chromatography-pulsed amperometric detection) [J. Liq. Chromatogr., 6, 1577 (1983)].

The composition ratio can also be analyzed by the fluorescence labeling method using 2-aminopyridine. Specifically, the composition ratio can be calculated by fluorescence labeling an acid-hydrolyzed sample by 2-aminopyridylation according to a known method [Agric. Biol. Chem., 55(1), 283-284 (1991)] and then analyzing the composition ration by HPLC.

### (2) Structure analysis of sugar chain

A structure analysis of the sugar chain of an antibody molecule can be analyzed by two-dimensional sugar chain mapping [Anal. Biochem., 171, 73 (1988); Seibutsukagaku Jikkenho *(*Biochemical Experimentation Methods) 23 - Totanpakushitsu Tosa Kenkyuho (Methods of Studies on Glycoprotein Sugar Chains), Gakkai Shuppan Center, edited by Reiko Takahashi (1989)]. The two-dimensional sugar chain mapping is a method of deducing a sugar chain structure, for example, by plotting the retention time or elution position of a sugar chain by reversed phase chromatography as the X-axis and the retention time or elution position of the sugar chain by normal phase chromatography as the Y-axis, and comparing them with the results on known sugar chains.

Specifically, a sugar chain is released from an antibody by hydrazinolysis of the antibody and subjected to fluorescence labeling with 2-aminopyridine (hereinafter referred to as PA) [J. Biochem., 95, 197 (1984)]. After being separated from an excess PA-treating reagent by gel filtration, the sugar chain is subjected to reversed phase chromatography. Then, each peak of the sugar chain is subjected to normal phase chromatography. The sugar chain structure can be deduced by plotting the obtained results on a two-dimensional sugar chain map and comparing them with the spots of a sugar chain standard (manufactured by Takara Shuzo Co., Ltd.) or those in the literature [Anal. Biochem., 171, 73 (1988)].

The structure deduced by the two-dimensional sugar chain mapping can be confirmed by carrying out mass spectrometry, e.g., MALDI-TOF-MS, of each sugar chain.

### 5. Use of antibody composition obtainable in the present invention

An antibody composition obtainable in the present invention has high ADCC activity. The antibody composition having high ADCC activity is useful for preventing and treating various diseases including cancers, inflammatory diseases, immune diseases such as autoimmune diseases and allergies, cardiovascular diseases and viral or bacterial infections.

In the case of cancers, namely malignant tumors, cancer cells grow. General anti-tumor agents inhibit the growth of cancer cells. In contrast, an antibody having high ADCC activity can treat cancers by injuring cancer cells through its cell killing effect, and therefore, it is more effective as a therapeutic agent than the general anti-tumor agents. At present, in the therapeutic agent for cancers, an anti-tumor effect of an antibody medicament alone is insufficient in many cases, so that combination therapy with chemotherapy has been carried out *[*Science, 280, 1197 (1998)]. If higher anti-tumor effect is found by the antibody composition of the present invention alone, the dependency on chemotherapy will be decreased and side effects will be reduced.

In immune diseases such as inflammatory diseases, autoimmune diseases and allergies, *in vivo* reactions of the diseases are induced by the release of a mediator molecule by immunocytes, so that the allergic reaction can be inhibited by eliminating immunocytes using an antibody having high ADCC activity.

The cardiovascular diseases include arteriosclerosis and the like. The arteriosclerosis is treated using balloon catheter at present, but cardiovascular diseases can be prevented and treated by suppressing growth of arterial cells in restricture after treatment using an antibody having high ADCC activity.

Various diseases including viral and bacterial infections can be prevented and treated by suppressing proliferation of cells infected with a virus or bacterium using an antibody having high ADCC activity.

An antibody which recognizes a tumor-related antigen, an antibody which recognizes an allergy- or inflammation-related antigen, an antibody which recognizes cardiovascular disease-related antigen, an antibody which recognizes an autoimmune disease-related antigen or an antibody which recognizes a viral or bacterial infection-related antigen are exemplified below.

The antibody which recognizes a tumor-related antigen includes anti-CA125 antibody, anti-17-1A antibody, anti-integrin αvβ3 antibody, anti-CD33 antibody, anti-CD22 antibody, anti-HLA antibody, anti-HLA-DR antibody, anti-CD20 antibody, anti-CD19 antibody, anti-EGF receptor antibody [Immunology Today, 21, 403 (2000)], anti-CD10 antibody *[*American Journal of Clinical Pathology, 113, 374 (2000); Proc. Natl. Acad. Sci. USA, 79, 4386 (1982)], anti-GD₂ antibody [Anticancer Res., 13, 331 (1993)], anti-GD₃ antibody [Cancer Immunol. Immunother., 36, 260 (1993)], anti-GM₂ antibody [Cancer Res., 54, 1511 (1994)], anti-HER2 antibody [Proc. Natl. Acad. Sci. USA, 89, 4285 (1992)], anti-CD52 antibody *[*Nature, 332, 323-327 (1988)], anti-MAGE antibody [British J. Cancer, 83, 493 (2000)], anti-HM1.24 antibody [Molecular Immunol., 36, 387 (1999)], anti-parathyroid hormone-related protein (PTHrP) antibody [Cancer, 88, 2909 (2000)], anti-FGF8 antibody [Proc. Natl. Acad. Sci. USA, 86, 9911 (1989)], anti-basic fibroblast growth factor antibody, anti-FGF8 receptor antibody [J. Biol. Chem., 265, 16455 (1990)], anti-basic fibroblast growth factor receptor antibody, anti-insulin-like growth factor antibody, anti-insulin-like growth factor receptor antibody [J. Neurosci. Res., 40, 647 (1995)], anti-PMSA antibody [J. Urology, 160, 2396 (1998)], anti-vascular endothelial cell growth factor antibody *[*Cancer Res., 57, 4593 (1997)], anti-vascular endothelial cell growth factor receptor antibody [Oncogene, 19, 2138 (2000)] and the like.

The antibody which recognizes an allergy- or inflammation-related antigen includes anti-IgE antibody, anti-CD23 antibody, anti-CD11a antibody [Immunology Today, 21, 403 (2000)], anti-CRTH2 antibody [J. Immunol., 162, 1278 (1999)], anti-CCR8 antibody (WO99/25734), anti-CCR3 antibody (US6207155), anti-interleukin 6 antibody [Immunol. Rev., 127, 5 (1992)], anti-interleukin 6 receptor antibody [Molecular Immunol., 31, 371 (1994)], anti-interleukin 5 antibody [Immunol. Rev., 127, 5 (1992)], anti-interleukin 5 receptor antibody, anti-interleukin 4 antibody [Cytokine, 3, 562 (1991)], anti-interleukin 4 receptor antibody [J. Immunol. Meth., 217, 41 (1998)], anti-tumor necrosis factor antibody [Hybridoma, 13, 183 (1994)], anti-tumor necrosis factor receptor antibody [Molecular Pharmacol., 58, 237 (2000)], anti-CCR4 antibody [Nature, 400, 776 (1999)], anti-chemokine antibody [J. Immuno. Meth., 174, 249 (1994)], anti-chemokine receptor antibody [J. Exp. Med., 186, 1373 (1997)] and the like.

The antibody which recognizes a cardiovascular disease-related antigen includes anti-GpIIb/IIIa antibody [J. Immunol., 152, 2968 (1994)], anti-platelet-derived growth factor antibody *[*Science, 253, 1129 (1991)], anti-platelet-derived growth factor receptor antibody [J. Biol. Chem., 272, 17400 (1997)], anti-blood coagulation factor antibody [Circulation, 101, 1158 (2000)] and the like.

The antibody which recognizes an antigen relating to autoimmune diseases such as psoriasis, rheumatoid arthritis, crohn' disease, uncreative colitis, systemic lupus erythema tosus, and multiple sclerosis includes an anti-auto-DNA antibody [Immunol. Letters, 72, 61 (2000)], anti-CD11a antibody, anti-ICAM3 antibody, anti-CD80 antibody, anti-CD2 antibody, anti-CD3 antibody, anti-CD4 antibody, anti-integrin α4β7 antibody, anti-CD40L antibody, anti-IL-2 receptor antibody [Immunology Today, 21, 403 (2000)] and the like.

The antibody which recognizes a viral or bacterial infection-related antigen includes anti-gp120 antibody [Structure, 8, 385 (2000)], anti-CD4 antibody [J. Rheumatology, 25, 2065 (1998)], anti-CCR5 antibody and anti-Vero toxin antibody [J. Clin. Microbiol., 37, 396 (1999)] and the like.

These antibodies can be obtained from public organizations such as ATCC (The American Type Culture Collection), RIKEN Gene Bank at The Institute of Physical and Chemical Research and National Institute of Bioscience and Human Technology, Agency of Industrial Science and Technology, or private reagent sales companies such as Dainippon Pharmaceutical, R & D SYSTEMS, PharMingen, Cosmo Bio and Funakoshi.

A pharmaceutical composition comprising the antibody composition of the present invention may be administered alone as a therapeutic agent. However, it is preferably mixed with one or more pharmaceutically acceptable carriers and provided as a pharmaceutical preparation produced by an arbitrary method well known in the technical field of pharmaceutics.

It is desirable to administer the pharmaceutical composition by the route that is most effective for the treatment. Suitable administration routes include oral administration and parenteral administration such as intraoral administration, intratracheal administration, intrarectal administration, subcutaneous administration, intramuscular administration and intravenous administration. In the case of an antibody preparation, intravenous administration is preferable.

The pharmaceutical preparation may be in the form of spray, capsules, tablets, granules, syrup, emulsion, suppository, injection, ointment, tape, and the like.

The pharmaceutical preparations suitable for oral administration include emulsions, syrups, capsules, tablets, powders and granules.

Liquid preparations such as emulsions and syrups can be prepared using, as additives, water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), flavors (e.g., strawberry flavor and peppermint), and the like.

Capsules, tablets, powders, granules, *etc.* can be prepared using, as additives, excipients (e.g., lactose, glucose, sucrose and mannitol), disintegrators (e.g., starch and sodium alginate), lubricants (e.g., magnesium stearate and talc), binders (e.g., polyvinyl alcohol, hydroxypropyl cellulose and gelatin), surfactants (e.g., fatty acid esters), plasticizers (e.g., glycerin), and the like.

The pharmaceutical preparations suitable for parenteral administration include injections, suppositories and sprays.

Injections can be prepared using carriers comprising a salt solution, a glucose solution, or a mixture thereof, *etc.* It is also possible to prepare powder injections by freeze-drying the antibody composition according to a conventional method and adding sodium chloride thereto.

Suppositories can be prepared using carriers such as cacao butter, hydrogenated fat and carboxylic acid.

The antibody composition may be administered as such in the form of spray, but sprays may be prepared using carriers which do not stimulate the oral or airway mucous membrane of a recipient and which can disperse the antibody composition as fine particles to facilitate absorption thereof

Suitable carriers include lactose and glycerin. It is also possible to prepare aerosols, dry powders, *etc.* according to the properties of the antibody composition and the carriers used. In preparing these parenteral preparations, the above-mentioned additives for the oral preparations may also be added.

The dose and administration frequency will vary depending on the desired therapeutic effect, the administration route, the period of treatment, the patient's age and body weight, *etc.* However, an appropriate dose of the active ingredient for an adult person is generally 10 µg/kg to 20 mg/kg per day.

The anti-tumor effect of the antibody composition against various tumor cells can be examined by in vitro tests such as CDC activity measurement and ADCC activity measurement and in vivo tests such as anti-tumor experiments using tumor systems in experimental animals (e.g., mice).

The CDC activity and ADCC activity measurements and anti-tumor experiments can be carried out according to the methods described in the literature [Cancer Immunology Immunotherapy, 36, 373 (1993); Cancer Research, 54, 1511 (1994); *etc.*]*.*

### Brief Description of the Drawings

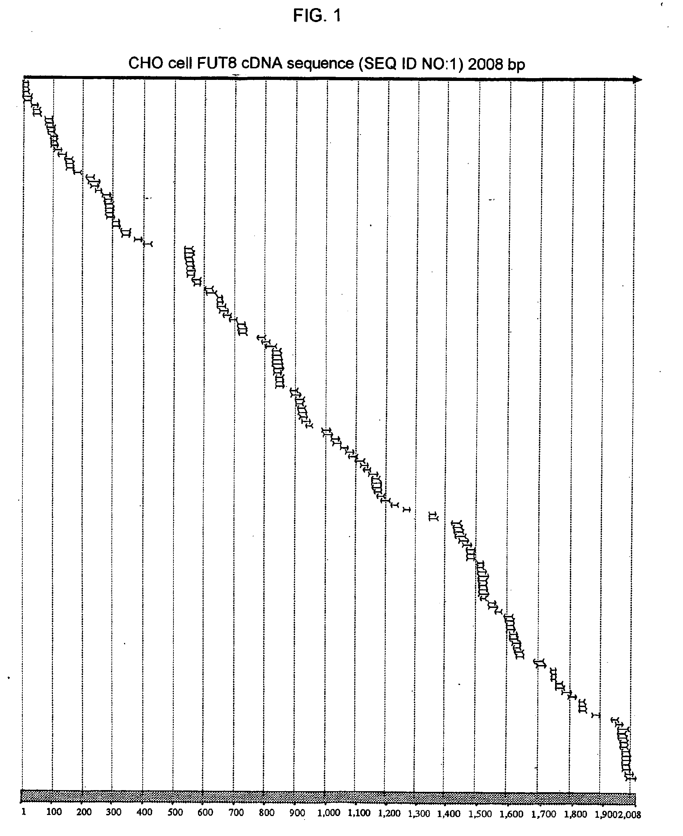

Fig. 1 shows distribution of target sequences of 159 clones obtained by the screening of effective siRNA target sequences targeting at FUT8 using an siRNA expression vector library. The moieties shown by A to J in the drawing are regions selected as the target sequences.

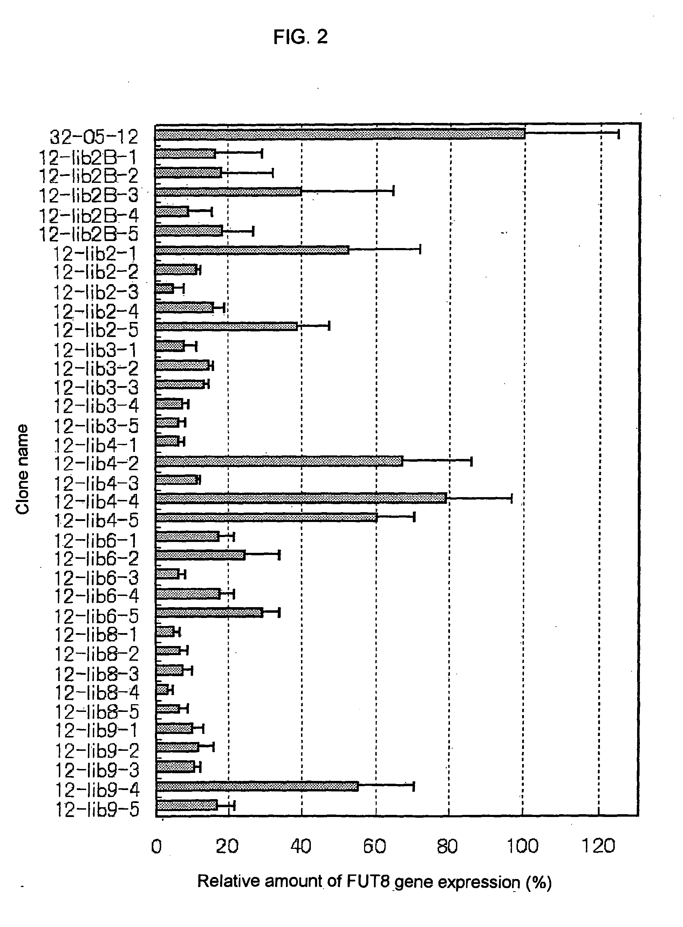

Fig. 2 shows expression levels of FUT8 gene in lectin-resistant clones obtained by introducing a FUT8-targeting siRNA expression vector, and its parent clone. The expression levels of the FUT8 gene were shown by defining, as 100, the expression level of FUT8 gene in the parent clone standardized with the expression level of β-actin gene.

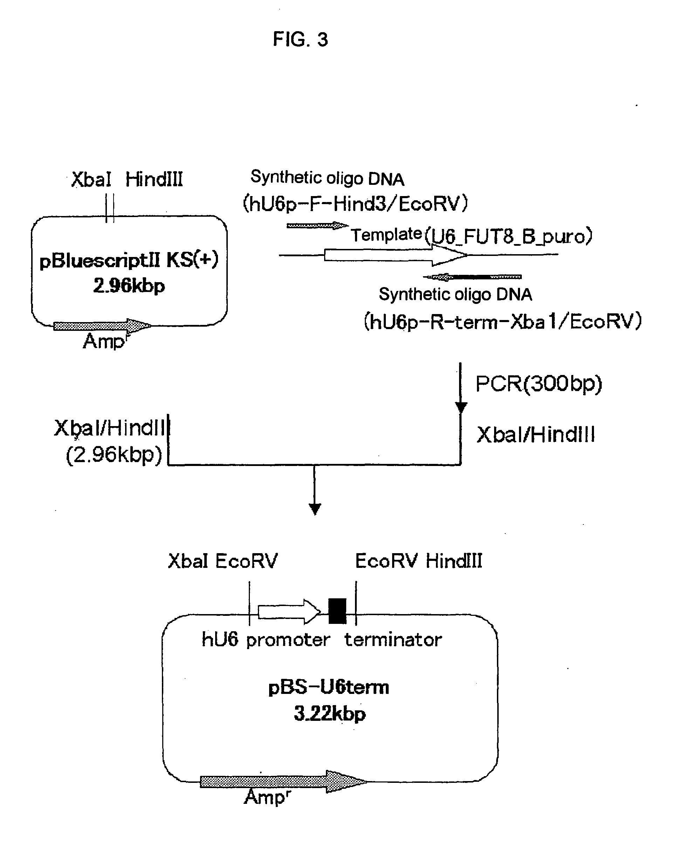

Fig. 3 shows construction of a plasmid pBS-U6term having a human U6 promoter, a cloning site and a terminator expression cassette.

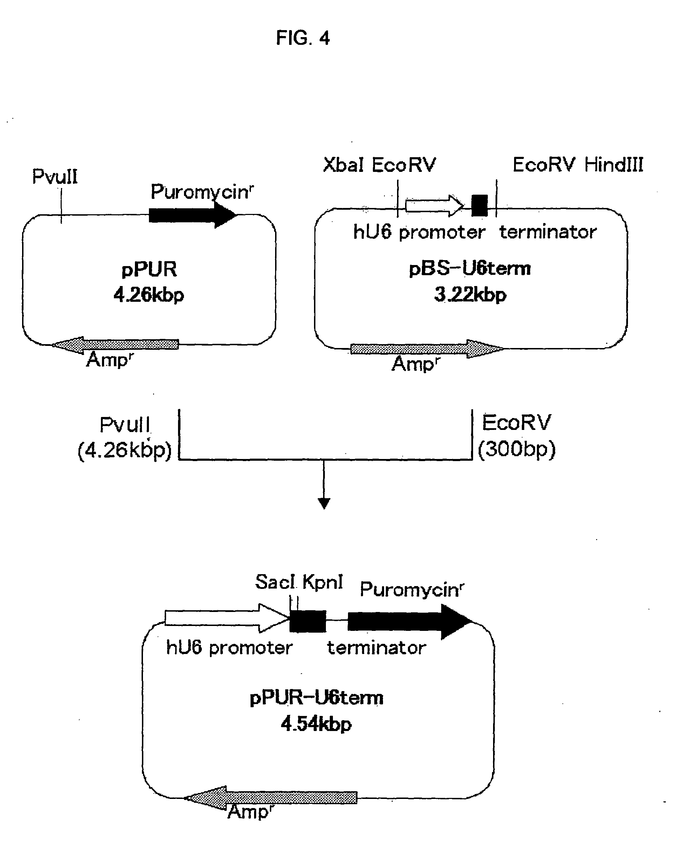

Fig. 4 shows construction of a plasmid pPUR-U6term having a human U6 promoter, a cloning site, a terminator expression cassette and a puromycin-resistant gene expression cassette.

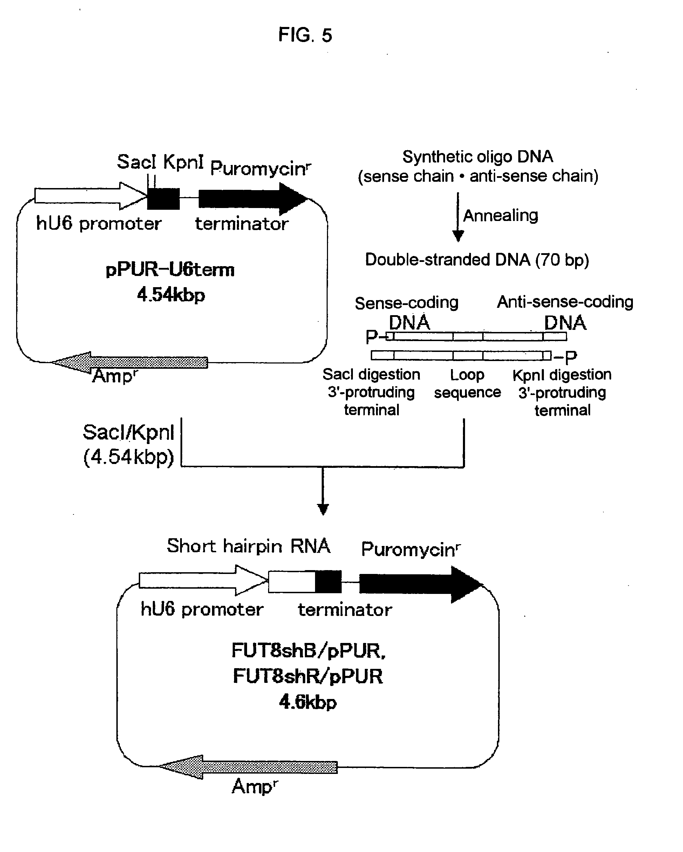

Fig. 5 shows construction of plasmids FUT8shB/pPUR and FUT8shR/pPUR having a FUT8-targeting short hairpin RNA expression cassette using human U6 promoter and a puromycin-resistant gene expression cassette.

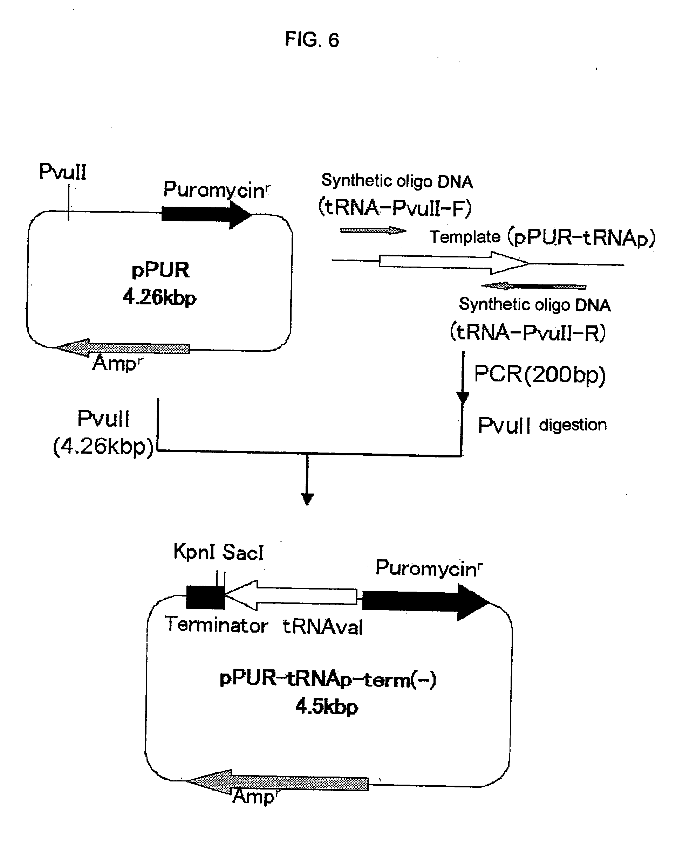

Fig. 6 shows construction of a plasmid pPUR-tRNAp-term(-) having a human tRNA-val promoter, a cloning site, a terminator expression cassette and a puromycin-resistant gene expression cassette.

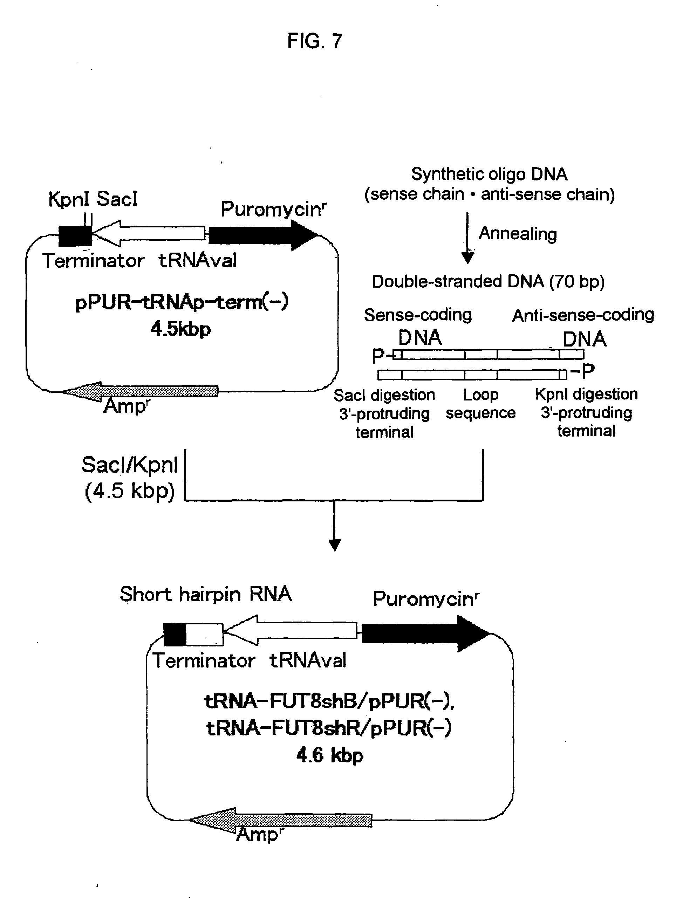

Fig. 7 shows construction of plasmids tRNA-FUT8shB/pPUR(-) and tRNA-FUT8shR/pPUR(-) having a FUT8-targeting short hairpin RNA expression cassette using a human tRNA-val promoter and a puromycin-resistant gene expression cassette.

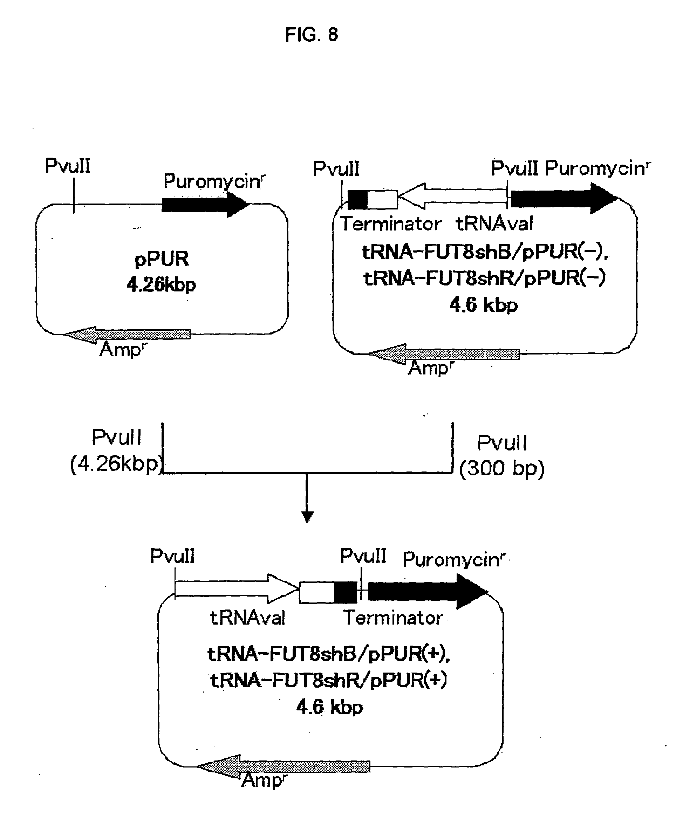

Fig. 8 shows construction of plasmids tRNA-FUT8shB/pPUR(+) and tRNA-FUT8shR/pPUR(+) having a FUT8-targeting short hairpin RNA expression cassette using a human tRNA-val promoter and a puromycin-resistant gene expression cassette.

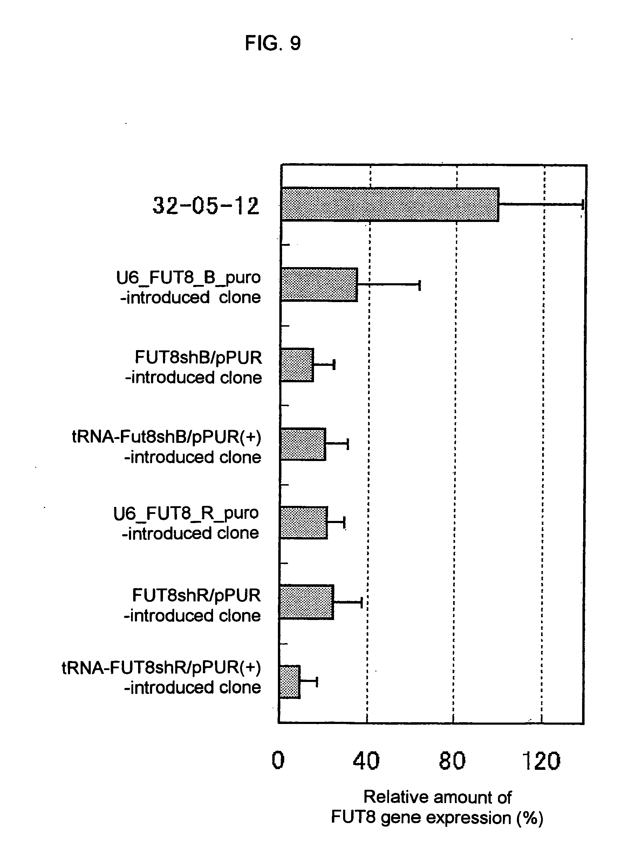

Fig. 9 shows expression levels of FUT8 gene in lectin-resistant pooled clones obtained by introducing a FUT8-targeting siRNA expression vector, and its parent cell line. The expression levels of the FUT8 gene were shown by defining, as 100, the expression level of FUT8 gene in the parent clone standardized with the expression level of β-actin gene.

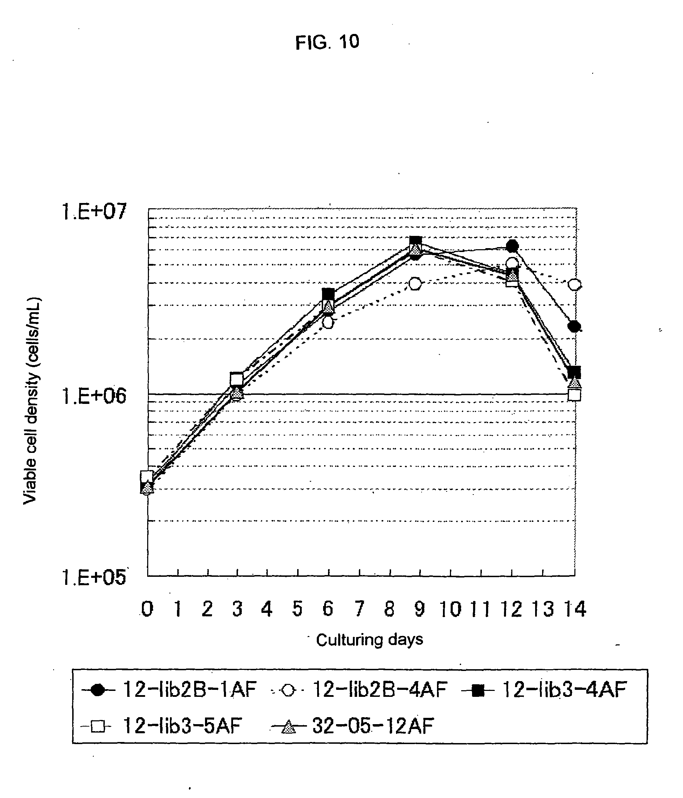

Fig. 10 shows a viable cell density at each point of time after the starting of culturing in serum-free fed-batch culturing using a lectin-resistant clone into which the FUT8-targeting siRNA expression plasmid was introduced, and which was neutralized to a serum-free medium. The abscissa shows the number of cultured days, and the ordinate shows the viable cell density by logarithm.

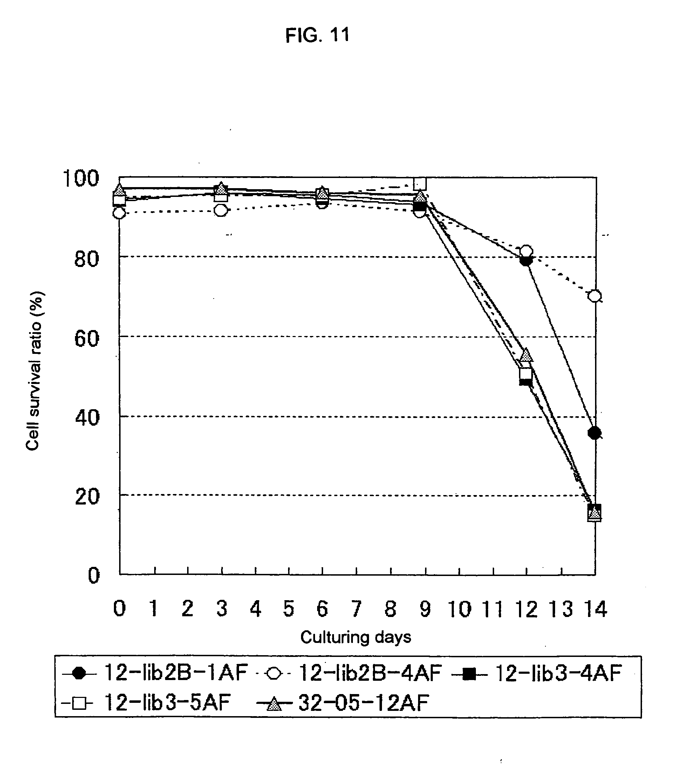

Fig. 11 shows a cell survival ratio at each point of time after the starting of culturing in serum-free fed-batch culturing using a lectin-resistant clone into which the FUT8-targeting siRNA expression plasmid was introduced, and which was neutralized to a serum-free medium. The abscissa shows the number of cultured days, and the ordinate shows the cell survival ratio.

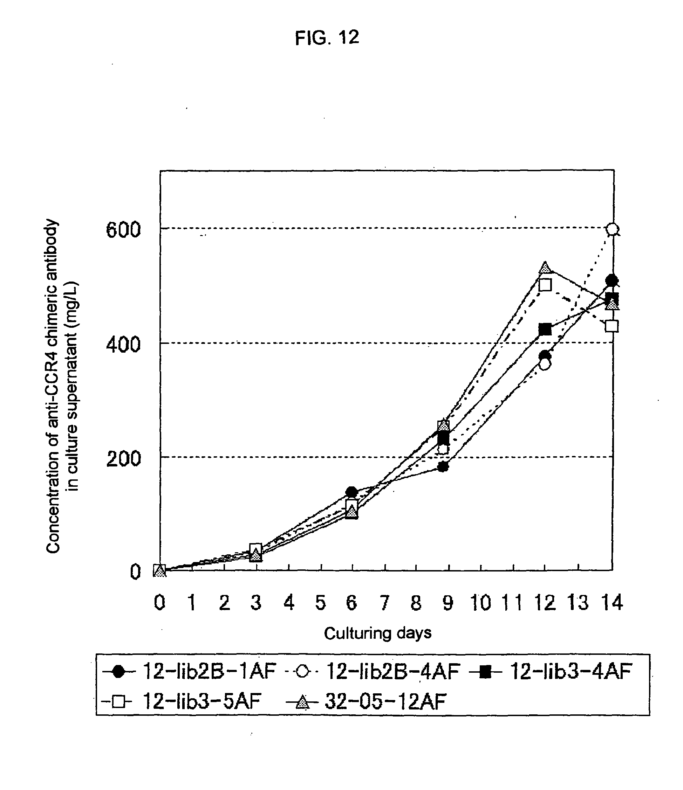

Fig. 12 shows a concentration of anti-CCR4 chimeric antibody in culture supernatant at each point of time after the starting of culturing in serum-free fed-batch culturing using a lectin-resistant clone into which the FUT8-targeting siRNA expression plasmid was introduced, and which was neutralized to a serum-free medium. The abscissa shows the number of cultured days, and the ordinate shows the antibody concentration determined by ELISA.

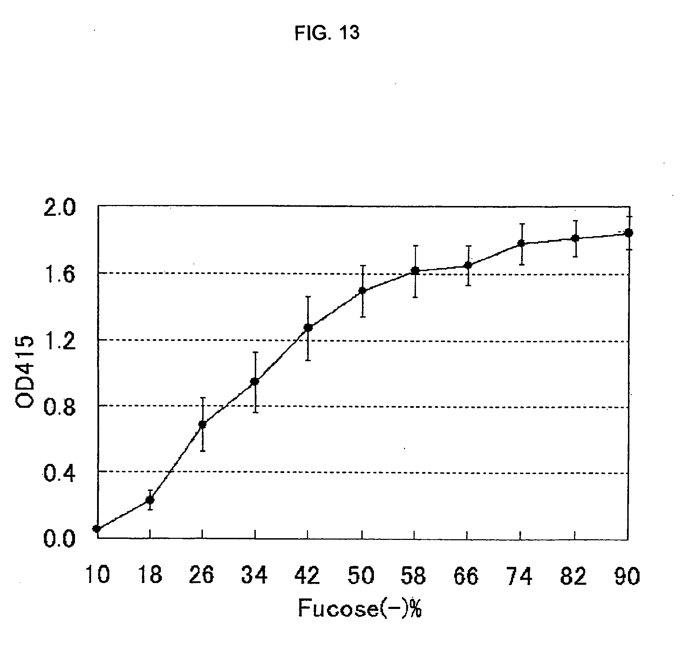

Fig. 13 shows the binding activity to shFcγRIIIa of standard antibodies having a known ratio of sugar chains in which 1-position of fucose is not bound to 6-position of N-acetylglucosamine in the reducing end through α-bond [fucose(-)%]. The abscissa shows fucose(-)% of each standard antibody, and the ordinate shows the measured value of OD415 by ELISA, indicating the binding activity to shFcγRIIIa of each standard antibody.

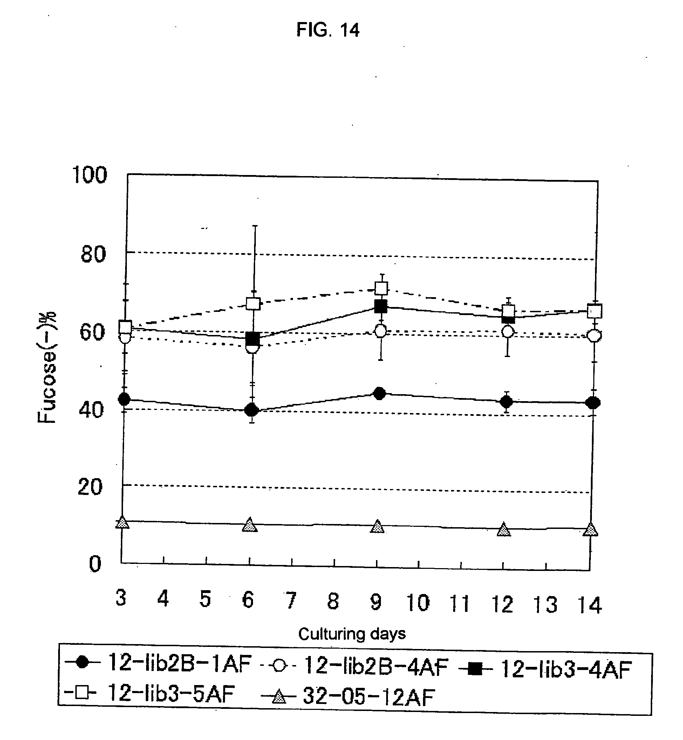

Fig. 14 shows the ratio of sugar chains in which 1-position of fucose is not bound to 6-position of N-acetylglucosamine in the reducing end through α-bond in the anti-CCR4 chimeric antibody [fucose(-)%] in culture supernatant at each point of time after the starting of culturing in serum-free fed-batch culturing using a lectin-resistant clone neutralized.to a serum-free medium. The abscissa shows the number of cultured days, and the ordinate shows fucose(-)% calculated from the results of ELISA, indicating binding activity to shFcγRIIIa.

The present invention is explained below based on Examples. However, Examples are simple illustrations and the scope of the present invention is not limited thereto.

### Examples

### Example 1

### Screening of siRNA target sequence effective for obtaining lectin-resistant clone using FUT8-targeting small interfering (si) RNA expression vector library

### 1. Construction of FUT8-targeting siRNA expression vector library FUT8shRNAlib/pPUR

### (1) Obtaining of CHO cell-derived FUT8 cDNA sequence

A cDNA encoding FUT8 was cloned from a single-stranded cDNA prepared from Chinese hamster ovary-derived CHO/DG44 cell according to the procedure described in WO00/61739.

First, 5'-untranslated region-specific forward primer (SEQ ID NO:31) and 3'-untranslated region-specific reverse primer (SEQ ID NO:32) were designed based upon the nucleotide sequence of mouse FUT8 cDNA(GenBankAcc. No. AB025198).

Then, after preparing 25 µL of a reaction solution [ExTaq buffer (manufactured by TaKaRa), 0.2 mmol/L dNTPs, 4% DMSO, and 0.5 µmol/L specific primers described above (SEQ ID NOs:31 and 32)] containing 1 µL of CHO/DG44 cell-derived single-stranded cDNA, PCR was carried out using DNA polymerase ExTaq (manufactured by TaKaRa). After heating at 94°C for 1 minute, the PCR was carried out by 30 cycles, one cycle consisting of reaction at 94°C for 30 seconds, reaction at 55°C for 30 seconds and reaction at 72°C for 2 minutes, followed by reaction at 72°C for 10 minutes.

After the PCR, the reaction solution was subjected to 0.8% agarose gel electrophoresis, and a specifically amplified fragment (about 2 kb) was recovered. The DNA fragment was ligated to plasmid pCR2.1 using TQPO TA cloning Kit (manufactured by Invitrogen) according to the manufacturer's instruction, and *E. coli* DH5α was transformed with the ligation solution. Among the resulting kanamycin-resistant colonies, plasmid DNAs were isolated from 8 clones with which cDNA was inserted according to the known method.

After reaction using BigDye Terminator Cycle Sequencing FS Ready Reaction Kit (manufactured by Applied Biosystems) according to the manufacturer's instruction, the sequence of cDNA of each isolated plasmid were analyzed using DNA sequencer ABI PRISM 377 manufactured by Applied Biosystems. It was confirmed that all the cDNAs inserted in the plasmids were cDNA encoding full-length Chinese hamster FUT8 ORF. Among cDNA inserted in the plasmid DNAs whose sequences were determined, a plasmid DNA, free of readout errors of nucleotide resulting from PCR, was selected. Hereinafter, the plasmid is referred to as "CHfFUT8-pCR2.1". The nucleotide sequence of Chinese hamster FUT8 cDNA, determined in this manner, is represented by SEQ ID NO:1.

### (2) Preparation of FUT8-targeting siRNA expression vector library

Human tRNA-val promoter type FUT8-targeting siRNA expression vector library was constructed using CHfFUT8-pCR2.1 obtained in the (1), in the same manner as the method described in Example 13 of WO03/46186. Also, pPUR (manufactured by CLONTECH) was used as a vector, using a recognition sequence of a restriction enzyme *Bam*HI as a loop sequence between antisense and sense DNAs. Hereinafter, the prepared library is referred to as "FUT8shRNAlib/pPUR/DH10B".

LB agar medium containing 100 µg/mL ampicillin was prepared using sterilized dishes [243 mm × 243 mm × 18 mm (manufactured by Nalgenunc)], and 50 µL/dish FUTBshRNAlib/pPUR/DH10B glycerol stock was plated. After stationary culture overnight at 37°C, the *E. coli* on the plates were collected in suspension with sterilized water, and a plasmid DNA was recovered according to the known method. Hereinafter, the recovered plasmid is referred to as "FUT8shRNAlib/pPUR".

### 2. Obtaining of lectin-resistant clone into which FUT8-targeting siRNA expression library was introduced

FUT8-targeting siRNA expression library plasmid, FUT8shRNAlib/pPUR obtained in item 1 of this Example was introduced into clone 32-05-12 which is one of the anti-CCR4 chimeric antibody producing clones obtained by the method described in Reference Example using CHO/DG44 cell as the host cell, and clones resistant to LCA, a lectin which specifically recognizes α1,6-fucose, were isolated as follows.

Plasmid FUT8shRNAlib/pPUR obtained in the item 1 of this Example was digested with a restriction enzyme *Fsp*I (manufactured by New England Biolabs) to be linearized, and after 10 µg of the linearized plasmid FUT8shRNAlib/pPUR was introduced into 1.6×10⁶ cells of clone 32-05-12 by electroporation [Cytotechnology, 3, 133 (1990)], the cells were suspended in a basal medium [IMDM (manufactured by Invitrogen) containing 10% fetal bovine serum (manufactured by Invitrogen), 50 µg/mL gentamicin (manufactured by Nacalai Tesque), and 500 nmol/L MTX (manufactured by SIGMA)], and inoculated at 8 mL into 3 dishes of 10 cm for adherent cell culture (manufactured by Falcon). Also, transfection was carried out 10 times under the same conditions, and the cells were cultured in a total of 30 culture dishes of 10 cm. After culturing in a 5% CO₂ incubator at 37°C for 24 hours, the medium was exchanged with 8 mL of a basal medium containing 12 µg/mL puromycin (manufactured by SIGMA). After culturing in a 5% CO₂ incubator at 37°C for 7 days, the medium was exchanged with 8 mL of a basal medium containing 12 µg/mL puromycin (manufactured by SIGMA) and 0.5 mg/mL LCA (manufactured by VECTOR), and the culture was continued for further 6 to 8 days to isolate lectin-resistant clones.

### 3. Analysis of target sequence of FUT8-targeting siRNA expression plasmid

### (1) Isolation of siRNA expression cassette on genomic DNA of lectin-resistant clone

siRNA expression cassette was isolated from genomic DNA of lectin-resistant clones obtained in the item 2 of this Example as follows.

Lectin-resistant clones were collected into a flat-bottom plate for adherent cells (manufactured by Greiner) according to the known method [Gene Targeting, Oxford University Press (1993)], and cultured in a basal medium containing 12 µg/mL puromycin (manufactured by SIGMA) at 37°C for 1 week in the 5% CO₂ incubator.

After culturing, each clone of the plate was treated with trypsin, and dispensed onto 2 flat-bottom 96-well plates for adherent cells (manufactured by Greiner). One plate was used as a replica plate, and another was freeze-stored as a master plate. After the replica plate was cultured in a basal medium containing 12 µg/mL puromycin (manufactured by SIGMA) at 37°C for 1 week in a 5% CO₂ incubator, genomic DNA was prepared from each clone according to the known method [Analytical Biochemistry, 201, 331 (1992)], and dissolved in 30 µL each of TE-RNase buffer (pH 8.0) [10 mmol/L Tris-HCl, 1 mmol/L EDTA, and 200 µg/mL RNase A] overnight, then diluted at 0.05 µg/µL with sterilized water.

In addition, a forward primer which binds to the upstream of the tRNA-val promoter region of the siRNA expression cassette (SEQ ID NO:33) and a reverse primer which binds to the downstream of the terminator sequence of the siRNA expression cassette (SEQ ID NO:34) were each designed for FUT8-targeting siRNA expression plasmid, FUT8shRNAlib/pPUR.

Polymerase chain reaction (PCR) was carried out with KOD polymerase (manufactured by TOYOBO), using the genomic DNA prepared from each clone as a template. A reaction solution (50 µL) [KOD Buffer1 (manufactured by TOYOBO), 0.2 mmol/L dNTPs, 1 mmol/L MgCl₂, and 0.4 µmol/L of the above primers (SEQ ID NOs:33 and 34)] containing 5 µL of the genomic DNA solution described above was prepared for each clone, and after heating at 94°C for 1 minute, the PCR was carried out by 25 cycles, one cycle consisting of reaction at 97°C for 10 seconds and reaction at 68°C for 30 seconds.

After the PCR, the reaction solution was subjected to agarose gel electrophoresis, and the amplified fragment (about 300 bp) containing the siRNA expression cassette region was recovered.

Also, 2 µg of plasmid pPUR (manufactured by CLONTECH) was digested with a restriction enzyme *Pvu*II (manufactured by New England Biolabs) at 37°C overnight. After the digestion reaction, the reaction solution was subjected to agarose gel electrophoresis, and a *Pvu*II fragment of about 4.3 kb was recovered. The PCR-amplified fragment (about 300 bp) obtained above was ligated to a *Pvu*II fragment derived from plasmid pPUR using Ligation High (manufactured by TOYOBO) in the presence of the restriction enzyme *Pvu*II. *E. coli* DH5α was transformed with the reaction solution. Plasmid DNAs were isolated using QIAprep spin Mini prep Kit (manufactured by QIAGEN) from a number of obtained ampicillin-resistant colonies according to the known method.

### (2) Analysis of target sequence contained in the siRNA expression unit

FUT8-targeting sequences contained in the siRNA expression cassette of the plasmids obtained in the item (1) were analyzed

First, after reaction with BigDye Terminator v3:0 Cycle sequencing Kit (manufactured by Applied Biosystems) according to the manufacturer's instruction, nucleotide sequences of siRNA expression cassette which were inserted into each plasmid DNA obtained in the item (1) were analyzed using DNA sequencer ABI PRISM 377 (manufactured by Applied Biosystems). Among nucleotide sequences determined for 159 clones, homology of target sequences against FUT8 was compared with the sequence of CHO cell FUT8 cDNA (SEQ ID NO:1), and distribution of respective target sequences in the nucleotide sequence represented by SEQ ID NO:1 and the start points and end points of each target sequence, which corresponded to SEQ ID NO:1, are shown in Fig. 1.

**Table 1**

| Clone No. | Start point of target sequence | End point of target sequence | Target sequence length (bp) |
|---|---|---|---|
| 1 | 1 | 19 | 19 |
| 2 | 1 | 20 | 20 |
| 3 | 1 | 22 | 22 |
| 4 | 2 | 31 | 30 |
| 5 | 5 | 30 | 26 |
| 6 | 29 | 53 | 25 |
| 7 | 35 | 60 | 26 |
| 8 | 35 | 62 | 28 |
| 9 | 76 | 103 | 28 |
| 10 | 78 | 105 | 28 |
| 11 | 83 | 112 | 30 |
| 12 | 87 | 112 | 26 |
| 13 | 95 | 120 | 26 |
| 14 | 96 | 120 | 25 |
| 15 | 97 | 121 | 25 |
| 16 | 109 | 133 | 25 |
| 17 | 121 | 146 | 26 |
| 18 | 144 | 170 | 27 |
| 19 | 148 | 174 | 27 |
| 20 | 150 | 174 | 25 |
| 21 | 175 | 200 | 26 |
| 22 | 216 | 242 | 27 |
| 23 | 221 | 260 | 40 |
| 24 | 230 | 256 | 27 |
| 25 | 245 | 267 | 23 |
| 26 | 268 | 296 | 29 |
| 27 | 275 | 300 | 26 |
| 28 | 276 | 306 | 31 |
| 29 | 278 | 308 | 31 |
| 30 | 279 | 306 | 28 |
| 31 | 283 | 309 | 27 |
| 32 | 301 | 326 | 26 |
| 33 | 302 | 328 | 27 |
| 34 | 330 | 361 | 32 |
| 35 | 334 | 359 | 26 |
| 36 | 372 | 398 | 27 |
| 37 | 401 | 428 | 28 |
| 38 | 534 | 563 | 30 |
| 39 | 534 | 566 | 33 |
| 40 | 536 | 563 | 28 |
| 41 | 539 | 565 | 27 |
| 42 | 543 | 567 | 25 |
| 43 | 543 | 570 | 28 |
| 44 | 545 | 569 | 25 |
| 45 | 561 | 589 | 29 |
| 46 | 567 | 589 | 23 |
| 47 | 603 | 629 | 27 |
| 48 | 608 | 640 | 33 |
| 49 | 642 | 660 | 19 |
| 50 | 642 | 663 | 22 |
| 51 | 642 | 670 | 29 |
| 52 | 650 | 679 | 30 |
| 53 | 663 | 689 | 27 |
| 54 | 682 | 708 | 27 |
| 55 | 710 | 736 | 27 |
| 56 | 711 | 741 | 31 |
| 57 | 713 | 740 | 28 |
| 58 | 774 | 801 | 28 |
| 59 | 789 | 816 | 28 |
| 60 | 802 | 836 | 35 |
| 61 | 824 | 850 | 27 |
| 62 | 824 | 852 | 29 |
| 63 | 824 | 854 | 31 |
| 64 | 824 | 857 | 34 |
| 65 | 827 | 858 | 32 |
| 66 | 828 | 853 | 26 |
| 67 | 834 | 858 | 25 |
| 68 | 834 | 858 | 25 |
| 69 | 834 | 860 | 27 |
| 70 | 880 | 906 | 27 |
| 71 | 886 | 913 | 28 |
| 72 | 898 | 926 | 29 |
| 73 | 900 | 922 | 23 |
| 74 | 905 | 930 | 26 |
| 75 | 907 | 934 | 28 |
| 76 | 912 | 937 | 26 |
| 77 | 917 | 946 | 30 |
| 78 | 932 | 952 | 21 |
| 79 | 950 | 968 | 19 |
| 80 | 986 | 1013 | 28 |
| 81 | 990 | 1019 | 30 |
| 82 | 1015 | 1042 | 28 |
| 83 | 1022 | 1049 | 28 |
| 84 | 1046 | 1071 | 26 |
| 85 | 1062 | 1089 | 28 |
| 86 | 1073 | 1102 | 30 |
| 87 | 1095 | 1124 | 30 |
| 88 | 1112 | 1137 | 26 |
| 89 | 1122 | 1145 | 24 |
| 90 | 1138 | 1169 | 32 |
| 91 | 1149 | 1174 | 26 |
| 92 | 1149 | 1182 | 34 |
| 93 | 1150 | 1181 | 32 |
| 94 | 1157 | 1181 | 25 |
| 95 | 1166 | 1191 | 26 |
| 96 | 1180 | 1207 | 28 |
| 97 | 1211 | 1237 | 27 |
| 98 | 1254 | 1278 | 25 |
| 99 | 1340 | 1365 | 26 |
| 100 | 1340 | 1370 | 31 |
| 101 | 1416 | 1445 | 30 |
| 102 | 1422 | 1448 | 27 |
| 103 | 1425 | 1453 | 29 |
| 104 | 1428 | 1460 | 33 |
| 105 | 1441 | 1468 | 28 |
| 106 | 1451 | 1480 | 30 |
| 107 | 1463 | 1491 | 29 |
| 108 | 1464 | 1489 | 26 |
| 109 | 1465 | 1490 | 26 |
| 110 | 1498 | 1517 | 20 |
| 111 | 1498 | 1517 | 20 |
| 112 | 1499 | 1526 | 28 |
| 113 | 1501 | 1534 | 34 |
| 114 | 1502 | 1529 | 28 |
| 115 | 1504 | 1529 | 26 |
| 116 | 1504 | 1530 | 27 |
| 117 | 1504 | 1534 | 31 |
| 118 | 1508 | 1526 | 19 |
| 119 | 1532 | 1557 | 26 |
| 120 | 1535 | 1563 | 29 |
| 121 | 1555 | 1578 | 24 |
| 122 | 1584 | 1612 | 29 |
| 123 | 1588 | 1615 | 28 |
| 124 | 1591 | 1615 | 25 |
| 125 | 1591 | 1619 | 29 |
| 126 | 1602 | 1626 | 25 |
| 127 | 1602 | 1629 | 28 |
| 128 | 1610 | 1637 | 28 |
| 129 | 1613 | 1637 | 25 |
| 130 | 1619 | 1645 | 27 |
| 131 | 1622 | 1647 | 26 |
| 132 | 1680 | 1707 | 28 |
| 133 | 1687 | 1713 | 27 |
| 134 | 1729 | 1746 | 18 |
| 135 | 1730 | 1746 | 17 |
| 136 | 1730 | 1746 | 17 |
| 137 | 1744 | 1758 | 15 |
| 138 | 1744 | 1768 | 25 |
| 139 | 1744 | 1773 | 30 |
| 140 | 1765 | 1796 | 32 |
| 141 | 1786 | 1811 | 26 |
| 142 | 1821 | 1839 | 19 |
| 143 | 1821 | 1842 | 22 |
| 144 | 1821 | 1844 | 24 |
| 145 | 1863 | 1890 | 28 |
| 146 | 1927 | 1951 | 25 |
| 147 | 1940 | 1965 | 26 |
| 148 | 1948 | 1984 | 37 |
| 149 | 1949 | 1976 | 28 |
| 150 | 1951 | 1979 | 29 |
| 151 | 1957 | 1982 | 26 |
| 152 | 1957 | 1982 | 26 |
| 153 | 1963 | 1987 | 25 |
| 154 | 1963 | 1989 | 27 |
| 155 | 1963 | 1990 | 28 |
| 156 | 1964 | 1987 | 24 |
| 157 | 1965 | 1990 | 26 |
| 158 | 1974 | 2000 | 27 |
| 159 | 1978 | 2008 | 31 |

Among target sequences of the 159 clones, the representative target regions are shown by A to J in the drawing. Regarding the nucleotide sequences of FUT8 corresponding to the regions A to J, the region A is represented by SEQ ID NO:9, the region B is represented by SEQ ID NO:10, the region C is represented by SEQ ID NO:11, the region D is represented by SEQ ID NO:18, the region E is represented by SEQ ID NO:12, the region F is represented by SEQ ID NO:17, the region G is represented by SEQ ID NO:13, the region H is represented by SEQ ID NO:14, the region I is represented by SEQ ID NO:15, and the region J is represented by SEQ ID NO:16. In this connection, among the respective plasmids obtained in the item (1), the siRNA expression plasmid using a sequence contained in the SEQ ID NO:9 as the target sequence is hereinafter named FUT8shRNA/lib1/pPUR, the siRNA expression plasmid using a sequence contained in the SEQ ID NO:10 as the target sequence is hereinafter named FUT8shRNA/lib2/pPUR, the siRNA expression plasmid using a sequence contained in the SEQ ID NO:11 as the target sequence is hereinafter named FUT8shRNA/lib3/pPUR, the siRNA expression plasmid using a sequence contained in the SEQ ID NO:12 as the target sequence is hereinafter named FUT8shRNA/lib4/pPUR, the siRNA expression plasmid using a sequence contained in the SEQ ID NO:13 as the target sequence is hereinafter named FUT8shRNA/lib5/pPUR, the siRNA expression plasmid using a sequence contained in the SEQ ID NO:14 as the target sequence is hereinafter named FUT8shRNA/lib6/pPUR, the siRNA expression plasmid using a sequence contained in the SEQ ID NO:15 as the target sequence is hereinafter named FUT8shRNA/lib7/pPUR, the siRNA expression plasmid using a sequence contained in the SEQ ID NO:16 as the target sequence is hereinafter named FUTBshRNA/lib8/pPUR, the siRNA expression plasmid using a sequence contained in the SEQ ID NO:17 as the target sequence is hereinafter named FUTBshRNA/lib9/pPUR, and the siRNA expression plasmid using a sequence contained in the SEQ ID NO:18 as the target sequence is hereinafter named FUT8shRNA/lib10/pPUR.

### (3) Search of mouse, rat and human homologous sequences of target sequences contained in siRNA expression unit

Sequences corresponding to the target sequences represented by SEQ ID NOs:9 to 18 obtained in the item (2) were searched in mouse, rat and human FUT8 sequences as follows.

SEQ ID NOs:2, 3, and 4 show mouse, rat and human FUT8 sequences, respectively. Among the sequences, sequences corresponding to the target sequences represented by SEQ ID NOs:9 to 18 obtained in the item (2) were searched. In this search, completely matched with the target sequences represented by SEQ ID NOs:9 to 18 were excluded.

Each sequence number of the selected sequences is shown below. Mouse FUT8 sequence corresponding to SEQ ID NO:10 is represented by SEQ ID NO:19; human FUT8 sequence corresponding to SEQ ID NO:10 is represented by SEQ ID NO:20; human FUT8 sequence corresponding to SEQ ID NO:11 is represented by SEQ ID NO:21; human, mouse and rat FUT8 sequence corresponding to SEQ ID NO:12 is represented by SEQ ID NO:22; mouse FUT8 sequence corresponding to SEQ ID NO:13 is represented by SEQ ID NO:23; human FUT8 sequence corresponding to SEQ ID NO:13 is represented by SEQ ID NO:24; rat FUT8 sequences corresponding to SEQ ID NO:13 are represented by SEQ ID NO:25; mouse and rat FUT8 sequence corresponding to SEQ ID NO:14 is represented by SEQ ID NO:26; human FUT8 sequence corresponding to SEQ ID NO:14 is represented by SEQ ID NO:27; mouse FUT8 sequence corresponding to SEQ ID NO:15 is represented by SEQ ID NO:28; human FUT8 sequence corresponding to SEQ ID NO:15 is represented by SEQ ID NO:29; rat FUT8 sequence corresponding to SEQ ID NO:17 is represented by SEQ ID NO:30.

### Example 2

### Preparation of lectin-resistant CHO/DG44 cell by introducing FUT8-targeting siRNA expression plasmid and determination of the amount of FUT8 mRNA in the cell

### 1. Obtaining of lectin-resistant clone into which FUT8-targeting siRNA expression plasmid was introduced

Each of the siRNA expression plasmids FUT8shRNA/lib1/pPUR, FUT8shRNA/lib2/pPUR, FUT8shRNA/lib3/pPUR, FUT8shRNA/lib4/pPUR, FUT8shRNA/lib5/pPUR, FUT8shRNA/lib6/pPUR, FUT8shRNA/lib7/pPUR, FUT8shRNA/lib8/pPUR, FUT8shRNA/lib9/pPUR and FUT8shRNA/lib10/pPUR obtained in the item 3(1) of Example 1 was introduced into the clone 32-05-12 described in Reference Example to thereby obtain LCA-resistant clones.

Each of the siRNA expression plasmids described in the above was digested with a restriction enzyme *Fsp*I (manufactured by New England Biolabs) to be linearized, 10 µg of each of the linearized siRNA expression plasmids was introduced into 1.6×10⁶ cells of the clone 32-05-12 by electroporation [Cytotechnology, 3, 133 (1990)], and then the cells were suspended in a basal medium [IMDM (manufactured by Invitrogen) containing 10% fetal bovine dialyzed serum (manufactured by Invitrogen), 50 µg/mL gentamicin (manufactured by Nacalai Tesque) and 500 nmol/l MTX (manufactured by SIGMA)] and inoculated at 8 mL into four 10 cm-dishes for adhesion cell culture (manufactured by Falcon). After culturing them at 37°C for 24 hours in a 5% CO₂ incubator, the medium was exchanged with 8 mL of the basal medium containing puromycin (manufactured by SIGMA) at a concentration of 12 µg/mL. After culturing them at 37°C for 7 days in a 5% CO₂ incubator, the medium was exchanged with 8 mL of the basal medium containing puromycin (manufactured by SIGMA) at a concentration of 12 µg/mL and LCA (manufactured by VECTOR) at a concentration of 0.5 mg/mL, followed by culturing for further 6 to 8 days to obtain lectin-resistant clones. The culturing was further carried out for 6 to 8 days to obtain lectin-resistant clones. Hereinafter, the lectin-resistant clone into which the siRNA expression plasmid FUT8shRNA/lib1/pPUR was introduced is named 12-lib1, the lectin-resistant clone into which the siRNA expression plasmid FUT8shRNA/lib2/pPUR was introduced is named 12-lib2, the lectin-resistant clone into which the siRNA expression plasmid FUT8shRNA/lib3/pPUR was introduced is named 12-lib3, the lectin-resistant clone into which the siRNA expression plasmid FUT8shRNA/lib4/pPUR was introduced is named 12-lib4, the lectin-resistant clone into which the siRNA expression plasmid FUT8shRNA/lib5/pPUR was introduced is named 12-lib5, the lectin-resistant clone into which the siRNA expression plasmid FUT8shRNA/lib6/pPUR was introduced is named 12-lib6, the lectin-resistant clone into which the siRNA expression plasmid FUT8shRNA/lib7/pPUR was introduced is named 12-lib7, the lectin-resistant clone into which the siRNA expression plasmid FUT8shRNA/lib8/pPUR was introduced is named 12-lib8, the lectin-resistant clone into which the siRNA expression plasmid FUT8shRNA/lib9/pPUR was introduced is named 12-lib9, and the lectin-resistant clone into which the siRNA expression plasmid FUT8shRNA/lib10/pPUR was introduced is named 12-lib10, respectively.

### 2. Determination of the amount of FUT8 mRNA in lectin-resistant clone into which FUT8-targeting siRNA expression plasmid was introduced

The amount of FUT8 mRNA was determined in the lectin-resistant clones 12-lib1, 12-lib2, 12-lib3, 12-lib4, 12-lib5, 12-lib6, 12-lib7, 12-lib8, 12-lib9, 12-lib10 obtained in the item 1 of this Example and the clone 32-05-12 which is the parent clone of the lectin-resistant clones.

Each of the above-mentioned lectin-resistant clones was suspended at a cell density of 3×10⁵ cells/mL in a basal medium [Iscove's modified Dulbecco's medium (manufactured by Invitrogen) containing 10% fetal bovine dialyzed serum (manufactured by Invitrogen), 50 µg/mL gentamicin (manufactured by Nacalai Tesque) and 500 nmol/L MTX (manufactured by SIGMA)] supplemented with puromycin (manufactured by SIGMA) at a concentration of 12 µg/ml, inoculated into a T25 flask for adhesion cell (manufactured by Greiner), cultured at 37°C for 3 days in a 5% CO₂ incubator, and then treated with trypsin. Each of the cell suspensions obtained by the trypsin treatment was centrifuged for 5 minutes under conditions of 3000 rpm and 4°C, the supernatant was discarded, and the cells were suspended in Dulbecco's PBS buffer (manufactured by Invitrogen). After centrifugation again for 5 minutes under conditions of 3000 rpm and 4°C twice, the cells were frozen at -80°C. In addition, the parent clone 32-05-12 was also cultured in the same manner using the basal medium free from puromycin, and the cells were recovered.

Each of the cells obtained in the above was thawed at room temperature, and then total RNA was extracted using RNAeasy (manufactured by QIAGEN) in accordance with the manufacture's instructions. The thus obtained total RNA was dissolved in 45 µL of sterile water and subjected to a DNase treatment to degrade genomic DNA contaminated in each sample. After the reaction, each total RNA was again purified using RNAeasy (manufactured by QIAGEN) and dissolved in 40 µL of sterile water.

A single-stranded cDNA was synthesized from 3 µg of each of the thus obtained total RNAs by carrying out the reverse transcription reaction with oligo(dT) primers using SUPERSCRIPT™ Preamplification System for First Strand cDNA Synthesis (manufactured by Invitrogen) in accordance with the manufacture's instructions.

The transcription level of FUT8 gene and the transcription level of β-actin gene by competitive PCR were determined in the following manner.

An aqueous solution prepared by diluting the reaction solution containing the above-mentioned single-stranded cDNA 50-fold with sterile water was stored at -80°C until use. By carrying out competitive PCR using total cDNA derived from each clone as the template in accordance with the method described in Example 8 of WO 00/61739, the amount of FUT8 mRNA and the amount of β-actin mRNA of the total RNA derived from each clone were measured. When the relative value of the amount of FUT8 mRNA to the amount of β-actin mRNA was calculated based on the assumption that the amount of β-actin mRNA is the same degree among different cells, the amount of FUT8 mRNA was decreased in the lectin-resistant clones obtained by introducing the FUT8-targeting siRNA expression plasmid, in comparison with the parent clone.

### Example 3

### Obtaining of lectin-resistant clone into which FUT8-targeting siRNA expression plasmid was introduced, and production of antibody composition using the cells

### 1. Obtaining of lectin-resistant clone into which FUT8-targeting siRNA expression plasmid was introduced and culturing thereof

### (1) Preparation of lectin-resistant clone into which FUT8-targeting siRNA expression plasmid

In the lectin-resistant clones obtained in the item 2 of Example 2, a difference in the appearance frequency of resistant clone was found in response to each target sequence of the siRNA expression plasmid introduced in obtaining the clone. Accordingly, the following examination was carried out for the purpose of further analyzing the target sequences having high appearance frequency of resistant clones.

From the target sequences of siRNA for FUT8 obtained in the item 3(1) of Example 1, an siRNA expression plasmid FUT8shRNA/lib2/pPUR using the 31 nucleotides represented by SEQ ID NO:10 as the target sequence, an siRNA expression plasmid FUT8shRNA/lib2B/pPUR using the 26 nucleotides at the 5'-terminal contained in SEQ ID NO:10 as the target sequence, an siRNA expression plasmid FUT8shRNA/lib3/pPUR using the 33 nucleotides represented by SEQ ID NO:11 as the target sequence, an siRNA expression plasmid FUT8shRNA/lib4/pPUR using the 34 nucleotides contained in SEQ ID NO:12 as the target sequence, an siRNA expression plasmid FUT8shRNA/lib6/pPUR using the 28 nucleotides contained in SEQ ID NO:14 as the target sequence, an siRNA expression plasmid FUT8shRNA/lib8/pPUR using the 26 nucleotides contained in SEQ ID NO:16 as the target sequence, and an siRNA expression plasmid FUT8shRNA/lib9/pPUR using the 34 nucleotides represented by SEQ ID NO:17 as the target sequence were prepared in accordance with the method described in the item 3(1) of Example 1. Each of the thus prepared plasmids was introduced into the clone 32-05-12 described in Reference Example in accordance with the method described in the item 1 of Example 2 to prepare LCA-resistant clones.

### (2) Expansion culture of lectin (LCA)-resistant clones

The LCA-resistant clones obtained in the item (1) was expansion cultured by the following procedure.

The formed lectin-resistant colonies were scraped out and sucked in using Pipetteman (manufactured by GILSON) under stereoscopic microscope observation and collected into a U bottom 96-well plate for adhesion cell (manufactured by Asahi Techno Glass). After trypsin treatment, each clone was inoculated into a flat bottom 96-well plate for adhesion cell (manufactured by Greiner) and cultured for 1 week under conditions of 5% CO₂ and 37°C using the basal medium containing puromycin (manufactured by SIGMA) at a concentration of 12 µg/ml. After the culturing, expansion culturing was carried out on 5 clones per each siRNA expression plasmid using the basal medium containing puromycin (manufactured by SIGMA) at a concentration of 12 µg/ml.

Regarding the clones subjected to expansion culturing, the lectin-resistant clones into which FUT8shRNA/lib2/pPUR was introduced were named 12-lib2-1, 12-lib2-2, 12-lib2-3, 12-lib2-4 and 12-lib2-5, the lectin-resistant clones into which FUTBshRNA/lib2B/pPUR was introduced were named 12-lib2B-1, 12-lib2B-2, 12-lib2B-3, 12-lib2B-4 and 12-lib2B-5, the lectin-resistant clones into which FUT8shRNA/lib3/pPUR was introduced were named 12-lib3-1, 12-lib3-2, 12-lib3-3, 12-lib3-4 and 12-lib3-5, the lectin-resistant clones into which FUT8shRNA/lib4/pPUR was introduced were named 12-lib4-1, 12-lib4-2, 12-lib4-3, 12-lib4-4 and 12-lib4-5, the lectin-resistant clones into which FUT8shRNA/lib6/pPUR was introduced were named 12-lib6-1, 12-lib6-2, 12-lib6-3, 12-lib6-4 and 12-lib6-5, the lectin-resistant clones into which FUT8shRNA/lib8/pPUR was introduced were named 12-lib8-1, 12-lib8-2, 12-lib8-3, 12-lib8-4 and 12-lib8-5, and the lectin-resistant clones into which FUT8shRNA/lib9/pPUR was introduced were named 12-lib9-1, 12-lib9-2, 12-lib9-3, 12-lib9-4 and 12-lib9-5, and they were analyzed in the item 2 of this Example which is described in the following. In this connection, the clone 12-lib2B-4 and clone 12-lib3-5 have been deposited on July 1, 2004 as FERM BP-10052 and FERM BP-10053, respectively, in International Patent Organism Depositary, National Institute of Advanced Industrial Science and Technology (Central 6, 1-1, Higashi 1-chome, Tsukuba-shi, Ibaraki, Japan).

### 2. Determination of the amount of FUT8 mRNA in lectin-resistant clone into which FUT8-targeting siRNA expression plasmid was introduced

### (1) Preparation of total RNA

Total RNAs from the lectin-resistant clones obtained in the item 1 of this Example by introducing the FUT8-targeting siRNA expression plasmid and from the clone 32-05-12 which was the parent clone of the lectin-resistant clones were prepared, and single-stranded cDNAs were synthesized in accordance with the method described in the item 2 of Example 2. In this connection, the culturing was carried out using a 6 cm dish for adhesion cell (manufactured by Falcon), and the prepared total RNA was dissolved in 40 µL of sterile water.

### (2) Determination of transcription level of FUT8 gene by SYBR-PCR

The transcription level of mRNA derived from the FUT8 gene and the transcription level of mRNA derived from the β-actin gene were determined by the following procedure. In this connection, the FUT8 standard plasmid described in Example 9 of WO 02/31140 was diluted to a concentration of 0.0512 fg/µl, 0.256 fg/µl, 1.28 fg/µl, 6.4 fg/µl, 32 fg/µl or 160 fg/µl and used as the internal control of the FUT8 determination, and the β-actin standard plasmid described in Example 9 of WO 02/31140 was diluted to a concentration of 1.28 fg/µl, 6.4 fg/µl, 32 fg/µl, 160 fg/µl, 800 fg/µl or 4000 fg/µl and used as the internal control of the β-actin determination. Also, as the PCR primers, the forward primer represented by SEQ ID NO:36 and the reverse primer represented by SEQ ID NO:37 were used for the amplification of FUT8, and the forward primer represented by SEQ ID NO:38 and the reverse primer represented by SEQ ID NO:39 were used for the amplification of β-actin.

Using For Real Time PCR TaKaRa Ex Taq R-PCR Version (manufactured by Takara Bio), 20 µL of a reaction solution [R-PCR buffer (manufactured by Takara Bio), 2.5 mM Mg²⁺ Solution for R-PCR (manufactured by Takara Bio), 0.3 mM dNTP mixture (manufactured by Takara Bio), 0.3 µM forward primer, 0.3 µM reverse primer, 2×10⁻⁵-fold diluted SYBR Green I (manufactured by Takara Bio) and 1 unit of TaKaRa Ex Taq R-PCR] containing 5 µl for each of the single-stranded cDNA solution synthesized in the item (1) and diluted 50-fold with sterile water or the internal control plasmid solutions of the respective concentrations. The thus prepared reaction solution was dispensed into each well of 96-well Polypropylene PCR reaction Plate (manufactured by Falcon), and the plate was sealed using Plate Sealer (manufactured by Edge Biosystems). ABI PRISM 7700 Sequence Detection System was used for the PCR and analysis, and the amount of FUT8 mRNA and the amount of β-actin mRNA were determined in accordance with the manufacture's instructions.

Calibration curves were obtained based upon the measurements with internal control plasmids, and the amount of FUT8 mRNA and the amount of β-actin mRNA was converted into numerical terms. In addition, based on the assumption that the mRNA transcription level of β-actin is uniform among clones, the relative amount of FUT8 mRNA to the amount of β-actin mRNA was calculated and compared, and the results are shown in Fig. 2. In all of the clones obtained by introducing of the FUT8-targeting siRNA expression plasmid, the amount of FUT8mRNA was decreased to about 5% at the maximum in comparison with the parent cell line.

Among the clones obtained by introducing of the siRNA expression plasmid, the clone 12-lib2-3, clone 12-lib2B-4, clone 12-lib3-5, clone 12-lib4-1, clone 12-lib6-3, clone 12-lib8-4 and clone 12-lib9-1 were analyzed in the following item 3.

### 3. Production of antibody composition by lectin-resistant clone into which FUT8-targeting siRNA expression vector was introduced, and composition analysis of monosaccharide of the antibody composition

### (1) Production of antibody composition

Antibody compositions were produced by the following procedure using each of the clone 12-lib2-3, clone 12-lib2B-4, clone 12-lib3-5, clone 12-lib4-1, clone 12-lib6-3, clone 12-lib8-4 and clone 12-lib9-1 obtained in the item 1 of this Example as lectin-resistant clones into which the FUT8-targeting siRNA expression vector was introduced and the parent clone 32-05-12 of the lectin-resistant clones.

The clone 32-05-12 was suspended in the basal medium and the lectin-resistant clones into which the siRNA expression vector was introduced were suspended in the basal medium containing puromycin (manufactured by SIGMA) each at a concentration of 12 µg/ml to give a density of 3×10⁵ cells/ml, and inoculated at 25 ml into T182 flasks for adhesion cell (manufactured by Greiner). After culturing them for 5 days under conditions of 5% CO₂ and 37°C, the culture supernatant was discarded, the cells were washed twice with 20 ml of Dulbecco's PBS (manufactured by Invitrogen), and then 50 ml of EXCELL 301 medium (manufactured by JRH Bioscience) was injected. After culturing them for 7 days under conditions of 5% CO₂ and 37°C, the culture supernatant was recovered, and each antibody composition was purified using a MabSelect (manufactured by Amersham Bioscience) column in accordance with the manufacture's instructions.

### (2) Composition analysis of monosaccharide of antibody compositions

Composition analysis of monosaccharide of the antibodies obtained in the item (1) was carried out in accordance with a conventionally known method [Journal of Liquid Chromatography, 6, 1577 (1983)].

Ratios of complex type sugar chain having no fucose among the total complex type sugar chains, calculated from the composition ratio of monosaccharide of each antibody, is shown in Table 2.

**Table 2**

| Clone name | Ratio of sugar chains having no fucose |
|---|---|
| 32-05-12 | 9% |
| 12-lib2B-4 | 79% |
| 12-lib2-3 | 75% |
| 12-lib3-5 | 72% |
| 12-lib4-1 | 58% |
| 12-lib6-3 | 52% |
| 12-lib8-4 | 72% |
| 12-lib9-1 | 30% |

While the ratio of fucose-free sugar chains in the antibody produced by the parent clone 32-05-12 was 9%, the ratios of fucose-free sugar chains in the antibodies produced by the lectin-resistant clones into which the FUT8-targeting siRNA was introduced were increased to 30 to 79%, so that it was shown that the effect to inhibit addition of α1,6-fucose to the complex type sugar chains of antibodies produced by host cells can be obtained by introducing the FUT8-targeted siRNA expression vector FUTBshRNA/lib2/pPUR, FUT8shRNA/lib2B/pPUR, FUT8shRNA/lib3/pPUR, FUT8shRNA/lib4/pPUR, FUT8shRNA/lib6/pPUR, FUT8shRNA/lib8pPUR or FUT8shRNA/lib9/pPUR. In this connection, the same effect was obtained when the same test was carried out using other expression plasmids of siRNA molecules obtained in the item 3(1) of Example 1.

### Example 4

### Comparison of RNAi activity in different siRNA expression systems of FUT8-targeting effective siRNA

### 1. Construction of FUT8-targeting short hairpin type siRNA expression vector using human U6 promoter

For siRNA containing a nucleotide sequence contained in SEQ ID NO:10 as the target sequence and siRNA containing the nucleotide sequence represented by SEQ ID NO:18 as the target sequence, short hairpin type siRNA expression vectors using human U6 promoter were constructed by the following procedure.

### (1) Cloning of human U6 promoter-cloning site-terminator sequence expression cassette

A human U6 promoter-cloning site-terminator sequence expression cassette was obtained by the following procedure (Fig. 3).

First, each of a forward primer in which recognition sequences of restriction enzymes *Hin*dIII and *Eco*RV were added to the 5'-terminal of a nucleotide sequence which binds to a human U6 promoter sequence (GenBank, M14486) (hereinafter referred to as "hU6p-F-Hind3/EcoRV", represented by SEQ ID NO:39) and a reverse primer in which recognition sequences of restriction enzymes *Xba*I and *Eco*RV, continued 6 adenine nucleotides corresponding to a terminator sequence, and further recognition sequences of restriction enzymes *Kpn*I and *Sac*I for later use in the synthetic oligo DNA insertion, are added to the 5'-terminal of a nucleotide sequence which binds to the human U6 promoter sequence (hereinafter referred to as "hU6p-R-term-*Xba*I/EcoRV", represented by SEQ ID NO:40 was designed.

Next, using KOD polymerase (manufactured by TOYOBO), 50 µL of a reaction solution [KOD Buffer #1 (manufactured by TOYOBO), 0.1 mM dNTPs, 1 mM MgCl₂, 0.4 µM of hU6p-F-Hind3/EcoRV primer and 0.4 µM of hU6p-R-term-XbaI/EcoRV primer] containing 40 ng of the U6-FUT8-B-puro described in Example 12 of WO 03/85118 as the template was prepared to carry out PCR. The PCR was carried out by heating at 94°C for 2 minutes and then 30 cycles of the reaction, one cycle consisting of reaction at 94°C for 15 seconds, reaction at 65°C for 5 seconds and reaction at 74°C for 30 seconds.

After the PCR, the reaction solution was subjected to agarose gel electrophoresis to recover an amplified fragment of about 300 bp was recovered. The DNA fragment was digested at 37°C for 2 hours using a restriction enzyme *Xba*I (manufactured by New England Biolabs) and a restriction enzyme *Hin*dIII (manufactured by New England Biolabs). After the reaction, the reaction solution was subjected to phenol/chloroform extraction and ethanol precipitation.

On the other hand, dephosphorylation reaction of plasmid pBluescript II KS(+) (manufactured by STRATAGENE) was carried out at 37°C for 1 hour using restriction enzymes *Hind*III and *Xba*I (manufactured by New England Biolabs) and Alkaline Phosphatase *E. coli* C75 (manufactured by Takara Bio). After the reaction, the reaction solution was subjected to agarose gel electrophoresis to recover a plasmid pBluescript II KS(+)-derived *Hind*III*-Xba*I fragment of about 2.9 kb.

The PCR amplified fragment of about 300 bp obtained in the above was ligated with the plasmid pBluescript II KS(+)-derived *Hind*III*-Xba*I fragment of about 2.9 kb using Ligation High (manufactured by TOYOBO), *Escherichia coli* DH5α (manufactured by TOYOBO) was transformed by using the reaction solution, and each plasmids was isolated from the thus obtained ampicillin-resistant clones using QIAprep spin Mini prep Kit (manufactured by Qiagen). The nucleotide sequence of each of the thus isolated plasmids was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions to thereby confirm that the desired plasmid, pBS-U6term, was obtained.

### (2) Ligation of human U6 promoter-cloning site-terminator sequence expression cassette with pPUR

The human U6 promoter-cloning site-terminator sequence expression cassette contained in the plasmid pBS-U6term obtained in the item (1) was ligated with the expression vector pPUR by the following procedure (Fig. 4).

Firstly, the plasmid pBS-U6term prepared in the item (1) was digested at 37°C for 2 hours using a restriction enzyme *Eco*RV (manufactured by New England Biolabs). After the digestion, the reaction solution was subjected to agarose gel electrophoresis to recover a DNA fragment of about 350 bp containing the human U6 promoter-cloning site-terminator sequence expression cassette.

On the other hand, plasmid pPUR (manufactured by CLONTECH) was digested at 37°C overnight using a restriction enzyme *Pvu*II (manufactured by New England Biolabs). After the digestion, dephosphorylation reaction was carried out at 37°C for 1 hour using Alkaline Phosphatase *E. coli* C75 (manufactured by Takara Bio). After the reaction, the reaction solution was subjected to agarose gel electrophoresis to recover a *Pvu*II fragment of about 4.3 kb.

The DNA fragment of about 350 bp obtained in the above containing the human U6 promoter-cloning site-terminator sequence expression cassette was ligated with the *Pvu*II fragment of about 4.3 kb derived from the plasmid pPUR using Ligation High (manufactured by TOYOBO), and *Escherichia coli* DH5α (manufactured by TOYOBO) was transformed by using the reaction solution. Each of the plasmid DNAs was isolated from the thus obtained ampicillin-resistant clones using QIAprep spin Mini prep Kit (manufactured by Qiagen). Each of the plasmid DNAs was digested at 37°C for 2 hours using restriction enzymes *Sac*I and *Hind*III (manufactured by New England Biolabs). The reaction solution was subjected to agarose gel electrophoresis to confirm the presence of the desired fragment and its direction.

Further, the nucleotide sequence of each of the thus isolated plasmids was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions to thereby confirm that the sequence of U6 promoter region in the inserted DNA matched with the sequence of GenBank Acc. No. M14486 and there were no errors in the sequences of the primer regions used in the amplification of the human U6 promoter-cloning site-terminator sequence expression cassette and in the sequences of respective ligation regions. Among the thus obtained plasmids, a plasmid in which direction of the inserted hU6 promoter is the same direction of the puromycin-resistant gene expression unit was selected, and the plasmid is named pPUR-U6term hereinafter.

### (3) Insertion of synthetic oligo DNA into plasmid pPUR-U6term

A synthetic oligo DNA capable of forming a double-stranded DNA cassette which expresses an siRNA containing a sequence contained in SEQ ID NO:10 as the target sequence and an siRNA containing the sequence represented by SEQ ID NO:18 as the target sequence, among the target sequences of RNAi for FUT8 obtained in the item 3(1) of Example 1, was designed by the following procedure and was inserted into the cloning site of pPUR-U6term obtained in the above item (2) (Fig. 5).

The synthetic oligo DNA capable of forming a double-stranded DNA cassette was designed by the following procedure. Sequentially from 5'-terminal, the double-stranded DNA cassettes have 3'-cohesive end generated by digestion with a restriction enzyme *Sac*I, sense DNA, loop sequence of human miR-23-precursor-19 micro RNA consisting 10 bases (GenBank Acc. No. AF480558), an antisense DNA, and 3'-cohesive end generated by a restriction enzyme *Kpn*I. In addition, the 5'-terminal of the synthetic oligo DNA capable of forming a double-stranded DNA cassette was phosphorylated. The nucleotide sequence of sense strand of the synthetic oligo DNA designed for the target sequence contained in SEQ ID NO:10 (hereinafter referred to as "Ft-8-dsRNA-B-F") was represented by SEQ ID NO:42, and the nucleotide sequence of the antisense strand thereof (hereinafter referred to as "Ft-8-dsRNA-B-R") was represented by SEQ ID NO:43. The nucleotide sequence of the sense strand of the synthetic oligo DNA designed for the target sequence containing SEQ ID NO:18 (hereinafter referred to as "Ft-8-dsRNA-R-F") was represented by SEQ ID NO:44, and the nucleotide sequence of the antisense strand thereof (hereinafter referred to as "Ft-8-dsRNA-R-R") was represented by SEQ ID NO:45. The synthetic oligo DNA of which the 5'-terminal was phosphorylated was used in the following.

The synthetic oligo DNA was annealed by the following procedure. In 10 µL of an annealing buffer [10 mmol/L Tris (pH 7.5)-50 mmol/L NaCl-1 mmol/L EDTA], 200 pmol each of sense and antisense strands of the synthetic oligo DNAs were dissolved, followed by boiling for 2 minutes. Thereafter, the mixture was gradually cooled to room temperature over about 3 hours and then diluted 15-fold with sterile water.

On the other hand, a plasmid pPUR-U6term-derived *Kpn*I*-Sac*I fragment of about 4.5 kb was recovered from the plasmid pPUR-U6term in the same manner as the method described in the item 3(1) of Example 1.

The double-stranded synthetic oligo solution obtained in the above was ligated with the plasmid pPUR-U6term-derived *Kpn*I*-Sac*I fragment using Ligation High (manufactured by TOYOBO), and *Escherichia coli* DH5α (manufactured by TOYOBO) was transformed by using the reaction solution. Plasmid DNAs were isolated from the thus obtained ampicillin-resistant clones using QIAprep spin Mini prep Kit (manufactured by Qiagen).

The nucleotide sequence of each of the thus isolated plasmids was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions to thereby confirm that there were no errors in the sequences of the inserted synthetic oligo DNAs and ligation regions. Hereinafter, the plasmid into which a double-stranded DNA of the synthetic oligo DNA molecules Ft-8-dsRNA-B-F and Ft-8-dsRNA-B-R was inserted is named FUT8shB/pPUR, and the plasmid into which a double-stranded DNA of the synthetic oligo DNA molecules Ft-8-dsRNA-R-F and Ft-8-dsRNA-R-R was inserted is named FUT8shR/pPUR.

### 2. Construction of FUT8-targeting short hairpin type siRNA expression vector using human tRNA-val promoter

For siRNA containing a nucleotide sequence contained in SEQ ID NO:10 as the target sequence and an siRNA containing the nucleotide sequence represented by SEQ ID NO:18 as the target sequence, short hairpin type siRNA expression vectors using human tRNA promoter were constructed by the following procedure.

### (1) Cloning of human tRNA-val promoter-cloning site-terminator sequence expression cassette

A human tRNA-val promoter-cloning site-terminator sequence expression cassette was obtained by the following procedure (Fig. 6).

First, a plasmid DNA to be used as the template for obtaining the human tRNA-val promoter sequence was prepared by the following procedure from the siRNA expression vector library FUT8shRNAlib/pPUR/DH10B described in Example 1.

An *Escherichia coli* glycerol stock of the siRNA expression vector library FUT8shRNAlib/pPUR/DH10B was diluted to an appropriate density and inoculated onto the LB agar medium containing 100 µg/ml of ampicillin. After culturing them at 37°C overnight, a plasmid DNA was isolated from the thus obtained ampicillin-resistant clone using QIAprep spin Mini prep Kit (manufactured by Qiagen). The isolated plasmid DNA was digested at 37°C overnight using a restriction enzyme *Bam*HI (manufactured by New England Biolabs). After the digestion, the reaction solution was subjected to phenol/chloroform extraction and ethanol precipitation. The nucleotide sequence of each of the thus isolated plasmids was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions. Hereinafter, this plasmid is named pPUR-tRNAp.

Next, using the plasmid pPUR-tRNAp as the template, PCR was carried out using, as the primers, a synthetic oligo DNA in which recognition sequence of a restriction enzyme *Pvu*II is added to the 5'-terminal of a forward primer which binds to the human tRNA-val promoter sequence (hereinafter referred to as "tRNA-*Pvu*II-F", represented by SEQ ID NO:46) and a synthetic oligo DNA in which a recognition sequence of the restriction enzyme *Pvu*II, continued 6 adenine nucleotides corresponding to a terminator sequence, and further recognition sequences of restriction enzymes *Kpn*I and *Sac*I for use in the synthetic DNA insertion are added to the 5'-terminal of a reverse primer which binds to pPUR-tRNAp (hereinafter referred to as "tRNA-*Pvu*II-R", represented by SEQ ID NO:47). Using KOD polymerase (manufactured by TOYOBO), the PCR was carried out by preparing 50 µL of a reaction solution [KOD Buffer #1 (manufactured by TOYOBO), 0.1 mM dNTPs, 1 mM MgCl₂, 0.4 µM of the primer tRNA-*Pvu*II-F and 0.4 µM of the primer tRNA-*Pvu*II-R] containing 50 ng of pPUR-tRNAp as the template, heating the reaction solution at 94°C for 2 minutes and then 30 cycles of the reaction, one cycle consisting of reaction at 94°C for 15 seconds, reaction at 65°C for 5 seconds and reaction at 74°C for 30 seconds. After the reaction, the reaction solution was subjected to agarose gel electrophoresis to recover an amplified DNA fragment of about 200 bp. The recovered solution was subjected to ethanol precipitation, and the thus obtained DNA fragment was digested at 37°C for 3 hours using the restriction enzyme *Pvu*II (manufactured by New England Biolabs). After the digestion, the reaction solution was subjected to phenol/chloroform extraction and ethanol precipitation.

On the other hand, a plasmid pPUR-derived *Pvu*II fragment of about 4.3 kb was recovered from pPUR (manufactured by Clontech) in the same manner as the method described in the item 1(2) of this Example.

The DNA fragment of about 200 bp obtained in the above was ligated with the plasmid pPUR-derived *Pvu*II fragment of about 4.3 kb using Ligation High (manufactured by TOYOBO), *Escherichia coli* DH5α (manufactured by Invitrogen) was transformed by using the reaction solution, and a plasmid DNA was isolated from the thus obtained ampicillin-resistant clone using QIAprep spin Mini prep Kit (manufactured by Qiagen).

The nucleotide sequence of the thus isolated plasmid was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions to thereby confirm that there were no errors in the sequence of the inserted DNA and the ligation regions. Hereinafter, this plasmid is named pPUR-tRNAp-term(-). In this connection, the pPUR-tRNAp-term(-) was inserted into the *Pvu*II site of pPUR in the opposite direction of tRNA-val promoter-cloning site-terminator sequence expression cassette with the puromycin-resistant gene expression unit.

### (2) Insertion of synthetic oligo DNA into plasmid pPUR-tRNAp-term(-)

The synthetic oligo DNA designed in the item 1(3) of this Example was inserted into the pPUR-tRNAp-term(-) obtained in the item (1) by the following procedure (Fig. 7).

First, the plasmid pPUR-tRNAp-term(-) was digested at 37°C overnight using restriction enzymes *Kpn*I and *Sac*I (manufactured by New England Biolabs). After the digestion, the reaction solution was subjected to dephosphorylation reaction at 37°C for 1 hour using Alkaline Phosphatase *E. coli* C75 (manufactured by Takara Bio). After the reaction, the reaction solution was subjected to agarose gel electrophoresis to recover a *Kpn*I*-Sac*I fragment of about 4.5 kb derived from pPUR-tRNAp-term(-).

A double-stranded synthetic oligo DNA solution prepared by annealing the Ft8-dsRNA-B-F and Ft8-dsRNA-B-R obtained in the item 1(3) of this Example or a double-stranded synthetic oligo DNA solution prepared by annealing the Ft8-dsRNA-R-F and Ft8-dsRNA-R-R was ligated with the plasmid pPUR-tRNAp-term(-)-derived *Kpn*I*-Sac*I fragment of about 4.5 kb using Ligation High (manufactured by TOYOBO), and *Escherichia coli* DH5α (manufactured by Invitrogen) was transformed by using the reaction solution. Each plasmid DNAs was isolated from the thus obtained ampicillin-resistant clones using QIAprep spin Mini prep Kit (manufactured by Qiagen).

The nucleotide sequence of each of the thus isolated plasmids was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions to thereby confirm that there were no errors in the sequences of the inserted synthetic oligo DNAs and ligation regions. Hereinafter, the plasmid into which the double-stranded DNA of the synthetic oligo DNA Ft-8-dsRNA-B-F and Ft-8-dsRNA-B-R was inserted is named tRNA-FUT8shB/pPUR(-), and the plasmid into which the double-stranded DNA of the synthetic oligo DNA Ft-8-dsRNA-R-F and Ft-8-dsRNA-R-R was inserted is named tRNA-FUT8shR/pPUR(-).

### (3) Construction of tRNA promoter-short hairpin type siRNA expression vector(+)

From the tRNA-FUT8shB/pPUR(-) and tRNA-FUT8shR/pPUR(-) obtained in the item (2), a short hairpin type siRNA expression vector was constructed by the following procedure using a human tRNA promoter in which a human tRNA-val promoter-short hairpin RNA-terminator sequence expression cassette is inserted into the *Pvu*II site of pPUR in the same direction with the puromycin-resistant gene expression unit (Fig. 8).

The tRNA-FUT8shB/pPUR(-) or tRNA-FUT8shR/pPUR(-) was digested at 37°C overnight using the restriction enzyme *Pvu*II (manufactured by New England Biolabs). After the digestion, the reaction solution was subjected to agarose gel electrophoresis to recover a DNA fragment of about 300 bp.

On the other hand, a plasmid pPUR-derived *Pvu*II fragment of about 4.3 kb was recovered from pPUR (manufactured by Clontech) in the same manner as the method described in the item 1(2) of this Example.

The DNA fragment of about 300 bp obtained in the above was ligated with the plasmid pPUR-derived *Pvu*II fragment of about 4.3 kb using Ligation High (manufactured by TOYOBO), and *Escherichia coli* DH5α (manufactured by Invitrogen) was transformed by using the reaction solution. Plasmid DNAs were isolated from the thus obtained ampicillin-resistant clones using QIAprep spin Mini prep Kit (manufactured by Qiagen), and each plasmid DNA was digested at 37°C for 2 hours using the restriction enzyme *Hind*III (manufactured by New England Biolabs). After the digestion, the reaction solution was subjected to agarose gel electrophoresis to confirm the presence of the desired fragment and its direction, and then clones in which the human tRNA-val promoter-short hairpin RNA-terminator sequence expression cassette of the inserted fragment is the same direction of the puromycin-resistant gene expression unit were selected. The nucleotide sequence of each of the selected plasmids was determined by DNA sequence ABI PRISM 377 (manufactured by Applied Biosystems) after the reaction using BigDye Terminator v3.0 Cycle Sequencing Kit (manufactured by Applied Biosystems) in accordance with the manufacture's instructions to thereby confirm that there were no errors in the insertion sequences of the plasmids and sequences of the respective ligation regions. Hereinafter, a plasmid containing the human tRNA-val promoter-short hairpin RNA-terminator sequence expression cassette of the tRNA-FUT8shB/pPUR(-) is named tRNA-FUT8shB/pPUR(+), and a plasmid containing the human tRNA-val promoter-short hairpin RNA-terminator sequence expression cassette of the tRNA-FUT8shR/pPUR(-) is named tRNA-FUT8shR/pPUR(+).

### 3. Obtaining of lectin-resistant clone into which FUT8-targeting siRNA expression plasmid was introduced and culturing thereof

Each of the FUT8-targeting short hairpin type siRNA expression vectors FUT8shB/pPUR and FUT8shR/pPUR using human U6 promoter constructed in the item 1 of this Example, the FUT8-targeting short hairpin type siRNA expression vectors tRNA-FUT8shB/pPUR(+) and tRNA-FUT8shR/pPUR(+) using human tRNA-val promoter constructed in the item 2 of this Example and the FUT8-targeting tandem type siRNA expression vectors U6_FUT8_B_puro and U6_FUT8_R_puro using human U6 promoter described in Example 12 of WO 03/85118 constructed was introduced into the clone 32-05-12 in accordance with the method described in the item 1 of Example 2 to thereby obtain LCA-resistant clones. As a result, lectin-resistant clones were obtained by the use of any one of the siRNA expression systems.

### 4. Expansion culturing of lectin-resistant clone into which FUT8-targeting siRNA expression plasmids was introduced, and analysis of FUT8 mRNA expression

### (1) Preparation of total RNA

Total RNAs from the clone 32-05-12 and the lectin-resistant clones obtained in the item 3 of this Example were prepared and single-stranded cDNAs were synthesized in the same manner as in the item 2 of Example 2. In this connection, the culturing was carried out using a 6 cm-dish for adhesion cell (manufactured by Falcon), and each of the prepared total RNAs was dissolved in 40 µL of sterile water.

### (2) Determination of FUT8 gene transcription level by SYBR-PCR

The transcription level of mRNA derived from the FUT8 gene and the transcription level of mRNA derived from the β-actin gene were determined in the same manner as the method described in the item 2(3) of Example 3. In addition, based on the assumption that the transcription level of the mRNA derived from β-actin gene is uniform among the clones, the relative values of the amount of FUT8mRNA to the amount of β-actin mRNA were calculated and compared, and the results are shown in Fig. 9.

It was shown that the amount of FUT8 mRNA was decreased in all of the lectin-resistant clones obtained using any one of the siRNA expression systems, in comparison with the parent clone. Thus, it was shown that the RNAi activity by FUT8-targeting siRNA capable of converting the parent clone into lectin-resistant clones is observed by using any one of the siRNA expression systems.

### Example 5

### Serum-free fed-batch culture of lectin-resistant CHO/DG44 cell into which FUT8-targeting siRNA expression plasmid was introduced

### 1. Adaptation of lectin-resistant clone into which FUT8-targeting siRNA expression plasmids was introduced to serum-free medium

The clone 32-05-12 and the lectin-resistant clones, clone 12-lib2B-1, clone 12-lib2B-4, clone 12-lib3-4 and clone 12-lib3-5, into which the FUT8-targeting siRNA expression plasmid was introduced obtained in the item 1 of Example 3 were adapted to a serum-free medium by the following procedure.

The clone 32-05-12 was suspended in the basal medium, and each of the lectin-resistant clones into the which the FUT8-targeting siRNA expression plasmid was introduced was suspended in the basal medium containing puromycin (manufactured by SIGMA) at a concentration of 12 µg/mL to give a cell density of 3×10⁵ cells/mL, and inoculated at 15 mL into 75 cm² flasks for adhesion culture (manufactured by Greiner). Each clone was cultured for 3 days under conditions of 5% CO₂ and 35°C, each cell suspension was recovered by trypsin treatment, and the suspension was centrifuged at 1000 rpm for 5 minutes to discard the supernatant. The thus recovered cells of the clone 32-05-12 was suspended in EX-CELL 302 medium (manufactured by JRH) containing MTX (manufactured by SIGMA) at a concentration of 500 nM, L-glutamine (manufactured by Invitrogen) at a concentration of 6 mM and 3,3,5-triiodo-L-thyronine (manufactured by SIGMA) at a concentration of 100 nM (hereinafter referred to as "serum-free medium"), and those of each of the lectin-resistant clones into which FUT8-targeting siRNA expression plasmids were introduced was suspended in the serum-free medium containing puromycin (manufactured by SIGMA) at a concentration of 12 µg/mL at a density of 5×10⁵ cells/mL, and 15 mL of the cell suspension was inoculated into a 125 mL conical flask (manufactured by Coming). After ventilating the flask with 5% CO₂ (at least 4-fold volume of culture vessel) and sealing the flask, suspension rotation culture was carried out at 90-100 rpm and 35°C. Passage was repeated at 3 to 4 day intervals, and finally, clones which could grow in the serum-free medium were obtained. Hereinafter, the clone 32-05-12 adapted to the serum-free medium is named 32-05-12AF, the clone 12-lib2B-1 adapted to the serum-free medium is named 12-lib2B-1AF, the clone 12-lib2B-4 adapted to the serum-free medium is named 12-lib2B-4AF, the clone 12-lib3-4 adapted to the serum-free medium is named 12-lib3-4AF, and the clone 12-lib3-5 adapted to the serum-free medium is named 12-lib3-5AF.

### 2. Serum-free fed-batch culture of lectin-resistant clone into which FUT8-targeting siRNA expression plasmid was introduced and adapted to serum-free medium

Using the clone 32-05-12AF, clone 12-lib2B-1AF, clone 12-lib2B-4AF, clone 12-lib3-4AF and clone 12-lib3-5AF adapted to the serum-free medium in the item 1 of this Example, serum-free fed-batch culturing was carried out by the following procedure.

EX-CELL302 medium (manufactured by JRH) containing 500 nM MTX (manufactured by SIGMA), 6 mM L-glutamine (manufactured by Invitrogen), 100 nM 3,3,5-triiodo-L-thyronine (manufactured by SIGMA), 0.1% Pluronic F-68 (manufactured by Invitrogen), and 5000 mg/L D(+)-glucose (manufactured by Nacalai Tesque) (hereinafter referred to as "serum-free fed-batch medium") was used for fed-batch culture, and a medium containing amino acids prepared at higher concentrations than usual addition (0.177 g/L L-alanine, 0.593 g/L L-arginine monohydrochloride, 0.177 g/L L-asparagine monohydrate, 0.212 g/L L-asparatic acid, 0.646 g/L L-cystine dihydrochloride, 0.530 g/L L-glutamic acid, 5.84 g/L L-glutamine, 0.212 g/L glycine, 0.297 g/L L-histidine monohydrochloride dihydrate, 0.742 g/L L-isoleucine, 0.742 g/L L-leucine, 1.031 g/L L-lysine monohydrochloride, 0.212 g/L L-methionine, 0.466 g/L L-phenylalanine, 0.283 g/L L-proline, 0.297 g/L L-serine, 0.671 g/L L-threonine, 0.113 g/L L-tryptophan, 0.735 g/L L-tyrosine disodium dihydrate, and 0.664 g/L L-valine), vitamins (0.0918 mg/L d-biotin, 0.0283 g/L D-calcium pantothenate, 0.0283 g/L choline chloride, 0.0283 g/L folic acid, 0.0509 g/L myo-inositol, 0.0283 g/L niacinamide, 0.0283 g/L pyridoxal hydrochloride, 0.00283 g/L riboflavin, 0.0283 g/L thiamine hydrochloride, and 0.0918 mg/L cyanocobalamin) and 0.314 g/L insulin (hereinafter referred to as "feed medium") was used as a medium for feeding.

Each of the clone 32-05-12AF, clone 12-lib2B-1AF, clone 12-lib2B-4AF, clone 12-lib3-4AF and clone 12-lib3-5AF was suspended in the serum-free fed-batch culture medium at a cell density of 3×10⁵ cells/ml, and 40 mL each of the cell suspension was inoculated into a 250 ml conical flask (manufactured by Coming). After ventilating the flask with 5% CO₂ (at least 4-fold volume of culture vessel), and sealing the flask, suspension rotation culture was carried out at 90 to 100 rpm and 35°C. On days 3, 6, 9 and 12 after starting the culture, 3.3 mL of feed medium was added to supplement the consumption of amino acids and the like, and 20% (w/v) glucose solution was added at a final concentration of 5,000 mg/L to adjust the glucose concentration. On days 0, 3, 6, 9, 12 and 14 after starting the culture, 2-4 mL each of the culture was collected, and viable cell number and viability were measured by trypan blue staining and the concentration of antibody contained in each culture supernatant by the method for determining the concentration of antibody using ELISA described in the item 3(1) of this Example was measured. Results of the viable cell number, the viability and the concentration of antibody in culture supernatant at each point of time after starting the starting of culturing are shown in Fig. 10 to Fig. 12.

### 3. Determination of antibodies having sugar chains in which 1-position of fucose is not bound to 6-position of N-acetylglucosamine in the reducing end through α-bond using the binding activity to soluble human FcγRIIIa as an indicator

The ratio of sugar chains in which 1-position of fucose is not bound to 6-position of N-acetylglucosamine in the reducing end thorough α-bond in the anti-CCR4 chimeric antibody contained in the serum-free fed-batch culture samples of the clone 32-05-12AF, clone 12-lib2B-1AF, clone 12-lib2B-4AF, clone 12-lib3-4AF and clone 12-lib3-5AF, collected in the item 2 of this Example, was measured using the binding activity to soluble human FcγRIIIa (hereinafter referred to as "shFcγRIIIa") described in Reference Example 2 as an indicator according to the following procedure.

### (1) Determination of antibody concentration by ELISA

The antibody concentration in culture supernatant was determined by the following procedure.

In 750 mL of Dulbecco's PBS (manufactured by Invitrogen), 1 mL of anti-human IgG (H+L) antibody (manufactured by American Qualex) was dissolved, and the mixture was dispensed at 50 µl onto each well of an ELISA plate. After leaving overnight at 4°C, the solution was removed, and 100 µL of PBS containing 1% BSA (bovine serum albumin) (hereinafter referred to as "BSA-PBS") was added to each well, and the plate was left for approximately 1 hour at room temperature, and stored at -20°C. On measuring the amount of antibody, the plate was thawed at room temperature, and after removing the BSA-PBS in wells, 50 µL of the culture supernatant diluted with BSA-PBS was added to each well. After the plate was left for 1 to 2 hours at room temperature, the wells were washed with PBS containing 0.05% Tween20™ (hereinafter referred to as "Tween-PBS"). After removing the washing liquid, 50 µL of goat anti-human IgG (H&L)-HRP (manufactured by American Qualex) diluted 2000-fold with BSA-PBS, was added to each well as a second antibody. After the plate was left for 1 to 2 hours at room temperature, wells were washed with 0.05% Tween-PBS and then with resin water. After removing water from the walls, 50 µL of an ABTS substrate solution supplemented with 0.1% H₂O₂ was added to each well for color development. After the plate was left for approximately 15 minutes, when appropriate color developed, 50 µL, of 5% SDS solution was added to each well to stop the reaction. Absorption at 490 nm was measured with that of at 415 nm as reference using a microplate reader. Antibody concentrations of each diluted sample were calculated using the linear area of the sigmoid curve of the calibration curve prepared with a standard of purified antibody preparation. Each antibody concentration of culture supernatants was calculated by multiplying the antibody concentrations of the obtained diluted samples by the dilution rate.

### (2) Preparation of antibodies having different ratio of sugar chains in which fucose is not bound to N-acetylglucosamine in the reducing end group in the complex type N-glycoside-linked sugar chain

Standard samples anti-CCR4 chimeric antibody compositions with different ratio of antibody having sugar chains in which fucose is not bound to N-acetylglucosamine in the reducing end group in the complex type N-glycoside-linked sugar chains (hereinafter referred to as "fucose(-)% of antibody composition") were prepared. Fucose(-)% of antibody composition was measured by composition analysis of monosaccharide described in the item 3(2) of Example 3 for a total of 11 standard samples including KM2760-1, KM3060, and 9 standard samples prepared by mixing KM2760-1 and KM3060; KM2760-1 was 90%; KM3060 was 10%; 9 standard samples prepared were 82%, 74%, 66%, 58%, 50%, 42%, 34%, 26% and 18%, respectively.

### (3) Evaluation of the binding activity of antibody to shFcγRIIIa

50 µL/well of a BSA (bovine serum albumin) conjugate of a human CCR4 extracellular region peptide having the amino acid sequence represented by SEQ ID NO:35 with which the anti-CCR4 chimeric antibody prepared in the item 2 of Reference Example 1 can react was dispensed onto 96-well ELISA plates (manufactured by Greiner) at a concentration of 1 µg/mL, the mixture was left overnight at 4°C to adsorb. After washing with PBS, 100 µL/well of 1% BSA-PBS was added, and was allowed to react for 1 hour at room temperature to block remaining active groups. After washing each well with Tween-PBS, 50 µL/well of each of the culture supernatant solutions diluted with 1% BSA-PBS to 5.0 µg/ml based on the antibody concentration measured by the determination method of antibody concentration by ELISA described in the item (1), or a fucose(-)% standard sample of antibody composition diluted with 1% BSA-PBS to a protein concentration of 5.0 µg/ml, was added and allowed to react for 1 hour at room temperature. After washing each well with Tween-PBS, 50 µL/well of shFcγRIIIa solution prepared by the method shown in Reference Example 2 and diluted at 5 µg/mL with 1% BSA-PBS was added thereto, and was allowed to react for 1 hour at room temperature. After washing each well with Tween-PBS, 50 µL/well of HRP-labeled mouse antibody Penta-His HRP Conjugate (manufactured by QIAGEN) prepared with 1% BSA-PBS at 0.1 µg/mL was added, and the mixture was allowed to react for 1 hour at room temperature. After washing with Tween-PBS, 50 µL/well of ABTS substrate solution was added, and after color development, OD415 was measured.

The binding activity of the fucose(-)% standard sample of antibody composition prepared in the item (2) to shFcγRIIIa is shown in Fig. 13. A calibration curve of the binding activity of antibody composition to shFcγRIIIa, which is proportional to the fucose(-)% of antibody composition, was obtained.

The fucose(-)% of anti-CCR4 chimeric antibody composition contained in each cultured sample was calculated from the OD415 value showing the shFcγRIIIa binding activity of anti-CCR4 chimeric antibody contained in the serum-free fed-batch culture sample collected in the item 2 of this Example, using the calibration curve shown in Fig. 14. Regarding the sample derived from the clone 32-05-12AF, the fucose(-)% of antibody composition produced in culture was about 10%. On the other hand, in the case of the samples derived from the clone 12-lib2B-1AF, clone 12-lib2B-4AF, clone 12-lib3-4AF and clone 12-lib3-5AF which are lectin-resistant clones into which the FUT8-targeting siRNA expression plasmid was introduced, the fucose(-)% of antibody composition produced in culture was from 40 to 70%, thus showing that an antibody composition having high antibody composition fucose(-)% can be produced by introducing the FUT8-targeting siRNA expression plasmid.

### Reference Example 1

### Preparation of anti-CCR4 chimeric antibodies having a different ratio in which fucose is not bound to N-acetylglucosamine in the reducing end in the N-glycoside-linked sugar chains:

### 1. Preparation of antibody-producing cell using CHO/DG44 cell

Cells stably producing an anti-CCR4 chimeric antibody were prepared by introducing the anti-CCR4 chimeric antibody expression vector pKANTEX2160 described in WO 01/64754 to CHO/DG44 cell in the following manner.

After introducing 4 µg of the anti-CCR4 chimeric antibody expression vector pKANTEX2160 into 1.6×10⁶ cells of CHO/DG44 cell by electroporation [Cytotechnology, 3, 133 (1990)], the cells were suspended in 10 mL of IMDM-dFBS(10)-HT(1) [IMDM medium (manufactured by Invitrogen) comprising 10% dFBS (manufactured by Invitrogen) and 1 × concentration of HT supplement (manufactured by Invitrogen)] and dispensed in 100 µL/well into 96 well culture plates (manufactured by Iwaki Glass). After culturing at 37°C for 24 hours in a 5% CO₂ incubator, the medium was changed to IMDM-dFBS(10) (IMDM medium comprising 10% of dialyzed FBS), followed by culturing for 1 to 2 weeks. Culture supernatant was recovered from wells in which the growth was observed due to formation of a transformant showing HT-independent growth, and an amount of production of the anti-CCR4 chimeric antibody in the supernatant was measured by the ELISA described in the item 2 of this Reference Example.

Regarding the transformants in wells in which production of the anti-CCR4 chimeric antibody was observed in culture supernatants, in order to increase an amount of the antibody production using a DHFR gene amplification system, each of them was suspended in the IMDM-dFBS(10) medium comprising 50 nM MTX to give a density of 1 to 2×10⁵ cells/mL, and the suspension was dispensed in 0.5 mL into wells of 24 well plates (manufactured by Iwaki Glass). After culturing at 37°C for 1 to 2 weeks in a 5% CO₂ incubator, transformants showing 50 nM MTX resistance were induced. Regarding the transformants in wells in which the growth was observed, the MTX concentration was increased to 200 nM by the same method, and a transformant capable of growing in the IMDM-dFBS(10) medium comprising 200 nM MTX and of producing the anti-CCR4 chimeric antibody, clone 32-05-12, was obtained.

### 2. Antibody binding activity to CCR4 partial peptide (ELISA)

Compound 1 having the amino acid sequence represented by SEQ ID NO:35 was selected as a human CCR4 extracellular region peptide capable of reacting with the anti-CCR4 chimeric antibody. In order to use Compound 1 as the antigen in ELISA, a conjugate with BSA (bovine serum albumin) (manufactured by Nacalai Tesque) was prepared by the following procedure.

100 µL of a DMSO solution comprising 25 mg/mL SMCC [4-(*N-*maleimidomethyl)cyclohexane-1-carboxylic acid *N*-hydroxysuccinimide ester] (manufactured by Sigma) was added dropwise to 900 µL of a 10 mg BSA-containing PBS solution under stirring, followed by gently stirring for 30 minutes. To NAP-10 column equilibrated with 25 mL of PBS, 1 ml of the reaction solution was applied and then eluted with 1.5 mL of PBS and the resulting eluate was used as a BSA-SMCC solution (BSA concentration was calculated based on A₂₈₀ measurement). Next, 250 µL of PBS was added to 0.5 mg of Compound 1 and then completely dissolved by adding 250 µL of DMF, and the BSA-SMCC solution was added thereto under stirring, followed by gently stirring for 3 hours. The reaction solution was dialyzed against PBS at 4°C overnight, sodium azide was added thereto to give a final concentration of 0.05%, and the mixture was filtered through a 0.22 mm filter to be used as a BSA-compound 1 solution. Hereinafter, the solution is referred to as a BSA-compound 1 solution.

The above BSA-Compound 1 solution was dispensed at 0.05 µg/ml and 50 µl/well into a 96-well EIA plate (manufactured by Greiner) and left at 4°C overnight for adsorption. After washing each well with PBS, 1% BSA-PBS was added thereto in 100 µl/well and allowed to react at room temperature to block the remaining active groups. After washing each well with PBS containing 0.05% Tween 20 (hereinafter referred to as "Tween-PBS"), a culture supernatant of a transformant was added at 50 µl/well and allowed to react at room temperature for 1 hour. After the reaction, each well was washed with Tween-PBS, and then a peroxidase-labeled goat anti-human IgG(γ) antibody solution (manufactured by American Qualex) diluted 6000 times with 1% BSA-PBS as the secondary antibody was added at 50 µl/well and allowed to react at room temperature for 1 hour. After the reaction and subsequent washing with Tween-PBS, the ABTS substrate solution [solution prepared by dissolving 0.55 g of 2,2'-azino-bis(3-ethylbenzothiazoline-6-sulfonic acid) ammonium salt in 1 liter of 0.1 M citrate buffer (pH 4.2) and adding 1 µl/ml of hydrogen peroxide to the solution just before use] was added at 50 µl/well for color development. Thereafter, the absorbance at 415 nm (hereinafter referred to as OD₄₁₅) was measured by a plate reader Benchmark (manufactured by BIO RAD). The anti-CCR4 chimeric antibody obtained in the item 1 of this Reference Example showed the binding activity to CCR4.

### 3. Preparation of antibody-producing cell using rat myeloma YB2/0 cell

After introducing 10 µg of the anti-CCR4 chimeric antibody expression vector pKANTEX2160 into 4×10⁶ cells of rat myeloma YB2/0 cell (ATCC CRL 1662) by electroporation [Cytotechnology, 3, 133 (1990)], the cells were suspended in 40 ml of Hybridoma-SFM-FBS(5) [Hybridoma-SFM medium (manufactured by Invitrogen) comprising 5% FBS (manufactured by PAA Laboratories)] and dispensed in 200 µl/well into 96 well culture plates (manufactured by Sumitomo Bakelite). After culturing at 37°C for 24 hours in a 5% CO₂ incubator, G418 was added to give a concentration of 1 mg/ml, followed by culturing for 1 to 2 weeks. Culture supernatant was recovered from wells in which growth of transformants showing G418 resistance was observed by the formation of colonies, and the antigen binding activity of the anti-CCR4 chimeric antibody in the supernatant was measured by the ELISA described in the above item 2 to confirm that it had binding activity to CCR4.

Regarding the transformants in wells in which production of the anti-CCR4 chimeric antibody was observed in culture supernatants, in order to increase an amount of the antibody production using a dhfr gene amplification system, each of them was suspended in the Hybridoma-SFM-FBS(5) medium comprising 1 mg/ml G418 and 50 nmol/l DHFR inhibitor MTX (manufactured by SIGMA) to give a density of 1 to 2×10⁵ cells/ml, and the suspension was dispensed at 1 ml into wells of a 24-well plate (manufactured by Greiner). After culturing them at 37°C for 1 to 2 weeks in a 5% CO₂ incubator, transformants showing 50 nmol/l MTX resistance were induced. Antigen binding activity of the anti-CCR4 chimeric antibody in culture supernatants in wells in which growth of transformants was observed was measured by the ELISA described in the above item 2.

Regarding the transformants in wells in which production of the anti-CCR4 chimeric antibody was observed in culture supernatants, the MTX concentration was increased by the same method, and a transformant capable of growing in the Hybridoma-SFM-FBS(5) medium comprising 200 nmol/l MTX and of producing the anti-CCR4 chimeric antibody in a large amount was finally obtained. The obtained transformant was cloned by limiting dilution twice, and the obtained transformant clone was named KM2760 #58-35-16.

### 4. Purification of anti-CCR4 chimeric antibody

### (1) Culturing of antibody-producing cell derived from CHO-DG44 cell and purification of antibody

The anti-CCR4 chimeric antibody-producing transformant clone 5-03 obtained in the above item 1 was cultured at 37°C in a 5% CO₂ incubator using IMDM-dFBS(10) medium in a 182 cm² flask (manufactured by Greiner). When the cell density reached confluent after several days, the culture supernatant was discarded, and the cells were washed with 25 ml of PBS buffer and then mixed with 35 ml of EXCELL 301 medium (manufactured by JRH). After culturing at 37°C for 7 days in a 5% CO₂ incubator, the culture supernatant was recovered. The anti-CCR4 chimeric antibody was purified from the culture supernatant by using Prosep-A (manufactured by Millipore) column in accordance with the manufacture's instructions. The purified anti-CCR4 chimeric antibody was named KM3060.

### (2) Culturing of antibody-producing cell derived from YB2/0 cell and purification of antibody

The anti-CCR4 chimeric antibody-expressing transformant cell clone KM2760#58-35-16 obtained in the above item 3 was suspended in Hybridoma-SFM (manufactured by Invitrogen) medium comprising 200 nM MTX and 5% of Daigo's GF21 (manufactured by Wako Pure Chemical Industries) to give a density of 2×10⁵ cells/ml and subjected to fed-batch shaking culturing using a spinner bottle (manufactured by Iwaki Glass) in a constant temperature chamber of 37°C. After culturing for 8 to 10 days, the anti-CCR4 chimeric antibody was purified from the culture supernatant recovered using Prosep-A (manufactured by Millipore) column and gel filtration. The purified anti-CCR4 chimeric antibody was named KM2760-1.

When the binding activity to CCR4 of KM2760-1 and KM3060 was measured by the ELISA described in the above item 2, they showed equivalent binding activity.

### Reference Example 2

### Preparation of soluble human FcγRIIIa protein

### 1. Construction of a soluble human FcγRIIIa protein expression vector

### (1) Preparation of human peripheral blood monocyte cDNA

Heparin sodium (manufactured by Shimizu Pharmaceutical) was added to 30 ml of vein blood of a healthy donor and then gently mixed. From the mixture, a monocyte layer was separated using Lymphoprep (manufactured by Daiichi Pure Chemicals) according to the manufacture's instructions. After washing by centrifugation with PRMI1640 medium once and PRMI1640-FCS(10) medium once, the peripheral blood monocyte suspension suspended in RPMI1640-FBS(10) was prepared at a density of 2×10⁶ cells/ml. After 5 ml of the resulting peripheral blood monocyte suspension was centrifuged at room temperature and at 800 rpm for 5 minutes in 5 ml of PBS, the supernatant was discarded and the residue was suspended in 5 mL of PBS. After centrifugation at room temperature and at 800 rpm for 5 minutes, the supernatant was discarded and total RNA was extracted by QIAamp RNA Blood Mini Kit (manufactured by QIAGEN) and in accordance with the manufacture's instructions.

A single-stranded cDNA was synthesized by reverse transcription reaction to 2 µg of the resulting total RNA, in a series of 40 µl containing oligo(dT) as primers using SUPERSCRITP™ Preamplification System for First Strand cDNA Synthesis (manufactured by Life Technologies) according to the manufacture's instructions.

### (2) Obtaining of cDNA encoding human FcγRIIIa protein

A cDNA encoding a human FcγRIIIa protein (hereinafter referred to as "hFcγRIIIa") was obtained as follows.

First, a specific forward primer containing a translation initiation codon (represented by SEQ ID NO:48) and a specific reverse primer containing a translation termination codon (represented by SEQ ID NO:49) were designed from the nucleotide sequence of hFcγRIIIa cDNA [J. Exp. Med., 170, 481 (1989)].

Next, using a DNA polymerase ExTaq (manufactured by Takara Shuzo), 50 µL of a reaction solution [1× concentration ExTaq buffer (manufactured by Takara Shuzo), 0.2 mM dNTPs, 1 µM of the above gene-specific primers (SEQ ID NOs:48 and 49)] containing 5 µL of 20-fold diluted solution of the human peripheral blood monocyte-derived cDNA solution prepared in the above item 1 was prepared, and PCR was carried out. The PCR was carried out by 35 cycles of a reaction at 94°C for 30 seconds, at 56°C for 30 seconds and at 72°C for 60 seconds as one cycle.

After the PCR, the reaction solution was purified by using QIAquick PCR Purification Kit (manufactured by QIAGEN) and dissolved in 20 µL of sterile water. The products were digested with restriction enzymes *Eco*RI (manufactured by Takara Shuzo) and *Bam*HI (manufactured by Takara Shuzo) and subjected to 0.8% agarose gel electrophoresis to recover about 800 bp of a specific amplification fragment.

On the other hand, 2.5 µg of a plasmid pBluescript II SK(-) (manufactured by Stratagene) was digested with restriction enzymes *Eco*RI (manufactured by Takara Shuzo) and *Bam*HI (manufactured by Takara Shuzo), and digested products were subjected to 0.8% agarose gel electrophoresis to recover a fragment of about 2.9 kbp.

The human peripheral blood monocyte cDNA-derived amplification fragment of about 800 bp and the plasmid pBluescript II SK(-)-derived fragment of about 2.9 kbp obtained in the above were ligated by using DNA Ligation Kit Ver. 2.0 (manufactured by Takara Shuzo). *Escherichia coli* DH5α (manufactured by TOYOBO) was transformed by using the reaction solution. Each plasmid DNA was isolated from the resulting ampicillin-resistant colonies and then allowed to react using BigDye Terminator Cycle Sequencing FS Ready Reaction Kit (manufactured by Applied Biosystems) according to the manufacture's instructions, and the nucleotide sequence of cDNA inserted into each plasmid was determined by using DNA sequence ABI PRISM 377 (manufactured by Applied Biosystems). It was confirmed that all of the inserted cDNAs of which sequence was determined by this method encodes the full length of ORF of hRcγRIIIa. As a result, it was confirmed that pBSFcγRIIIa5-3 was obtained as a plasmid containing cDNA encoding hRcγRIIIa having the nucleotide sequence represented by SEQ ID NO:46. The amino acid sequence corresponding to the nucleotide sequence represented by SEQ ID NO:50 is represented by SEQ ID NO:51.

### (3) Obtaining of cDNA encoding soluble hFcγRIIIa

A cDNA encoding soluble hFcγRIIIa (hereinafter referred to as "shFcγRIIIa") having the extracellular region of hFcγRIIIa (positions 1 to 193 in SEQ ID NO:51) and a His-tag sequence at the C-terminal was constructed as follows.

First, a primer FcgR3-1 (represented by SEQ ID NO:52) specific for the extracellular region was designed from the nucleotide sequence of hFcγRIIIa cDNA represented by SEQ ID NO:50.

Next, using a DNA polymerase ExTaq (manufactured by Takara Shuzo), 50 µL of a reaction solution [1× concentration ExTaq buffer (manufactured by Takara Shuzo), 0.2 mM dNTPs, 1 µM of the primer FcgR3-1, 1 µM of the primer M13M4 (manufactured by Takara Shuzo)] containing 5 ng of the plasmid pBSFcγRIIIa5-3 prepared in the above (2) was prepared, and PCR was carried out. The PCR was carried out by 35 cycles of a reaction at 94°C for 30 seconds, at 56°C for 30 seconds and at 72°C for 60 seconds as one cycle. After the PCR, the reaction solution was purified by using QIAquick PCR Purification Kit (manufactured by QIAGEN) and dissolved in 20 µL of sterile water. The products were digested with restriction enzymes *Pst*I (manufactured by Takara Shuzo) and *Bam*HI (manufactured by Takara Shuzo) and subjected to 0.8% agarose gel electrophoresis to recover about 110 bp of a specific amplification fragment.

On the other hand, 2.5 µg of the plasmid pBSFcγRIIIa5-3 was digested with restriction enzymes *Pst*I (manufactured by Takara Shuzo) and *Bam*HI (manufactured by Takara Shuzo), and the digested products were subjected to 0.8% agarose gel electrophoresis to recover a fragment of about 3.5 kbp.

The hFcγRIIIa cDNA-derived amplification fragment and plasmid pBSFcγRIIIa5-3-derived fragment obtained in the above were ligated by using DNA Ligation Kit Ver. 2.0 (manufactured by Takara Shuzo). The strain *Escherichia coli* DH5α (manufactured by TOYOBO) was transformed by using the reaction solution. Each plasmid DNA was isolated from the resulting transformants and then allowed to react using BigDye Terminator Cycle Sequencing FS Ready Reaction Kit (manufactured by Parkin Elmer) according to the manufacture's instructions, and the nucleotide sequence of cDNA inserted into each plasmid was determined by using DNA sequence ABI PRISM 377 (manufactured by Parkin Elmer) to confirm that pBSFcγRIIIa+His3 was obtained.

The thus determined full length cDNA sequence for shFcγRIIIa is represented by SEQ ID NO:53, and its corresponding amino acid sequence is represented by SEQ ID NO:54.

### (4) Construction of shFcγRIIIa expression vector

shFcγRIIIa expression vector was constructed as follows.

After the plasmid pBSFcγRIIIa+His3 obtained in the above item (3) was digested with restriction enzymes *Eco*RI (manufactured by Takara Shuzo) and *Bam*HI (manufactured by Takara Shuzo), the reaction solution was subjected to agarose gel electrophoresis to recover fragments of each about 620 bp.

On the other hand, the plasmid pKANTEX93 was digested with restriction enzymes *Eco*RI (manufactured by Takara Shuzo) and *Bam*HI (manufactured by Takara Shuzo), and the reaction solution was subjected to 0.8% agarose gel electrophoresis to recover a fragment of about 10.7 kbp.

The DNA fragment containing shFcγRIIIa cDNA and the plasmid pKANTEX93-derived fragment obtained in the above were ligated by using DNA Ligation Kit Ver. 2.0 (manufactured by Takara Shuzo). The *Escherichia coli* DH5α (manufactured by TOYOBO) was transformed by using the reaction solution. Each plasmid DNA was isolated from the resulting transformants and then allowed to react using BigDye Terminator Cycle Sequencing FS Ready Reaction Kit (manufactured by Parkin Elmer) according to the manufacture's instructions, and the nucleotide sequence of cDNA inserted into each plasmid was determined by using DNA sequence ABI PRISM 377 (manufactured by Parkin Elmer) to confirm that expression vector pKANTEXFcγRIIIa-His3 was obtained.

### 2. Preparation of cell stably producing shFcγRIIIa

Cells stably producing shFcγRIIIa were prepared by introducing the shFcγRIIIa expression vector pKANTEXFcγRIIIa-His constructed in the above item 1 into rat myeloma YB2/0 cell [ATCC CRL-1662, J. Cell. Biol., 93, 576 (1982)] in the same manner as the method described in the item 3 of Reference Example 1. Also, the amount of shFcγRIIIa expression in the culture supernatant was measured by ELISA described in the item 4 of this Reference Example. Finally, a transformant capable of growing in the Hybridoma-SFM-FBS(10) medium containing 1.0 mg/mL G418 and 200 nM MTX and also of highly producing shFcγRIIIa was obtained. The resulting transformant was cloned twice by limiting dilution. The transformant cell clone KC 1107 producing shFcγRIIIa was obtained.

### 3. Purification of shFcγRIIIa

The shFcγRIIIa-producing transformant cell clone KC1107 obtained in the item 2 of this Reference Example was suspended in Hybridoma-SFM-GF(5) [Hybridoma-SFM medium (manufactured by Life Technologie) containing 5% Daigo's GF21 (manufactured by Wako Pure Chemical Industries)] to give a density of 3×10⁵ cells/mL and dispensed at 50 mL into 182 cm² flasks (manufactured by Greiner). After culturing at 37°C for 4 days in a 5% CO₂ incubator, the culture supernatants were recovered. shFcγRIIIa was purified from the culture supernatants by using Ni-NTA agarose (manufactured by QIAGEN) column according to the manufacture's instructions.

### 4. Detection of shFcγRIIIa (ELISA)

shFcγRIIIa in culture supernatant or purified shFcγRIIIa was detected or determined by the ELISA shown below.

A solution of a mouse antibody against His-tag, Tetra·His Antibody (manufactured by QIAGEN), adjusted to 5 µg/mL with PBS was dispensed at 50 µL/well into each well of a 96 well plate for ELISA (manufactured by Greiner) and allowed to react at 4°C for 12 hours or more. After the reaction, 1% BSA-PBS was added at 100 µL/well and allowed to react at room temperature for 1 hour to block the remaining active groups. After 1% BSA-PBS was discarded, culture supernatant of the transformant or each of various dilution solutions of purified shFcγRIIIa was added at 50 µL/well and allowed to react at room temperature for 1 hour. After the reaction and subsequent washing of each well with Tween-PBS, a biotin-labeled mouse anti-human CD16 antibody solution (manufactured by PharMingen) diluted 50-fold with 1% BSA-PBS was added at 50 µL/well and allowed to react at room temperature for 1 hour. After the reaction and subsequent washing with Tween-PBS, a peroxidase-labeled Avidin D solution (manufactured by Vector) diluted 4,000-fold with 1% BSA-PBS was added at 50 µL/well and allowed to react at room temperature for 1 hour. After the reaction and subsequent washing with Tween-PBS, the ABTS substrate solution was added at 50 µL/well to develop color, and 5 minutes thereafter, the reaction was stopped by adding 5% SDS solution at 50 µL/well. Then, OD415 was measured.
Free Text in Sequence Listing
SEQ ID NO:10-Explanation of artificial sequence: Synthetic RNA
SEQ ID NO: 11-Explanation of artificial sequence: Synthetic RNA
SEQ ID NO: 12-Explanation of artificial sequence: Synthetic RNA
SEQ ID NO: 13-Explanation of artificial sequence: Synthetic RNA
SEQ ID NO:14-Explanation of artificial sequence: Synthetic RNA
SEQ ID NO:15-Explanation of artificial sequence: Synthetic RNA
SEQ ID NO:16-Explanation of artificial sequence: Synthetic RNA
SEQ ID NO:17-Explanation of artificial sequence: Synthetic RNA
SEQ ID NO:18-Explanation of artificial sequence: Synthetic RNA
SEQ ID NO:22-Explanation of artificial sequence: Synthetic RNA
SEQ ID NO:23-Explanation of artificial sequence: Synthetic RNA
SEQ ID NO:24-Explanation of artificial sequence: Synthetic RNA
SEQ ID NO:25-Explanation of artificial sequence: Synthetic RNA
SEQ ID NO:26-Explanation of artificial sequence: Synthetic RNA
SEQ ID NO:27-Explanation of artificial sequence: Synthetic RNA
SEQ ID NO:28-Explanation of artificial sequence: Synthetic RNA
SEQ ID NO:29-Explanation of artificial sequence: Synthetic RNA
SEQ ID NO:30-Explanation of artificial sequence: Synthetic RNA
SEQ ID NO:31-Explanation of artificial sequence: Synthetic DNA
SEQ ID NO:32-Explanation of artificial sequence: Synthetic DNA
SEQ ID NO:33-Explanation of artificial sequence: Synthetic DNA
SEQ ID NO:34-Explanation of artificial sequence: Synthetic DNA
SEQ ID NO:36-Explanation of artificial sequence: Synthetic DNA
SEQ ID NO:37-Explanation of artificial sequence: Synthetic DNA
SEQ ID NO:38-Explanation of artificial sequence: Synthetic DNA
SEQ ID NO:39-Explanation of artificial sequence: Synthetic DNA
SEQ ID NO:40-Explanation of artificial sequence: Synthetic DNA
SEQ ID NO:41-Explanation of artificial sequence: Synthetic DNA
SEQ ID NO:42-Explanation of artificial sequence: Synthetic DNA
SEQ ID NO:43-Explanation of artificial sequence: Synthetic DNA
SEQ ID NO:44-Explanation of artificial sequence: Synthetic DNA
SEQ ID NO:45-Explanation of artificial sequence: Synthetic DNA
SEQ ID NO:46-Explanation of artificial sequence: Synthetic DNA
SEQ ID NO:47-Explanation of artificial sequence: Synthetic DNA
SEQ ID NO:48-Explanation of artificial sequence: Synthetic DNA
SEQ ID NO:49-Explanation of artificial sequence: Synthetic DNA
SEQ ID NO:52-Explanation of artificial sequence: Synthetic DNA

## Claims

1. A process for producing an antibody composition using a cell, which comprises using a cell into which a double-stranded RNA comprising an RNA selected from the following (a) or (b) and its complementary RNA is introduced:
(a) an RNA comprising the nucleotide sequence represented by any one of SEQ ID NOs:9 to 30;
(b) an RNA consisting of a nucleotide sequence in which one or several nucleotide(s) is/are deleted, substituted, inserted and/or added in the nucleotide sequence represented by any one of SEQ ID NOs:9 to 30 and having activity of suppressing the function of an enzyme relating to the modification of a sugar chain in which 1-position of fucose is bound to 6-position of N-acetylglucosamine in the reducing end through α-bond in a complex type N-glycoside-linked sugar chain.

2. The process according to claim 1, wherein the enzyme relating to the modification of a sugar chain in which 1-position of fucose is bound to 6-position of N-acetylglucosamine in the reducing end through α-bond in the complex type N-glycoside-linked sugar chain is α1,6-fucosyltransferase.

3. The process according to claim 2, wherein the α1,6-fucosyltransferase is a protein encoded by a DNA selected from the group consisting of the following (a) to (h):
(a) a DNA comprising the nucleotide sequence represented by SEQ ID NO:1;
(b) a DNA comprising the nucleotide sequence represented by SEQ ID NO:2;
(c) a DNA comprising the nucleotide sequence represented by SEQ ID NO:3;
(d) a DNA comprising the nucleotide sequence represented by SEQ ID NO:4;
(e) a DNA which hybridizes with a DNA consisting of the nucleotide sequence represented by SEQ ID NO:1 under stringent conditions and encodes a protein having α1,6-fucosyltransferase activity;
(f) a DNA which hybridizes with a DNA consisting of the nucleotide sequence represented by SEQ ID NO:2 under stringent conditions and encodes a protein having α1,6-fucosyltransferase activity;
(g) a DNA which hybridizes with a DNA consisting of the nucleotide sequence represented by SEQ ID NO:3 under stringent conditions and encodes a protein having α1,6-fucosyltransferase activity;
(h) a DNA which hybridizes with a DNA consisting of the nucleotide sequence represented by SEQ ID NO:4 under stringent conditions and encodes a protein having α1,6-fucosyltransferase activity.

4. The process according to claim 2, wherein the α1,6-fucosyltransferase is a protein selected from the group consisting of the following (a) to (1):
(a) a protein comprising the amino acid sequence represented by SEQ ID NO:5;
(b) a protein comprising the amino acid sequence represented by SEQ ID NO:6;
(c) a protein comprising the amino acid sequence represented by SEQ ID NO:7;
(d) a protein comprising the amino acid sequence represented by SEQ ID NO:8;
(e) a protein consisting of an amino acid sequence in which one or more amino acid(s) is/are deleted, substituted, inserted and/or added in the amino acid sequence represented by SEQ ID NO:5 and having α1,6-fucosyltransferase activity;
(f) a protein consisting of an amino acid sequence in which one or more amino acid(s) is/are deleted, substituted, inserted and/or added in the amino acid sequence represented by SEQ ID NO:6 and having α1,6-fucosyltransferase activity;
(g) a protein consisting of an amino acid sequence in which one or more amino acid(s) is/are deleted, substituted, inserted and/or added in the amino acid sequence represented by SEQ ID NO:7 and having α1,6-fucosyltransferase activity;
(h) a protein consisting of an amino acid sequence in which one or more amino acid(s) is/are deleted, substituted, inserted and/or added in the amino acid sequence represented by SEQ ID NO:8 and having α1,6-fucosyltransferase activity;
(i) a protein consisting of an amino acid sequence which has 80% or more homology to the amino acid sequence represented by SEQ ID NO:5 and having α1,6-fucosyltransferase activity;
(j) a protein consisting of an amino acid sequence which has 80% or more homology to the amino acid sequence represented by SEQ ID NO:6 and having α1,6-fucosyltransferase activity;
(k) a protein consisting of an amino acid sequence which has 80% or more homology to the amino acid sequence represented by SEQ ID NO:7 and having α1,6-fucosyltransferase activity;
(l) a protein consisting of an amino acid sequence which has 80% or more homology to the amino acid sequence represented by SEQ ID NO:8 and having α1,6-fucosyltransferase activity.

5. The process according to any one of claims 1 to 4, wherein the cell into which the RNA having activity of suppressing the function of an enzyme relating to the modification of a sugar chain in which 1-position of fucose is bound to 6-position of N-acetylglucosamine in the reducing end through α-bond in a complex type N-glycoside-linked sugar chain is introduced is a cell which is resistant to a lectin which recognizes a sugar chain structure in which 1-position of fucose is bound to 6-position of N-acetylglucosamine in the reducing end through α-bond in an N-glycoside-linked sugar chain.

6. The process according to claim 5, wherein the cell is resistant to at least one lectin selected from the group consisting of the following (a) to (d):
(a) a *Lens culinaris* lectin;
(b) a *Pisum sativum* lectin;
(c) a *Vicia faba* lectin;
(d) an *Aleuria aurantia* lectin.

7. The process according to any one of claims 1 to 6, wherein the cell is selected from the group consisting of a yeast cell, an animal cell, an insect cell and a plant cell.

8. The process according to any one of claims 1 to 7, wherein the cell is a cell selected from the group consisting of the following (a) to (i):
(a) a CHO cell derived from Chinese hamster ovary tissue;
(b) a rat myeloma cell line YB2/3HL.P2.G11.16Ag.20 cell;
(c) a mouse myeloma cell line NS0 cell;
(d) a mouse myeloma cell line SP2/0-Ag14 cell;
(e) a BHK cell derived from Syrian hamster kidney tissue;
(f) an antibody-producing hybridoma cell;
(g) a human leukemia cell line Namalwa cell;
(h) an embryonic stem cell;
(i) a fertilized egg cell.

9. The process according to any one of claims 1 to 8, wherein the cell is a transformant into which a gene encoding an antibody molecule is introduced.

10. The process according to claim 9, wherein the antibody molecule is selected from the group consisting of the following (a) to (d):
(a) a human antibody;
(b) a humanized antibody;
(c) an antibody fragment comprising the Fc region of (a) or (b);
(d) a fusion protein comprising the Fc region of (a) or (b).

11. The process according to claim 9 or 10, wherein the antibody molecule belongs to an IgG class.

12. The process according to any one of claims 1 to 11, wherein the antibody composition is an antibody composition having higher antibody-dependent cell-mediated cytotoxic activity than an antibody composition produced by a parent cell into which a double-stranded RNA comprising an RNA selected from the following (a) or (b) and its complementary RNA is not introduced:
(a) an RNA comprising the nucleotide sequence represented by any one of SEQ ID NOs:9 to 30;
(b) an RNA consisting of a nucleotide sequence in which one or several nucleotide(s) is/are deleted, substituted, inserted and/or added in the nucleotide sequence represented by any one of SEQ ID NOs:9 to 30 and having activity of suppressing the function of an enzyme relating to the modification of a sugar chain in which 1-position of fucose is bound to 6-position of N-acetylglucosamine in the reducing end through α-bond in a complex type N-glycoside-linked sugar chain.

13. The process according to claim 12, wherein the antibody composition having higher antibody-dependent cell-mediated cytotoxic activity is an antibody composition which comprises antibody molecules having complex type N-glycoside-linked sugar chains in the Fc region, and in which a ratio of sugar chains in which fucose is not bound to N-acetylglucosamine in the reducing end in the sugar chains among the complex type N-glycoside-linked sugar chains is higher than that of an antibody composition produced by the parent cell.

14. The process according to claim 13, wherein the complex type N-glycoside-linked sugar chains are sugar chains in which 1-position of fucose is not bound to 6-position of N-acetylglucosamine in the reducing end through α-bond in the sugar chains.

15. The process according to any one of claims 12 to 14, wherein the antibody composition having higher antibody-dependent cell-mediated cytotoxic activity is an antibody composition which comprises antibody molecules having complex type N-glycoside-linked sugar chains in the Fc region, and in which the ratio of sugar chains in which fucose is not bound to N-acetylglucosamine in the reducing end in the sugar chains among the complex type N-glycoside-linked sugar chains is 20% or more.

16. The process according to any one of claims 12 to 15, wherein the antibody composition having higher antibody-dependent cell-mediated cytotoxic activity is an antibody composition which comprises antibody molecules having complex type N-glycoside-linked sugar chains in the Fc region, and in which the complex type N-glycoside-linked sugar chains are sugar chains in which fucose is not bound to N-acetylglucosamine in the reducing end.

17. A cell into which an RNA capable of suppressing the function of an enzyme relating to the modification of a sugar chain in which 1-position of fucose is bound to 6-position of N-acetylglucosamine in the reducing end through α-bond in a complex type N-glycoside-linked sugar chain is introduced, and which is used in the process according to any one of claims 1 to 16.

18. The cell according to claim 17, wherein the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.

19. A cell in which an RNA selected from RNAs of the group consisting of the nucleotide sequences represented by any one of SEQ ID NOs:9 to 30 is introduced or expressed.

20. A double-stranded RNA consisting of an RNA selected from the following (a) or (b) and its complementary RNA:
(a) an RNA comprising the nucleotide sequence represented by any one of SEQ ID NOs:9 to 30;
(b) an RNA consisting of a nucleotide sequence in which one or several nucleotide(s) is/are deleted, substituted, inserted and/or added in the nucleotide sequence represented by any one of SEQ ID NOs:9 to 30 and having activity of suppressing the function of an enzyme relating to the modification of a sugar chain in which 1-position of fucose is bound to 6-position of N-acetylglucosamine in the reducing end through α-bond in a complex type N-glycoside-linked sugar chain.

21. A DNA corresponding to the RNA described in claim 20 and a complementary DNA to the DNA.

22. A recombinant DNA which is obtainable by introducing a DNA corresponding to the RNA described in claim 20 and a complementary DNA to the DNA into a vector.

23. The recombinant DNA according to claim 22, which expresses the double-stranded RNA according to claim 20.

24. A transformant which is obtainable by introducing the recombinant DNA according to claim 22 or 23 into a cell.

25. A method for constructing a cell which is resistant to a lectin which recognizes a sugar chain structure in which 1-position of fucose is bound to 6-position of N-acetylglucosamine in the reducing end through α-bond in a complex type N-glycoside-linked sugar chain, which comprises introducing or expressing the double-stranded RNA described in claim 20 in a cell.

26. The method according to claim 25, wherein the cell which is resistant to a lectin which recognizes a sugar chain structure in which 1-position of fucose is bound to 6-position of N-acetylglucosamine in the reducing end through α-bond in a complex type N-glycoside-linked sugar chain is resistant to at least one lectin selected from the group consisting of the following (a) to (d):
(a) a *Lens culinaris* lectin;
(b) a *Pisum sativum* lectin;
(c) a *Vicia faba* lectin;
(d) an *Aleuria aurantia* lectin.

27. A method for suppressing the function of an enzyme relating to the modification of a sugar chain in which 1-position of fucose is bound to 6-position of N-acetylglucosamine in the reducing end through α-bond in a complex type N-glycoside-linked sugar chain, which comprises using an RNA selected from RNAs of the group consisting of the nucleotide sequences of any one of SEQ ID NOs:9 to 30.

28. The method according to claim 27, wherein the enzyme relating to the modification of a sugar chain in which 1-position of fucose is bound to 6-position of N-acetylglucosamine in the reducing end through α-bond in a complex type N-glycoside-linked sugar chain is α1,6-fucosyltransferase.
